# EUROPÄISCHE PATENTSCHRIFT

(11) **EP 3 134 422 B1**
(45) Veröffentlichungstag und Bekanntmachung des Hinweises auf die Patenterteilung: **12.06.2019**
(21) Anmeldenummer: 15720928.9
(22) Anmeldetag: 24.04.2015
(51) Int. Cl.: C07F 11/00, B01J 31/00, C08F 4/00, B01J 31/18, B01J 31/22, C07C 67/333, C07C 319/20, C07C 6/04, C07C 253/30, C08G 61/08, C08G 61/12

(54) **N-HETEROZYKLISCHE CARBENKOMPLEXE VON METALLIMIDOALKYLIDENEN UND METALLOXOALKYLIDENEN UND DEREN VERWENDUNG**
N-HETEROCYCLIC CARBENE COMPLEXES OF METAL IMIDO ALKYLIDENES AND METAL OXO ALKYLIDENES, AND THE USE OF SAME
COMPLEXE CARBÈNE N-HÉTÉROCYCLIQUE D'IMIDO-ALKYLIDÈNES MÉTALLIQUES ET D'OXO-ALKYLIDÈNES MÉTALLIQUES ET LEUR UTILISATION

(30) Priorität: 25.04.2014 DE 102014105885
(43) Veröffentlichungstag der Anmeldung: 01.03.2017
(73) Patentinhaber: Universität Stuttgart, 70174 Stuttgart (DE)
(72) Erfinder: BUCHMEISER, Michael R., 73630 Remshalden (DE); SEN, Suman, Tamluk West Bengal 721636 (IN); SCHOWNER, Roman, 78647 Trossingen (DE)
(74) Vertreter: Held, Stephan
(86) Internationale Anmeldenummer: PCT/EP2015/058888
(87) Internationale Veröffentlichungsnummer: WO 2015/162245

(56) Entgegenhaltungen:
- MICHAEL R. BUCHMEISER ET AL: "N-Heterocyclic Carbene, High Oxidation State Molybdenum Alkylidene Complexes: Functional-Group-Tolerant Cationic Metathesis Catalysts", ANGEWANDTE CHEMIE INTERNATIONAL EDITION, Bd. 53, Nr. 35, 25. August 2014 (2014-08-25), Seiten 9384-9388, XP055203685, ISSN: 1433-7851, DOI: 10.1002/anie.201404655
- CEZARY SAMOJLOWICZ ET AL: "Ruthenium-Based Olefin Metathesis Catalysts Bearing N -Heterocyclic Carbene Ligands", CHEMICAL REVIEWS, Bd. 109, Nr. 8, 12. August 2009 (2009-08-12), Seiten 3708-3742, XP055071094, ISSN: 0009-2665, DOI: 10.1021/cr800524f

## Beschreibung

Die Erfindung betrifft N-heterozyklische Carbenkomplexe von Metallimidoalkylidenen und Metalloxoalkylidenen und deren Verwendung als Katalysatoren in Olefinmetathesereaktionen.

Alkylidenkomplexe von Metallen der Gruppe VI (Cr, Mo, W) in ihrer höchsten Oxidationsstufe (*"high oxidation state metal alkylidenes"*) sind seit vielen Jahren bekannt (Chem. Rev. 2002, 102, 145; Chem. Commun, 2005, 2773; Chem. Rev. 2009, 109, 3211). Verbindungen der allgemeinen Formeln M(NR)(CHR')X¹X² und M(O)(CHR') X¹X², in denen R für Alkoxy, einen Aryl- oder Adamantylrest, R' für t-Butyl oder CMe₂Ph und X¹ und X² für Alkoxy-, Aryloxy-, Pyrrolidreste und Ähnliches stehen, während M ein Metall in von Form vom Molybdän oder Wolfram ist, werden auch als *"Schrock-Carbene"* bzw. *"Schrock-Katalysatoren"* bezeichnet. Derartige Verbindungen weisen eine hohe Aktivität in verschiedenen (asymmetrischen und desymmetrisierenden) Olefinmetathesereaktionen auf und konnten erfolgreich in Ringschlussmetathesen, Kreuzmetathesen, ringöffnenden Kreuzmetathesen, (Kreuz-)En-in-Metathesen, Ringschluss-En-in-Metathesen, Kreuz-Endiin-Metathesen, tandem-Ringöffnungs-Ringschluss-Metathesen, ringöffnenden Metathesepolymerisationen (ROMP), 1-Alkinpolymerisationen, acyklischen Metathesepolymerisationen (ADMET) oder in Cyclopolymerisationen von α,ω-Diinen eingesetzt werden. Bei den bekannten Schrock-Katalysatoren hat sich jedoch deren geringe Toleranz gegenüber funktionellen Gruppen, wie insbesondere Ketonen, Aldehyden und Isocyanaten und protischen Verbindungen, wie Alkoholen, Thiolen, Carbonsäuren, und primären oder sekundären Aminen, als nachteilig erwiesen. Bei Substraten, die solche funktionelle Gruppen enthalten, wurde deshalb eine relativ schnelle Deaktivierung oder Zersetzung des Katalysators beobachtet.

Aus diesem Grund besteht ein Bedarf an Katalysatoren des *"Schrock-Typs",* die eine möglichst hohe Toleranz gegenüber verschiedenen funktionellen Gruppen aufweisen und gleichzeitig eine möglichst hohe Aktivität aufweisen. Der Erfindung liegt demzufolge die Aufgabe zugrunde, ein vorteilhaftes Katalysatorsystem vorzuschlagen, das diese Mängel behebt.

Diese Aufgabe wird erfindungsgemäß gelöst durch einen N-heterozyklischen Carbenkomplex der allgemeinen Formel I, II, III oder IV 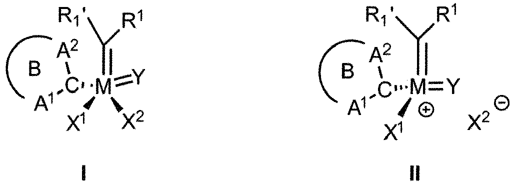 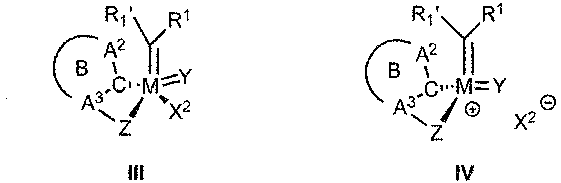
der dadurch gekennzeichnet ist, dass A¹ für NR² oder PR², A² für CR²R^{2'}, NR², PR², O oder S steht, A³ für N oder P steht, C für ein Carben-Kohlenstoffatom steht,
der Ring B ein unsubstituierter oder ein ein- oder mehrfach substituierter 5- bis 7-gliedriger Ring ist, der neben A¹, A² bzw. A³ weitere Heteroatome in Form von Stickstoff, Phosphor, Sauerstoff oder Schwefel enthalten kann und dessen Substituenten die für R² beschriebene Bedeutung haben können,
die Substituenten R² und R^{2'} unabhängig voneinander für H, einen linearen, teilcyclischen oder verzweigten C₁-C₁₈-Alkyl-, insbesondere einen C₁-C₇-Alkyl-, einen linearen, teilcyclischen oder verzweigten C₂-C₁₈-Alkenyl-, insbesondere einen C₂-C₇-Alkenyl-, einen C₃-C₁₂-Cycloalkyl-, insbesondere einen C₃-C₆-Cycloalkyl-, einen linearen, teilcyclischen oder verzweigten C₆-C₁₀₀-Polyoxaalkyl-, insbesondere C₆-C₃₀-Polyoxaalkyl-, einen C₅-C₁₄-Aryl- oder -Heteroaryl-Rest, einen C₅-C₁₄-Aryloxy-, einen linearen, teilcyclischen oder verzweigten C₁-C₁₈-Perfluoralkyl-, insbesondere C₁-C₇-Perfluoralkyl-, einen linearen, teilcyclischen oder verzweigten C₁-C₁₈-Perchloralkyl-, insbesondere einen C₁-C₇-Perchloralkyl-, einen linearen, teilcyclischen oder verzweigten teilfluorierten C₁-C₁₈-Alkyl-, insbesondere einen teilfluorierten C₁-C₇-Alkyl-, einen linearen, teilcyclischen oder verzweigten teilchlorierten C₁-C₁₈-Alkyl-, insbesondere einen teilchlorierten C₁-C₇-Alkyl-, einen per- oder teilfluorierten C₆-C₁₄-Aryl-, einen per- oder teilchlorierten C₅-C₁₄-Aryl-Rest steht, und, wenn A¹ und A² jeweils für NR² oder PR² stehen, R² gleich oder verschieden sein kann, oder
R² und R^{2'} zusammen einen linearen oder verzweigten C₁-C₁₈-Alkylen-, insbesondere einen C₁-C₇-Alkylenrest bilden,
M in den Formeln I, II, III oder IV für Cr, Mo oder W steht,
X¹ bzw. X² in Formeln I bis IV gleich oder verschieden sind und aus der Gruppe umfassend C₁-C₁₈ Carboxylate, C₁-C₁₈-Alkoxide, fluorierte C₁-C₁₈ Alkoxide, C₁-C₁₈ mono- oder polyhalogenierte Carboxylate, un-, ein- oder mehrfach substituierte C₆-C₁₈-Mono-, Bi- oder Terphenolate, Trifluormethansulfonat, nicht koordinierende Anionen, inbesonders Tetrakis(3,5-bis(trifluormethyl)phenyl)borat, Tetrakis(pentafluorophenyl)borat, Tetrakis(nonafluoro-t-butoxy)aluminat, Tetrafluoroborat, Hexafluorophosphat und Hexafluoroantimonat ausgewählt sind, wobei die Substituenten an den Mono-, Bi- oder Terphenolaten neben Halogen die gleiche Bedeutung haben können wie R²,
Y Sauerstoff, Schwefel, ein N-Adamantyl-, ein N-*tert*-Butyl, ein C₆-C₁₄-N-Aryl-Rest, insbesondere ein C₆-C₁₀-N-Aryl-Rest ist, wobei der Aryl-Rest ein- oder mehrfach mit Halogen, einem linearen oder verzweigten C₁-C₁₈ Alkyl-, einem linearen oder verzweigten C₁-C₁₈ Alkyloxy- oder einem unsubstituierten oder substituierten Phenyl-Rest substituiert sein kann, dessen Substituenten die gleiche Bedeutung haben wie R²,
Z eine lineare, teilcyclische oder verzweigte C₁-C₁₀-Alkylenoxy-, insbesondere eine C₁-C₆-Alkylenoxy-, eine lineare, teilcyclische oder verzweigte C₁-C₁₀-Alkylenthio-, insbesondere eine C₁-C₅-Alkylenthio-, eine lineare, teilcyclische oder verzweigte C₁-C₁₀-Alkylen-NR²-, insbesondere eine C₁-C₅-Alkylen-NR²-, eine C₆-C₁₀-Arylenoxy-, eine per- oder teilfluorierte C₆-C₁₄-Arylenoxy-, eine per- oder teilchlorierte C₆-C₁₄-Arylenoxy-, eine per- oder teilbromierte C₆-C₁₄-Arylenoxy-, eine C₆-C₁₄-Arylenthio-, eine per- oder teilfluorierte C₆-C₁₄-Arylenthio-, eine per- oder teilchlorierte C₆-C₁₄-Arylenthio-, eine per- oder teilbromierte C₆-C₁₄-Arylenthio- oder eine C₆-C₁₄-Arylen-NR²-, eine per- oder teilfluorierte C₆-C₁₄-Arylen-NR²-, eine per- oder teilchlorierte C₆-C₁₄-Arylen-NR²-, eine per- oder teilbromierte C₆-C₁₄-Arylen-NR²-, , eine C₆-C₁₄-Arylen-PR²-, eine per- oder teilfluorierte C₆-C₁₄-Arylen-PR²-, eine per- oder teilchlorierte C₆-C₁₄-Arylen-PR²-, eine per- oder teilbromierte C₆-C₁₄-Arylen-PR²-, eine Carboxyl, eine Thiocarboxyl oder eine Dithiocarboxyl-Gruppe und
R¹ und R^{1'} in den Formeln I bis IV unabhängig voneinander H oder ein aliphatischer oder aromatischer Rest sind, insbesondere eine lineare oder verzweigte C₁-C₁₈ Alkyl-Gruppe, bevorzugt in Form einer *tert-*Butyl*-* oder CMe₂Ph-Gruppe, oder eine unsubstituierte oder ein- oder mehrfach substituierte C₆-C₁₄-Aryl-Gruppe, wobei die Substituenten die für R² genannten Bedeutungen haben, bevorzugt in Form von 2-(2-Propoxy)phen-1-yl, 2-Methoxyphen-1-yl, 2,4,5-Trimethoxyphenyl oder Ferrocenyl.

Ferner betrifft die Erfindung die Verwendung dieser Verbindungen als Katalysator in Olefinmetathesereaktionen.

Bevorzugte Ausgestaltungen der erfindungsgemäßen Carbenkomplexe ergeben sich aus den Ansprüchen 2 bis 6, während die Ansprüche 7 bis 13 bevorzugte Ausgestaltungen der erfindungsgemäßen Verwendungslehre darstellen.

Für die erfindungsgemäßen Carbenkomplexe der allgemeinen Formeln I bis IV ist es bevorzugt, wenn die für die Substituenten R₂ und R^{2'} erwähnte lineare, teilcyclische oder verzweigte C₁-C₁₈-Alkylgruppe als C₁-C₁₀-Alkylgruppe, bevorzugt als C₁-C₇-Alkylgruppe und insbesondere als C₁-C₄-Alkylgruppe vorliegt. Besonders geeignet sind Methyl, Ethyl und Propylgruppen.

Die lineare, teilcyclische oder verzweigte C₂-C₁₈-Alkenylgruppe liegt zweckmäßigerweise in Form einer C₂-C₁₀-Alkenylgruppe, insbesondere in Form einer C₂-C₇-Alkenylgruppe und bevorzugt in Form Butenyl oder Hexenyl vor. Für die C₃-C₁₂-Cycloalkylgruppe ist es bevorzugt, wenn diese in Form einer C₃-C₆-Cycloalkylgruppe vorliegt. Als geeignete Gruppen sind in diesem Zusammenhang Cyclopentyl und Cyclohexyl zu nennen. Fall es sich bei dem Substituenten R² oder R^{2'} um einen linearen, teilcyclischen oder verzweigten C₆-C₁₀₀-Polyoxaalkyl-Rest handelt, ist es vorteilhaft, wenn dieser in Form eines C₆-C₃₀-Polyoxaalkyl-Rests und insbesondere in Form eines C₆-C₁₅-Polyoxaalkyl-Rests vorliegt. Geeignete Reste sind z.B. Methyloxyethyl oder Methyloxyethyloxy.

Der substituierte oder unsubstituierte C₅-C₁₄-Aryl- oder -Heteroarylrest liegt bevorzugt in Form eines C₆-C₁₄-Aryl- oder -Heteroarylrests, insbesondere einen C₆-C₁₀-Aryl- oder -Heteroaryl-Rests vor. In diesem Zusammenhang haben sich Phenyl-, Naphtyl- oder Ferrocenylreste als besonders geeignet herausgestellt.

Als substituierte oder unsubstituierte C₅-C₁₄-Aryloxyreste sind C₆-C₁₄-AryloxyReste und insbesondere C₆-C₁₀-Aryloxy-Reste bevorzugt. Besonders geeignete unsubstituierte Aryloxyreste sind Phenyloxy oder Naphtyloxy.

Der lineare, teilcyclische oder verzweigte C₁-C₁₈-perfluorierten Alkyl-Rest liegt insbesondere in Form eines C₁-C₁₀-perfluorierten Alkyl-Rests, bevorzugt in Form eines C₁-C₇- perfluorierten Alkyl-Rests, und besonders bevorzugt in Form eines C₁-C₄-Perfluoroalkyl-Restes vor, wobei Trifluormethyl als Rest am meisten bevorzugt ist.

Ebenso liegt der lineare, teilcyclische oder verzweigte C₁-C₁₈-perchlorierten Alkyl-Rest, insbesondere in Form eines C₁-C₁₀-perchlorierten Alkyl-Rests, bevorzugt in Form eines C₁-C₇-perchlorierten Alkyl-Rests und besonders bevorzugt in Form eines C₁-C₄-Perchloralkyl-Restes vor, wobei Trichlormethyl als Rest am meisten bevorzugt ist.

Der lineare, teilcyclische oder verzweigte teilfluorierte C₁-C₁₈-Alkyl-Rest liegt bevorzugt als teilfluorierter C₁-C₁₀-Alkyl-Rest, und insbesondere als teilfluorierter C₁-C₇-Alkyl-Rest vor. Ein Beispiel für einen solchen Rest ist Trifluorethyl.

Der lineare, teilcyclische oder verzweigte teilchlorierte C₁-C₁₈-Alkyl-Rest liegt bevorzugt als teilchlorierter C₁-C₁₀-Alkyl-Rest, und insbesondere als teilchlorierter C₁-C₇-Alkyl-Rest vor. Ein Beispiel für einen solchen Rest ist Trichlorethyl.

Der perfluorierte C₅-C₁₄-Aryl-Rest liegt insbesondere als perfluorierter C₆-C₁₄-Aryl-Rest, bevorzugt als perfluorierter C₆-C₁₀-Aryl-Rest und besonders bevorzugt in Form eines Pentafluorophenylrests vor.

Ebenso liegt der teilfluorierte C₅-C₁₄-Aryl-Rest insbesondere als teilfluorierter C₆-C₁₄-Aryl-Rest, bevorzugt als teilfluorierter C₆-C₁₀-Aryl-Rest und besonders und bevorzugt in Form von Fluorophenyl vor.

Der perchlorierte C₅-C₁₄-Aryl-Rest liegt insbesondere als perchlorierter C₆-C₁₄-Aryl-Rest, bevorzugt als perchlorierter C₆-C₁₀-Aryl-Rest und besonders und bevorzugt in Form eines Pentachlorophenylrests vor.

Ebenso liegt der teilchlorierte C₅-C₁₄-Aryl-Rest insbesondere als teilchlorierter C₆-C₁₄-Aryl-Rest, bevorzugt als teilchlorierter C₆-C₁₀-Aryl-Rest und besonders und bevorzugt in Form von Chlorophenyl vor.

Wenn A¹ und A² jeweils für NR² oder PR² stehen, können die Reste R² und R² gleich oder verschieden sein.

Generell gilt es als bevorzugt für den Substituenten R², sofern er direkt an einen der Substituenten A¹ oder A² gebunden ist, dass er ein von Wasserstoff verschiedener Substituent ist.

Für den Fall, dass R² und R^{2'} zusammen eine lineare oder verzweigte C₁-C₁₈-Alkylen-Gruppe bilden, liegt diese bevorzugt als C₁-C₇-Alkylen-Gruppe und besonders bevorzugt in Form einer Butylen oder Pentylengruppe vor.

Im Rahmen der vorliegenden Erfindung steht A¹ bevorzugt für NR². Unabhängig davon steht A² bevorzugt für NR² oder S und besonders bevorzugt für NR².

Bei dem Ring B handelt es sich um einen heterocyclischen 5- bis 7-gliedrigen Ring, der in direkter Nachbarschaft zum carbenoiden Kohlenstoff (d.h. dem Kohlenstoffatom, das in Form eines Carbens vorliegt) mindestens ein Stickstoffatom sowie weiterhin entweder ein weiteres Stickstoffatom, Schwefelatom, Sauerstoffatom, Phosphoratom oder quartäres Kohlenstoffatom aufweist. Bevorzugt weist der heterozyklische 5- bis 7-gliedrige Ring in direkter Nachbarschaft zum carbenoiden Kohlenstoff mindestens ein Stickstoffatom sowie weiterhin entweder ein weiteres Stickstoffatom oder Schwefelatom auf. Die Stickstoffatome bzw. Phosphoratome weisen in diesem Fall einen Substituenten R² auf, der nicht in Form vom Wasserstoff vorliegt, so dass es sich bei den Stickstoffatomen im Ring B um tertiäre Amine bzw. Phosphine handelt. Zudem kann der heterocyclische Ring B substituiert sein, zum Beispiel mit Phenyl oder mit einem weiteren, vorzugsweise aromatischen Ring ein bicyclisches oder polycyclisches System bilden. So kann es sich bei dem Ring B beispielsweise um einen benzannelierten, naphthannellierten, Phenanthren- oder Anthrachinon-annellierten 5- bis 7-gliedrigen Ring handeln. Zudem kann der Ring B weitere Substituenten in Form von Halogenen, C₁-C₁₈-Carbonsäureestern, Carbonsäureamiden, Sulfonamiden, Sulfonsäureestern, Alkylnitrilen, Ethern, Thiethern, Aminen aufweisen. Für die Substituenten ist es bevorzugt, wenn sie 1 bis 8 Kohlenstoffatome enthalten. Besonders bevorzugte Substituenten sind Fluor, Chlor und Brom, sowie Methoxycarbonyl und Ethoxycarbonyl.

Im Rahmen der vorliegenden Erfindung hat es sich als besonders zweckmäßig erwiesen, wenn der Ring B ein aus der Gruppe, umfassend 1,3-disubstituierte Imidazol-2-ylidene, 1,3-disubstituierte Imidazolin-2-ylidene, 1,3-disubstituierte Tetrahydropyrimidin-2-ylidene, 1,3-disubstituierte Diazepin-2-ylidene, 1,3-disubstituierte Dihydrodiazepin-2-ylidene, 1,3-disubstituierte Tetrahydrodiazepin-2-ylidene, N-substituierte Thiazol-2-ylidene, N-substituierte Thiazolin-2-ylidene, N-substituierte Triazol-2-ylidene, N-substituierte Dihydrotriazol-2-ylidene, ein- oder mehrfach substituierte Triazolin-2-ylidene, N-substituierte Thiadiazol-2-ylidene, ein- oder mehrfach substituierte Thiadiazolin-2-ylidene und ein- oder mehrfach substituierte Tetrahydrotriazol-2-ylidene ausgewählter Heterozyklus ist.

Besonders bevorzugt ist der Ring B von einem 1,3-disubstituierten Imidazol-2-yliden oder einem 1,3-disubstituierten Imidazolin-2-yliden abgeleitet. Der Substituent R² besteht in diesem Fall zweckmäßigerweise aus einem verzweigten C₃-C₆-Alkyl-Rest, insbesondere in Form eines t-Butyl- oder eines substituierten ArylRestes, insbesondere in Form eines 2,4,6-Trimethylphenyl-Restes (auch als Mesityl-Gruppe bezeichnet).

Bei dem Metall in dem Carbenkomplex der allgemeinen Formeln I bis IV handelt es sich vorzugsweise um Mo oder W, insbesondere um Mo.

Liegt mindestens einer der beiden Substituenten X¹ und X² in Form eines C₁-C₁₈-Carboxylats vor, so ist es bevorzugt, wenn es sich dabei um ein C₁-C₈-Carboxylat handelt. Als besonders geeignete Carboxylate sind das Acetat, Propionat und Benzoat zu nennen.

Liegt mindestens einer der beiden Substituenten X¹ und X² in Form eines C₁-C₁₈-Alkoxids vor, so ist es bevorzugt, wenn es sich dabei um ein C₁-C₈-Alkoxid handelt. Als besonders geeignete Alkoxide sind das 2-Propoxid und das tert-Butoxid zu nennen.

Liegt mindestens einer der beiden Substituenten X¹ und X² in Form eines fluorierten C₁-C₁₈-Alkoxids vor, so ist es bevorzugt, wenn es sich dabei um ein fluoriertes C₁-C₈-Alkoxid handelt. Als besonders geeignete fluorierten Alkoxide sind das Hexafluoro-2-propoxid und ein Hexafluoro-*tert*-butoxid zu nennen.

Liegt mindestens einer der beiden Substituenten X¹ und X² in Form eines mono- oder polyhalogenierten C₁-C₁₈-Carboxylats vor, so ist es bevorzugt, wenn es sich dabei um ein mono- oder polyhalogeniertes C₁-C₈-Carboxylat handelt. Als besonders geeignete mono- oder polyhalogenierte C₁-C₁₈-Carboxylate sind Trichloracetat, Trifluoracetat, Pentafluoropropionat, Heptafluorobutyrat, und Pentafluorobenzoat zu nennen.

Bevorzugte un-, ein- oder mehrfach substituierte Mono-, Bi- oder Terphenolate sind das 2,6-Diphenylphenolat, 2',2",6',6"-Tetrakis(2-propyl)-2,6-diphenylphenolat und das 2',2",6',6"-Tetramethyl-2,6-diphenylphenolat.

Generell sollte es sich bei den Substituenten X¹ und X² um schwach oder nicht koordinierende Anionen und insbesondere anionische P-, B- Al-, oder Sb-basierte Anionen handeln.

Für die Substituenten X¹ und X² haben sich insbesondere schwach koordinierende Substituenten, wie beispielsweise Trifluormethansulfonat, Tetrakis(3,5-bis(trifluormethyl)phenyl)borat, Hexafluorophosphat und Hexafluoroantimonat-Substituenten als besonders zweckmäßig herausgestellt. Zudem können Substituenten, wie fluorierte und nicht fluorierte C₁-C₁₈-Alkoxide, insbesondere in Form von C₁-C₄-Alkoxiden, verwendet werden. Besonders geeignete Alkoxide sind Ethanolat, 2-Propanolat, *tert*-Butanolat, Hexafluoro-2-propoxid oder Hexafluoro-*tert-*butanolat.

Als Substituent Y kommen die im vorstehenden bezeichneten Substituenten in Betracht. Für bevorzugte Ausführungsformen dieser Substituenten gilt folgendes: Der C₆-C₁₄-N-Aryl-Rest liegt bevorzugt in Form eines C₆-C₁₀-N-Aryl-Rests vor, wobei der Aryl-Rest ein- oder mehrfach mit Halogen, C₁-C₁₈-Alkyl-Resten, insbesondere C₁-C₈-Alkyl-Resten, C₁-C₁₈-Alkyloxy-Resten, insbesondere C₁-C₈-Alkyloxy-Resten, wobei Methoxy- oder 2-Propoxy-Gruppen besonders bevorzugt sind, oder einem unsubstituierten oder substituierten Phenyl-Rest, dessen Substituenten die gleiche Bedeutung haben können wie für R², substituiert sein kann.

Als besonders bevorzugte Substituenten Y sind insbesondere 2,6-disubstituierte N-Arylreste, vorzugsweise in Form von N-Phenyl-Resten, zu nennen, bei denen die Substituenten bevorzugt als Alkylreste, wie *tert*-Butyl, *iso*-Propyl oder Methyl, oder als Halogene, wie Chlor, Fluor oder Brom oder Gemischen davon, vorliegen. Weiterhin bevorzugte Substituenten Y sind N-Alkylreste, bei denen das Kohlenstoffatom in direkter Nachbarschaft zum Stickstoff ein quartäres Kohlenstoffatom ist. Beispiele solcher N-Alkylreste sind der N-*tert*-Butyl-oder der N-Adamantyl-Rest. Besonders bevorzugte Substituenten Y sind im Rahmen der vorliegenden Erfindung der N-2,6-Dimethylphenyl-, der 2,6-Bis(2-propyl)phenyl, der Pentafluorophenyl, der N-2,6-Dichlorphenyl-, der 2-*tert*-Butylphenyl, der N-*tert*-Butyl- sowie der N-Adamantyl-Rest.

Für bevorzugte Ausgestaltungen des Sunbstituenten Z gilt Folgendes:
- die lineare, teilcyclische oder verzweigte C₁-C₁₀-Alkylenoxy-Gruppe ist bevorzugt eine C₁-C₃-Alkylenoxy-Gruppe, und insbesondere eine Ethylenoxy-Gruppe;
- die lineare, teilcyclische oder verzweigte C₁-C₁₀-Alkylenthio-Gruppe ist bevorzugt eine C₁-C₃-Alkylenthio-Gruppe, und insbesondere eine Ethylenthio-Gruppe;
- die lineare, teilzyklische oder verzweigte C₁-C₁₀-Alkylen-NR²-Gruppe ist bevorzugt eine C₁-C₃-Alkylen-NR²-Gruppe, und insbesondere eine Ethylen-NR²-Gruppe;
- die C₆-C₁₄-Arylenoxy-Gruppe ist bevorzugt eine C₆-C₁₀-Arylenoxy-Gruppe, und insbesondere eine 2-Phenylen-oxygruppe;
- die perfluorierte C₆-C₁₄-Arylenoxy-Gruppe ist bevorzugt eine perfluorierte C₆-C₁₀-Arylenoxy-Gruppe, und insbesondere eine Tetrafluorphenyl-2-en-oxygruppe;
- die teilfluorierte C₆-C₁₄-Arylenoxy-Gruppe ist bevorzugt eine teilfluorierte C₆-C₁₀-Arylenoxy-Gruppe, und insbesondere eine Fluorphenyl-2-en-oxygruppe;
- die perchlorierte C₆-C₁₄-Arylenoxy-Gruppe ist bevorzugt eine perchlorierte C₆-C₁₀-Arylenoxy-Gruppe, und insbesondere eine Tetrachlorphenyl-2-en-oxygruppe;
- die teilchlorierte C₆-C₁₄-Arylenoxy-Gruppe ist bevorzugt eine teilchlorierte C₆-C₁₀-Arylenoxy-Gruppe, und insbesondere eine Chlorphenyl-2-en-oxygruppe;
- die perbromierte C₆-C₁₄-Arylenoxy-Gruppe ist bevorzugt eine perbormierte C₆-C₁₀-Arylenoxy-Gruppe, und insbesondere eine Tetrabromphenyl-2-en-oxygruppe;
- die teilbromierte C₆-C₁₄-Arylenoxy-Gruppe ist bevorzugt eine teilbromierte C₆-C₁₀-Arylenoxy-Gruppe, und insbesondere eine Bromphenyl-2-en-oxygruppe;
- die C₆-C₁₄-Arylenthio-Gruppe ist bevorzugt eine C₆-C₁₀-Arylenthio-Gruppe, und insbesondere eine 2-Phenylen-thiogruppe;
- die perfluorierte C₆-C₁₄-Arylenthio-Gruppe ist bevorzugt eine perfluorierte C₆-C₁₀-Arylenthio-Gruppe, und insbesondere eine Tetrafluorphenyl-2-en-thiogruppe;
- die teilfluorierte C₆-C₁₄-Arylenthio-Gruppe ist bevorzugt eine teilfluorierte C₆-C₁₀-Arylenthio-Gruppe, und insbesondere eine Fluorphenyl-2-en-thiogruppe;
- die perfbromierte C₆-C₁₄-Arylenthio-Gruppe ist bevorzugt eine perbromierte C₆-C₁₀-Arylenthio-Gruppe, und insbesondere eine Tetrabromphenyl-2-en-thiogruppe;
- die teilbromierte C₆-C₁₄-Arylenthio-Gruppe ist bevorzugt eine teilbromierte C₆-C₁₀-Arylenthio-Gruppe, und insbesondere eine Bromphenyl-2-en-thiogruppe;
- die perchlorierte C₆-C₁₄-Arylenthio-Gruppe ist bevorzugt eine perchlorierte C₆-C₁₀-Arylenthio-Gruppe, und insbesondere eine Tetrachlorphenyl-2-en-thiogruppe;
- die teilchlorierte C₆-C₁₄-Arylenthio-Gruppe ist bevorzugt eine teilchlorierte C₆-C₁₀-Arylenthio-Gruppe, und insbesondere eine Chlorphenyl-2-en-thiogruppe;
- die C₆-C₁₄-Arylen-NR²-Gruppe ist bevorzugt eine C₆-C₁₀- Arylen-NR²-Gruppe, und insbesondere eine N-Methylphenyl-2-en- oder N-Ethylphenyl-2-engruppe;
- die perfluorierte C₆-C₁₄-Arylen-NR²-Gruppe ist bevorzugt eine perfluorierte C₆-C₁₀- Arylen-NR²-Gruppe, und insbesondere eine N-Methyltetrafluorophenyl-2-en- oder N-Ethyltetrafluorophenyl-2-engruppe;
- die teilfluorierte C₆-C₁₄-Arylen-NR²-Gruppe ist bevorzugt eine teilfluorierte C₆-C₁₀-Arylen-NR²-Gruppe, und insbesondere eine N-Methylfluorophenyl-2-en- oder N-Ethylfluorophenyl-2-engruppe;
- die perchlorierte C₆-C₁₄-Arylen-NR²-Gruppe ist bevorzugt eine perchlorierte C₆-C₁₀-Arylen-NR²-Gruppe, und insbesondere eine N-Methyltetrachlorophenyl-2-en- oder N-Ethyltetrachlorophenyl-2-engruppe;
- die teilchlorierte C₆-C₁₄-Arylen-NR²-Gruppe ist bevorzugt eine teilchlorierte C₆-C₁₀-Arylen-NR²-Gruppe, und insbesondere eine N-Methylchlorophenyl-2-en- oder N-Ethylchlorophenyl-2-engruppe;
- die perbromierte C₆-C₁₄-Arylen-NR²-Gruppe ist bevorzugt eine perbromierte C₆-C₁₀- Arylen-NR²-Gruppe, und insbesondere eine N-Methyltetrabromphenyl-2-en- oder N-Ethyltetrabromphenyl-2-engruppe;
- die teilbromierte C₆-C₁₄-Arylen-NR²-Gruppe ist bevorzugt eine teibromierte C₆-C₁₀-Arylen-NR²-Gruppe, und insbesondere eine N-Methylbromphenyl-2-en- oder N-Ethylbromphenyl-2-engruppe;
- die C₆-C₁₄-Arylen-PR²-Gruppe ist bevorzugt eine C₆-C₁₀-Arylen-PR²-Gruppe, und insbesondere eine P-Methylphenyl-2-en-, P-Phenyl-phenyl-2-en- oder P-Ethylphenyl-2-engruppe;
- die perfluorierte C₆-C₁₄-Arylen-PR²-Gruppe ist bevorzugt eine perfluorierte C₆-C₁₀-Arylen-PR²-Gruppe, und insbesondere eine P-Methyltetrafluorophenyl-2-en-, Perfluoro-P-phenyl-phenyl-2-en- oder P-Ethyltetrafluorophenyl-2-engruppe;
- die teilfluorierte C₆-C₁₄-Arylen-PR²-Gruppe ist bevorzugt eine teilfluorierte C₆-C₁₀-Arylen-PR²-Gruppe, und insbesondere eine P-Methylfluorophenyl-2-en- oder P-Ethylfluorophenyl-2-engruppe;
- die perchlorierte C₆-C₁₄-Arylen-PR²-Gruppe ist bevorzugt eine perchlorierte C₆-C₁₀-Arylen-PR²-Gruppe, und insbesondere eine P-Methyltetrachlorophenyl-2-en- oder P-Ethyltetrachlorophenyl-2-engruppe;
- die teilchlorierte C₆-C₁₄-Arylen-PR²-Gruppe ist bevorzugt eine teilchlorierte C₆-C₁₀-Arylen-PR²-Gruppe, und insbesondere eine P-Methylchlorophenyl-2-en- oder P-Ethylchlorophenyl-2-engruppe;
- die perbromierte C₆-C₁₄-Arylen-PR²-Gruppe ist bevorzugt eine perbromierte C₆-C₁₀-Arylen-PR²-Gruppe, und insbesondere eine p-Methyltetrabromphenyl-2-en- oder P-Ethyltetrabromphenyl-2-engruppe;
- die teilbromierte C₆-C₁₄-Arylen-PR²-Gruppe ist bevorzugt eine teibromierte C₆-C₁₀-Arylen-PR²-Gruppe, und insbesondere eine P-Methylbromphenyl-2-en- oder P-Ethylbromphenyl-2-engruppe.

Den Substituenten R¹, R^{1'} kommt im Rahmen der hier beschriebenen Carbenkomplexe die Aufgabe zu, ein Metallalkyliden zur Verfügung zu stellen, dass einerseits stabil, andererseits jedoch auch noch ausreichend metatheseaktiv ist. Besonders geeignete Substituenten R¹, R¹' sind daher neben R¹' = H große Alkyl- oder ArylReste die eine gute sterische Abschirmung des Metallalkylidens gewährleiten. Demzufolge ist es bevorzugt, wenn R¹ kein Wassertsoffatom ist. Besonders zweckmäßig ist das Kohlenstoffatom in R¹, das in direkter Nachbarschaft zum Metallalkyliden steht, ein quartäres Kohlenstoffatom, das keinen Wasserstoff-Substituenten aufweist. Als Substituenten dieses quartären Kohlenstoffatoms kommen unter anderem auch die für die Substituenten R² ausgeführten Reste in Betracht. Anhand dieser Vorgaben kann ein geeigneter Substituent R¹ fachmännisch ausgewählt werden. Es hat sich insbesondere als vorteilhaft herausgestellt, wenn R¹ in den Formeln I bis IV für *t*-Butyl, ein unsubstituiertes oder substituiertes Phenyl, wie 2-(2-Propoxy)phen-1-yl, 2-Methoxyphen-1-yl, 2,4,5-Trimethoxyphenyl, oder Ferrocenyl oder CMe₂Ph steht, wobei die Substituenten am Phenyl die gleiche Bedeutung haben können wie R², insbesondere aber 2-(2-Propoxy) oder 2-Methoxy bedeuten können. Zudem hat es sich als vorteilhaft herausgestellt, wenn als C₁-C₁₈-Alkylgruppe eine C₁-C₁₀-Alkylgruppe, und als C₆-C₁₄-Arylgruppe eine C₆-C₁₀-Arylgruppe herangezogen wird.

In einer bevorzugten Ausführungsform handelt es sich bei dem N-heterocyclischen Carbenkomplex um einen N-heterozyklischen Carbenkomplex der allgemeinen Formeln V oder VI 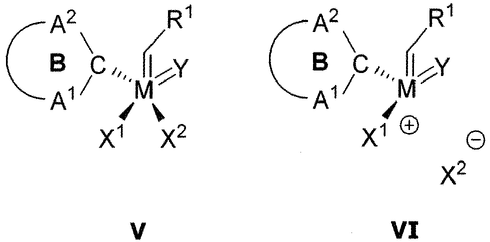
der dadurch gekennzeichnet ist, dass A¹ für NR² und A² für NR² oder S steht, C für ein Carbenoid-Kohlenstoffatom steht,
der Ring B ein 5 bis 7-gliedriger Ring ist, der neben A¹ und A² weitere Heteroatome in Form von Stickstoff oder Schwefel enthalten kann,
der Substituent R² für einen linearen oder verzweigten C₁-C₁₀-Alkyl-, einen linearen oder verzweigten C₂-C₁₀-Alkenyl-, insbesondere einen C₃-C₆-Cycloalkyl-, einen linearen oder verzweigten C₆-C₁₀₀-Polyoxaalkyl einen C₅-C₁₀-Aryl- oder einen C₅-C₁₀-Heteroaryl-Rest, einen C₅-C₁₀-Aryloxy-, einen linearen oder verzweigten C₁-C₁₀-Perfluoralkyl-, einen linearen oder verzweigten C₁-C₁₀-Perchloralkyl-, einen linearen oder verzweigten teilfluorierten C₁-C₁₀-Alkyl-, einen linearen oder verzweigten teilchlorierten C₁-C₁₀-Alkyl-, einen perfluorierten C₅-C₁₀-Aryl-, einen teilfluorierten C₅-C₁₀-Aryl-, einen perchlorierten C₅-C₁₀-Aryl- oder teilchlorierten C₅-C₁₀-Aryl-Rest steht, und, wenn A¹ und A² jeweils für NR² stehen, gleich oder verschieden sind,
M in den Formeln V oder VI für Cr, Mo oder W steht,
X¹ und X² in Formeln V oder VI gleich oder verschieden sind und aus der Gruppe umfassend C₁-C₁₈ Carboxylate, C₁-C₁₈-Alkoxylate, C₁-C₁₈ mono- oder polyhalogenierte Carboxylate, ein- oder mehrfach substituierte C₆-C₁₈-Mono-, Bi- oder Terphenolate, Trifluormethansulfonat, Tetrafluoroborat, Hexafluorophosphat und Hexafluoroantimonat ausgewählt sind, wobei die Substituenten an den Mono-, Bi- oder Terphenolaten neben Halogen die gleiche Bedeutung haben können wie R², Y in den Formeln V oder VI eine Oxo-Gruppe, ein N-Adamantyl- oder ein N-Aryl-Rest ist, wobei der Aryl-Rest ein- oder mehrfach mit Halogen, einem C₁-C₁₀ Alkyl-, C₁-C₁₀ Alkyloxy- oder Phenyl-Rest substituiert sein kann, und
R¹ in den Formeln I oder II ein aliphatischer oder aromatischer Rest, der vorzugsweise 4 bis etwa 20 Kohlenstoffatome aufweist, ist.

Für bevorzugte Ausführungsformen der Substituenten R , R², A¹, A², X¹, X² und Y in den Formeln V und VI gelten die vorstehenden Ausführungen analog.

Die erfindungsgemäßen Carbenkomplexe können zusätzlich zu den in den Formeln I bis IV, bzw V oder VI gezeigten Substituenten einen oder mehrere nicht geladenen Liganden aufweisen, der das Metallzentrum koordiniert. Die Funktion dieser nicht geladenen Liganden besteht darin, das Metallzentrum koordinativ weiter abzusättigen sowie den Metallkomplex zu stabilisieren. Die Liganden sind dabei Elektronen-Donoren und labil, d.h. sie können vom Metallzentrum abdissoziieren und durch Substrat ersetzt werden. Geeignete Liganden sind beispielsweise 1,2-Dimethoxyethan, Tetrahydrofuran, Acetonitril, Phosphine, wie Triphenylphosphin, Tri-n-butylphosphin oder Trimethylphosphin sowie Phosphite wie beispielsweise Trimethyl- oder Triethyl oder Triphenylphosphit.

Die im Vorstehenden beschriebenen erfindungsgemäßen Carbenkomplexe können in Lösung als Katalysator eingesetzt werden, es ist jedoch auch möglich, die Komplexe, beispielsweise mit Hilfe einer Abstandsgruppe, an einem festen Träger zu immobilisieren. Die Abstandsgruppe zwischen dem Trägermaterial und dem Ring B dient dazu, die Metallkomplexe am festen Träger zu fixieren. Die Abstandsgruppe muss dabei vorteilhafter Weise so beschaffen sein, dass der Metallkomplex einen hinreichenden Abstand zum Träger hat, so dass eine gute Zugänglichkeit der Substrate gewährleistet. Sie ist dermaßen beschaffen, dass sie zwei funktionelle Gruppen enthält mit denen eine Anbindung einerseits an den Katalysator, und andererseits an den Träger möglich ist. Der Abstand zwischen Träger und Katalysator sollte hingegen nicht so groß sein, dass bimetallische Reaktionen zwischen zwei immobilisierten Metallkomplexen stattfinden können. Weiter sollte die Abstandsgruppe so beschaffen sein, dass sie mittels einfacher chemischer Reaktionen sowohl mit dem Träger als auch mit dem Ring B oder dem Metallzentrum verbunden werden kann. Grundsätzlich kommen dabei alle aliphatischen oder aromatischen α,ω-difunktionalen Verbindungen in Frage.

Als geeignete feste Träger sind in diesem Zusammenhang insbesondere polymere Träger, wie solche auf Basis von Polystyrol/Poly(divinylbenzol) (PS-DVB), vernetzten Poly(methacrylat)en, vernetzten Poly(acrylamid)en aber auch vernetzten Po-ly(norbornen)en zu nennen. Der Träger hat die Aufgabe, die Anbindung des Katalysators, und dadurch eine heterogene Reaktionsführung zu ermöglichen. Der Träger weist dazu zweckmäßig eine mittlere Teilchengröße im Bereich von 2-1000 Mikrometern, vorzugsweise im Beriech von 20-200 Mikrometern, besonders bevorzugt im Bereich von 40-60 Mikrometern auf und kann porös oder nicht porös sein.

Als Abstandsgruppe kann in diesem Fall zweckmäßig eine C₁-C₂₀-Alkylenoxygruppe, C₁-C₂₀-α,ω-Dioxoalkylengruppe, eine C₁-C₂₀ α,ω-Diaminoalkylengruppe, eine C₁-C₂₀ α,ω-Dicarboxyl-alkylengruppe, eine C₆-C₁₈-Dioxoarylengruppe, eine C₆-C₁₈ -Diaminoarylengruppe oder eine Dicarboxy-C₆-C₁₈ -arylengruppe eingesetzt werden.

Wenn die Abstandsgruppe direkt an das Metallzentrum bindet, kann sie in den Formeln I - III einen X-Substituenten am Metall ersetzen. Alternativ kann die Abstandsgruppe in den Formeln III - IV auch mit einer P- oder N-haltigen Z-Gruppe verbunden sein. Beispiele für geeignete Abstandsgruppen sind in diesem Zusammenhang:
- eine lineare, teilcyclische oder verzweigte aliphatische α,ω-difunktionale C₁-C₂₀-Alkylengruppe, insbesonders eine lineare, teilcyclische oder verzweigte aliphatische α,ω-difunktionale C₁-C₁₀-Alkylengruppe, wobei die beiden funktionellen Gruppen gleich oder verschieden sein können und in Form von OH, NR'H, COOH, SH, SO₃H, SO₂H, PO₃H, PO₂H, Si(OR')OO, Si(OR')₂O vorliegen. R' kann in diesem Fall alle oben für R² genannten Bedeutungen haben, und insbesondere in Form von N-Methylpropargylsäure vorliegen, wobei die jeweiligen funktionellen Gruppen formell in der deprotonierten Form (anionisch) vorliegen.
- eine difunktionale halogenierte oder nicht-halogenierte C₆-C₁₄ aromatische Gruppe, vorzugsweise eine difunktionale halogenierte oder nicht-halogenierte C₆-C₁₀ aromatische Gruppe, wobei die beiden funktionellen Gruppen gleich oder verschieden sein können und in Form von OH, NR'H, COOH, SH, SO₃H, SO₂H, PO₃H, PO₂H, Si(OR')OO, Si(OR')₂O vorliegen. R' kann in diesem Fall alle oben für R² genannten Bedeutungen haben, und insbesondere in Form von p-Aminophenol, p-Aminosulfonsäure, Perfluoroaminosulfonsäure vorliegen, wobei die jeweiligen funktionellen Gruppen formell in der deprotonierten Form (anionisch) vorliegen.

In einer alternativen Ausführungsform kann es sich bei dem festen Träger auch um einen anorganischen Träger, wie beispielsweise einen Träger auf Basis von Glas, Siliziumdioxid, Zirkonoxid oder Titandioxid handeln. Anorganische Träger haben den Vorteil in der Gegenwart von Lösemitteln nicht zu quellen und stellen somit druckstabile Trägermaterialien dar, welche wiederum für kontinuierliche heterogene Reaktionsführungen vorteilhaft eingesetzt werden können. In diesem Fall kann als Abstandsgruppe zweckmäßig eine Amino-, Hydroxy-, Carboxyl- oder Thionyl-Alkylen-Si(O)₃, eine Amino-, Hydroxy-, Carboxyl- oder Thionyl-Alkylen-SiR(O)₂-Gruppe oder eine Amino-, Hydroxy-, Carboxyl- oder Thionyl-Alkylen-SiRR'O-Gruppe eingesetzt werden, bei für die Reste R und R' die gleichen Substituenten in Betracht kommen, die für den Substituenten R² im Vorstehenden genannt sind.

Die kovalente Anbindung des Rings B an den Träger kann unter Verwendung eines entsprechenden Vorläufers erfolgen, wie beispielsweise der protonierten Form des Rings B (der Ring wird am Carbenkohlenstoffatom protoniert) oder des Alkoxy- oder CO₂-geschützten Rings B, die sich mit Hilfe einer der in der Literatur beschriebenen Methoden (z.B. Adv. Synth. Catal. 2006, 348, 2101; Adv. Synth. Catal. 2010, 352, 917; Chem. Eur. J. 2013, 19, 11661; Adv. Synth. Catal. 2002, 344, 712; Macromol. Rapid. Commun. 2004, 25, 231) herstellen lassen. Das Carben im Ring B wird dann durch beispielsweise den Zusatz einer Base aus der protonierten Form des Rings B oder thermisch durch Abspaltung von beispielsweise CO₂ aus dem CO₂-geschützten Ring B generiert und mit Verbindungen der allgemeinen Formel M(Y)(CR¹R^{1'})X¹X^{2.}Lₓ in denen R¹, R², X¹ und X² die oben angegebenen Bedeutungen haben, L = ein neutraler Ligand ist und x einen Wert von 0 bis 2 annehmen kann, zur Reaktion gebracht. Die N-heterocyclischen Carbenkomplexe der Formeln I bis IV können in Abhängigkeit des Lösungsmittels und ihrer Zusammensetzung in der durch die Formeln I oder III bezeichneten neutral geladenen oder in der durch die Formeln II oder IV bezeichneten ionischen Form vorliegen.

Ein weiterer Aspekt der vorliegenden Erfindung befasst sich, wie vorstehend bereits angedeutet, mit der Verwendung der erfindungsgemäßen Carbenkomplexe gemäß den Formeln I bis IV als Katalysator in Olefinmetathesereaktionen und der Polymerisation von Alkinen bzw. Cyclopolymerisation von Diinen. Bei diesen Olefinmetathesereaktionen kann es sich um alle über Schrock-Carbenkomplexe katalysierbaren (asymmetrischen und desymmetrisierenden) Olefinmetathesereaktionen handeln, insbesondere um Ringschlussmetathesen, Kreuzmetathesen, Olefinolysen von ungesättigten Verbindungen, wie insbesondere die Ethenolyse von natürlich vorkommenden pflanzlichen Ölen und Fetten, Ring öffnende Kreuzmetathesen, (Kreuz-)-En-in-Metathesen, Ringschluss-En-in-Metathesen, Kreuz-En-diin-Metathesen sowie tandem-Ringöffnungs-Ringschluss-Metathesen handeln. Damit können Verbindungen erhalten werden, die von großer Bedeutung z.B. für die Pharma-, Agro-, Polymer-, Riechstoff- oder Aromaindustrie sind. Des Weiteren können sie für ringöffnende Metathesepolymerisationen (ROMP), 1-Alkinpolymerisationen, acyclische Metathesepolymerisationen (ADMET) oder Cyclopolymerisationen von α,ω-Diinen, eingesetzt werden. Die so hergestellten Polymere können z.B. als Matrixpolymere für Faser-Matrix-Komposite, als Kompatibilisierer oder Basispolymer für Fasern verwendet werden.

Als Substrate für diese Olefinmetathesereaktionen kommen prinzipiell alle Substrate in Betracht, die diesen Typen von Metathesereaktionen zugänglich sind. Beispielsweise können cyclische Olefine, wie Norborn-2-ene, Norbornadiene, Cyclooctene, Cyclooctadiene, Cyclooctatetraene und/oder Cyclopentene aber auch Alkine wie Acetylen oder 2-Butin zur Anwendung kommen. Diese Auflistung soll nicht beschränkend sein. So können auch folgende cyclische Olefine in Frage kommen: Cyclopropene, Cyclobutene, Dicyclopentadiene und Cyclohexene. Diese cyclischen Olefine können ein- oder mehrfach substituiert sein. Zudem können Olefine, wie z.B. Ethylen, Propylen sowie substituierte Butene, Pentene, Hexene, Heptene, Octene, und/oder Styrole und Diene, wie 1,3-Butadien, Pentadiene, Hexadiene, Heptadiene und Octadiene, im Rahmen der bezeichneten Olefinmetathesereaktionen umgesetzt werden. Substrate für die Olefinolyse, und insbesondere für die Ethenolyse von natürlich vorkommenden pflanzlichen Ölen und Fetten, sind allgemein Fettsäureester und inbesondere Rizinusöl, Palmöl oder Kokosnußöl in Kombination mit z.B. Ethylen oder Buten.

Ein besonderer Vorteil der erfindungsgemäßen Carbenkomplexe gemäß den Formeln I bis IV besteht darin, dass sich diese als besonders tolerant gegenüber funktionellen Gruppen erwiesen haben. Besonders hervorzuheben ist hier die Toleranz gegenüber Alkoholen, Carbonsäuren, Thioethern, Aminen und Aldehyden. So sind damit ohne Weiteres auch Olefinmetathesereaktionen von funktionalisierten Olefinen, insbesondere in Form von z.B. 5,6-Bis((pentyloxy)methyl)bicyclo[2.2.1]hept-2-en, 7-Oxabicyclo[2.2.1] hept-5-en-2,3-diylbis(methylene)diacetat, 4,4,5,5-Tetrakis-(ethoxycarbonyl)-1,7-octadiin, 2,2-Di(prop-2-yn-1-yl)propan-1,3-diol, Diallyldiphenylsilan, 2-(N-Cyclohexylmethyl)norborn-5-en, 2-(N,N-Dimethylaminomethyl)norborn-5-en), 1,7-Octadiin-4,5-dicarbonsäure, 1,6-Heptadiin-4-carbonsäure, Norborn-5-en-2-carbaldehyd, 4,4-Dicyano-1,6-heptadiin möglich.

Ein weiterer Vorteil der erfindungsgemäßen Carbenkomplexe gemäß den Formeln I bis IV besteht darin, dass diese in einigen Metathesereaktionen sehr hohe Wechselzahlen erlauben. Darüber hinaus werden bei der Cyclopolymerisation von Diinen mit Carbenkomplexen gemäß den Formeln I bis IV sehr hohe Stereo- und Regioselektivitäten beobachtet. Zudem werden z.B. bei Homometathesen mit Carbenkomplexen gemäß den Formeln I bis IV, die kleine N-heterocyclischer Carbene und großer Phenoxide aufweisen, hohe Z-Selektivitäten erzielt.

Als von besonderem Vorteil hat es sich im Rahmen der vorliegenden Erfindung herausgestellt, dass die erfindungsgemäßen Carbenkomplexe gemäß der Formeln II oder IV durch geeignete Wahl eines Lösungsmittels und ihrer Zusammensetzung in ionischer Form vorliegen. Dies erlaubt es, die Olefinmetathesereaktionen unter zweiphasigen Bedingungen durchzuführen, was sich vorteilhaft in einem niedrigen Metallgehalt der hergestellten Produkte auswirkt.

Die Durchführungen von Olefinmetathesereaktionen unter zweiphasigen Bedingungen erfolgt zweckmäßig in der Weise, dass die ionischen Carbenkomplexe gemäß der Formeln II oder IV in einem organischen Lösungsmittel I oder in einer ionischen Flüssigkeit gelöst werden. Neben dem Einsatz in klassischen 2-phasigen (flüssig-flüssig) Reaktionen kann diese Lösung in Form eines Films, der sehr dünn sein kann und vorzugsweise eine Dicke von 0,05 bis 200 µm, insbesondere 0,5 bis 10 µm, aufweist, auf ein Trägermaterial aufgebracht und mit diesem Träger in ein Reaktionsgefäß wie eine Reaktionskolonne eingebracht werden. Anschließend kann der mit der Katalysatorlösung beschichtete Träger mit ein oder mehreren Substraten in Kontakt gebracht werden, die gegebenenfalls ihrerseits in einem Lösungsmittel II gelöst sind, das mit dem Lösungsmittel I oder der ionischen Flüssigkeit für die Verbindung der Formeln II oder IV nicht mischbar ist. Wird ohne Lösungsmittel II gearbeitet, dürfen die Substrate sowie die im Rahmen der jeweiligen Reaktion entstehenden Produkte nur schwach mit dem Lösungsmittel I oder der ionischen Flüssigkeit mischbar sein. So sollte die maximale Löslichkeit vom Lösungsmittel II oder von den Substraten aber auch von den Produkten im Lösungsmittel I oder in der ionischen Füssigkeit <10 vol.-% betragen. Das Lösungsmittel II für die Substrate oder die Substrate selbst weisen in diesem Fall für die Katalysatorverbindung möglichst ungünstige Lösungseigenschaften auf, damit sich diese nicht in substantieller Menge in dem Substratlösungsmittel lösen kann.

Bei dem Reaktionsgefäß handelt es sich sinnvollerweise, aber nicht zwangsläufig, um eine Reaktionskolonne, die für die Substratlösung einen Einlass sowie am entgegengesetzten Ende der Reaktionskolonne einen Auslass aufweist. Die vorstehende Reaktionsführung wird in der Fachterminologie auch als *"supported ionic liquid phase"* (SILP)-Technologie bezeichnet (sh. Topics in Catalysis, 2006, 40, 91).

Als organische Lösungsmittel I für die Carbenkomplexe gemäß der Formeln II oder IV können insbesondere polare aprotische Lösungsmittel wie z. B. Dimethylformamid, Dimethylacetamid oder Dimethylsulfoxid verwendet werden.

Geeignete ionische Flüssigkeiten im Zusammenhang mit der vorliegenden Erfindung sind insbesondere Verbindungen der allgemeinen Formel [Q⁺]ₙ [Z]ⁿ⁻, wobei das Kation [Q⁺]ₙ ein quaterniertes Ammonium-[R¹R²R³R⁴N⁺], Phosphonium-[R¹R²R³R⁴P⁺] oder Sulfonium-[R¹R²R³S⁺]-Kation oder ein analoger quaternierter Stickstoff-, Phosphor- oder Schwefel-Heteroaromat der folgenden Formeln 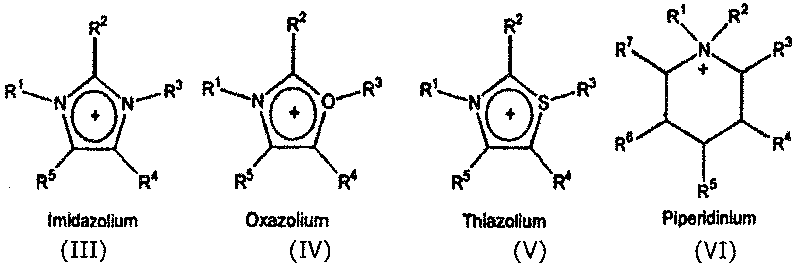 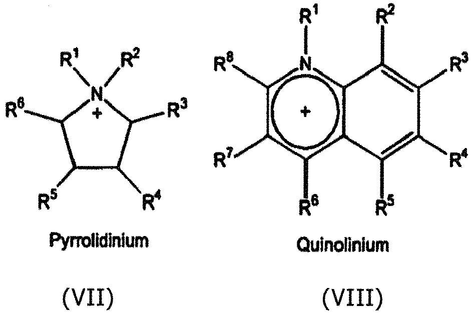 ist, und die Reste R¹, R², R³, R⁴ bzw. die Reste R¹ bis R⁸ in den Formeln (III) bis (VIII), unabhängig voneinander, lineare, cyclische, verzweigte, gesättigte oder ungesättigte Alkylreste, mono- oder polycyclische, aromatische oder heteroaromatische Reste oder mit weiteren funktionellen Gruppen substituierte Derivate dieser Reste sind. Dabei können die Reste R¹, R², R³ und R⁴ untereinander verbunden sein. Das Anion [Z]ⁿ⁻ liegt vorzugsweise in Form eines Carboxylats, Halogenids, Pseudohalogenids, Amids, oder in Form von Bor-, Phosphor- oder Nitroverbindungen vor.

Besonders geeignete ionische Flüssigkeiten sind insbesondere Imidazoliumsalze, bevorzugt ausgewählt aus der Gruppe umfassend 1-Ethyl-3-methylimidazolium-salze, 1,3-Dimethylimidazoliumsalze, 1,2,3-Trimethylimidazoliumsalze, 1-Butyl-3-methylimidazoniumsalze und 1-Butyl-2,3-dimethylimidazoliumsalze.

Bei dem mit dem Lösungsmittel I oder der ionischen Flüssigkeit für die Verbindung der Formeln II oder IV nicht mischbaren Lösungsmittel II handelt es sich vorzugsweise um einen aliphatischen oder aromatischen Kohlenwasserstoff, insbesondere um Toluol, Xylol, Pentan, Hexan, Heptan, Oktan, Trichlorbenzol oder Chlorbenzol oder Gemische davon. In Kombination mit den ionischen Komplexen gemäß Formel II und IV ist damit sichergestellt, dass sich diese Komplexe nicht im Lösungsmittel II lösen und so ein Ausbluten des Katalysators bei kontinuierlicher Reaktionsführung verhindert wird.

Als Trägermaterialien für die Lösungsfilme der Carbenkomplexe gemäß der Formeln II oder IV eignen sich im Zusammenhang mit der SILP-Technologie besonders anorganische Trägermaterialien, insbesondere auf Basis von Glas, Zirkonoxid, Titandioxid, Siliziumdioxid, oder polymerorganische Trägermaterialien, insbesondere in Form von polymerorganischen monolithischen Trägermaterialien, z. B. auf Basis von Poly(styrol)/Poly(divinylbenzol), Poly(methacrylat)en, Poly(acrylamid)en oder vernetzten Poly(norbornen)en oder Poly(cycloocten)en. Diese Trägermaterialien haben den Vorteil, dass sie nicht oder nur sehr wenig quellen und somit bei kontinuierlicher Reaktionsführung keine hohen Gegendrücke aufbauen. Eine kontinuierliche Umsetzung der Substrate ist deshalb von Vorteil, weil dabei ein oder mehreren Substrate kontinuierlich in das Reaktionsgefäß geleitet und die daraus resultierenden Reaktionsprodukte aus diesem kontinuierlich abgeführt werden was wiederum zu höheren Wechselzahlen führen kann. Auf diese Weise können Olefinmetathesereaktionen unter kontinuierlichen zweiphasigen Bedingungen durchgeführt werden (sh. Chem. Eur. J. 2012, 18, 14069).

Die vorstehenden Angaben zur Umsetzung von Substraten mit Carbenkomplexen gemäß der Formeln II oder IV, die in einem organischen Lösungsmittel oder in einer ionischen Flüssigkeit gelöst wird und in Form eines Films auf ein geeignetes Trägermaterial aufgebracht wird, betrifft gleichermaßen entsprechende Verwendungen dieser Verbindungen und Verfahren, die gemäß diesen Vorgaben durchgeführt werden.

Die Carbenkomplexe gemäß den Formeln I bis IV weisen eine wünschenswert hohe Reaktivität in Olefinmetathesereaktion, der 1-Alkinpolymerisation und der Cyclopolymerisation von Diinen auf und besitzen eine signifikant verbesserte Toleranz gegenüber funktionellen Gruppen als bisherige Gruppe VI Metallalkyliden-Komplexe. So sind beispielsweise Vertreter der erfindungsgemäßen N-heterozyklischen Carbenkomplexe nach einer der Formeln I bis IV in Gegenwart von Aldehyden, sekundären Aminen, Carbonsäuren, Nitrilen und Alkoholen stabil. Aufgrund dieser im Vergleich zu bekannten Schrock-Carben-Komplexen von Metallen der Gruppe VI deutlich erhöhten Funktionstoleranz wird das Einsatzspektrum in Olefinmetathesereaktionen deutlich verbreitert.

Die Erfindung soll nachfolgend anhand von Beispielen näher erläutert werden. Vorangestellt sind einige allgemeine Betrachtungen:
Falls nichts anderes ausgeführt, wurden alle Reaktionsschritte in der Abwesenheit von Sauerstoff und Feuchtigkeit unter N₂ oder Ar entweder mittels Schlenktechnik oder in Schutzgasboxen (MBraun LabMaster 130) in trockener Glasausrüstung durchgeführt. Das deuterierte Lösungsmittel CD₂Cl₂ wurde über P₂O₅ getrocknet und Vakuum-transferiert, Benzol wurde über Na getrocknet und destilliert. Toluol, Diethylether, THF und CH₂Cl₂ wurden mittels eines Lösungsmittelreinigungssystems (SPS, MBraun) gereinigt. Kommerziell erhältliche Reagenzien und das verwendete d₆-DMSO und CDCl₃ wurden ohne weitere Aufreinigung verwendet.

Die NMR-Spektren wurden mit Hilfe eines Bruker 400-Spektrometers (400 MHz für Proton, 101 MHz für Kohlenstoff und 376 MHz für Fluor) bei 20°C aufgenommen, auf die internen Lösungsmittel Restsignale kalibriert. Die Verschiebungen der Signale und sind in ppm angegeben. Die IR-Spektren wurden auf einem Bruker-Vektor 22 mittels ATR-Technologie aufgenommen. Die Molmassen und - verteilungen wurden mittels Hochtemperaturgelpermeationschromatographie (HT-GPC) auf drei konsekutiven Waters Styragel HR4 4.6x300 mm-Säulen in Trichlorbenzol bei 145°C auf einem PSS HAT-GPC-System aufgenommen. Die Flussrate betrug 1 ml/min. Eng verteilte Polystyrolstandards im Bereich von 162 < Mₙ < 6035000 g·mol⁻¹ (Easi Vial-red, gelb und grün) von Polymer Laboratories kamen zur Anwendung.

Die im Folgenden beschriebenen Beispiele 1 bis 16, 34 bis 36 und 38 bis 53 betreffen die Herstellung von Carbenkomplexen gemäß den Formeln I bis IV, während sich die weiteren Beispiele 17 bis 33, 37 und 55 bis 57 mit Olefinmetathesereaktionen mit Hilfe der erfindungsgemäßen Carbenkomplexe befassen.

### Beispiele

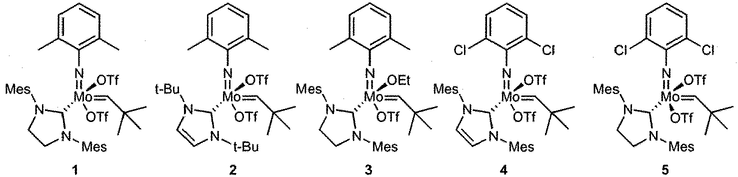

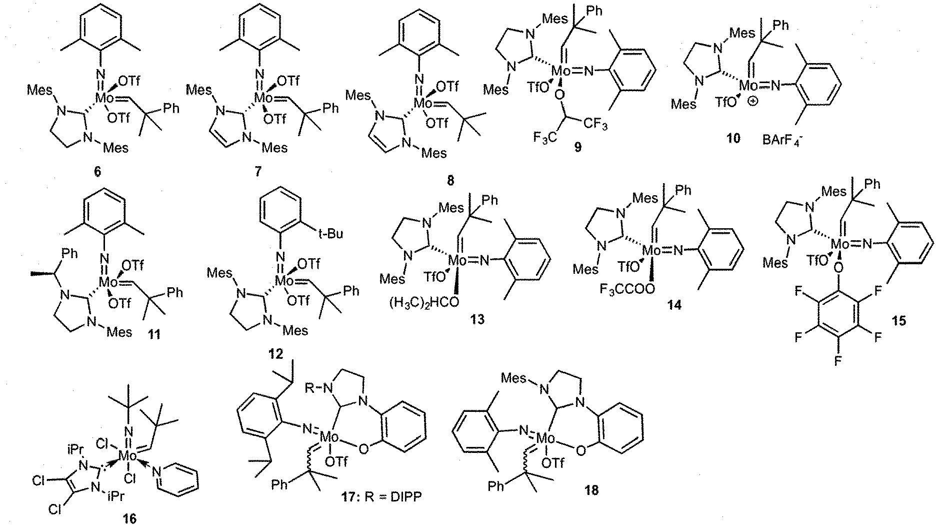

Struktur ausgewählter Mo-Katalysatoren. DIPP = 2,6-di(2-propyl)phen-1yl.
Beispiel 1 (Herstellung von Mo(N-2,6-Me₂-C₆H₃)(IMesH₂)(CH-tBu)(OTf)₂) (1) Mo(N-2,6-Me₂-C₆H₃)(CH-tBu)(OTf)₂ (DME) (0,300 g, 0,445 mmol) wurde in 8 mL Benzol gelöst und mit einer Lösung von 1,3-Bis(2,4,6-trimethylphenyl)-2-imidazolinylidene (0,136 g, 0,445 mmol) in 5 mL Benzol versetzt. Die Reaktionslösung wurde für drei Stunden gerührt, das Benzol abdekantiert und der Rückstand mit Benzol gewaschen. Ausbeute: 0,32 g (81%, gelbes Pulver). Kristallines Material konnte durch Umkristallisation aus CH₂Cl₂ gewonnen werden. ¹H NMR (CD₂Cl₂): δ (*syn*-Isomer, 99,9%) 12,76 (s, 1, C*H*CMe₃, *J*_{CH}=118 Hz), 7,06-6,61 (7H, Ar*H*), 3,98 (4H, C*H*₂NC), 2,69-1,71 (24H, Me), 0,93 (s, 9H, CH₂C*Me₃*); ¹⁹F NMR (CD₂Cl₂): δ -74,65 (SO₃CF₃), -76,7 (SO₃CF₃). ¹³C NMR (CD₂Cl₂): δ 320,9 (CH-*t*Bu), 208,7 (*C*N_{carben}), 154,6 (*Cᵢₚₛₒ*), 140,4 (*Cₒᵣₜₕₒ*)*,* 137,1 (C_{aryl}), 136,8 (C_{aryl}), 135,7 (C_{aryl}), 131,1 (*C*H_{aryl}), 130,5 (*C*H_{aryl}), 130,1 (*C*H_{aryl}), 128,2(*C*_{ary}l), 120,2 (q, CF₃, *J* = 319 Hz), 119,8 (q, CF₃, *J* = 320 Hz), 53,1 (*C*Me₃), 50,7 (*C*H₂-_{imidazolylene}), 30,5 (C*Me₃),* 21,3 (*C*H₃), 19,0 (*C*H₃), 18,9 (*C*H₃); Anal. ber. für C₃₆H₄₅F₆MoN₃O₆S₂·CH₂Cl₂: C, 45,54; H, 4,96; N, 4,31. Gefunden: C, 45,52; H, 4,75; N, 4,37.
Beispiel 2 (Herstellung von Mo(N-2,6-Me₂-C₆H₃)(I-*t*Bu)(CH-*t*Bu)(OTf)₂) **(2)** Mo(N-2,6-Me₂-C₆H₃)(CH-*t*Bu) (OTf)₂(DME) (0,100 g, 0,148 mmol) wurde in 3 mL Benzol gelöst. 1,3-Di-*t*-butylimidazol-2-ylidene (0,027 g, 0,15 mmol), ebenfalls gelöst in Benzol, wurde unter Rühren zugegeben. Nach dreistündigem Rühren wurde vom Niederschlag abdekantiert und der Rückstand mit Benzol gewaschen. Ausbeute: 0,060 g (65%, gelbes Pulver). Kristallines Material konnte durch Umkristallisation aus CH₂Cl₂ erhalten werden. ¹H NMR (CD₂Cl₂): *δ* 14,60 (s, 1H, C*H*CMe₃, *J*_{CH} = 121 Hz, *syn*-Isomer), 7,12-6,95 (3H, Ar*H*), 2,60 (2H, C*H*NC), 1,80-1,67 (24H, Me), 1,32 (s, 9H, CH₂C*Me₃*); ¹⁹F NMR (CD₂Cl₂): *δ* -77,68, -77,69, - 77,70, -77,71 (CF₃SO₃), -78.06, -78.07, -78.08, -78.09 (CF₃SO₃); ¹³C NMR (CD₂Cl₂): *δ* 329.6 (*C*H-*t*Bu), 175.4 (*C*N_{carben}), 154.3 (C*ᵢₚₛₒ*), 142.2 (C*_{aryl}*), 136.9 (*C*_{aryl}), 129.7 (*C*H_{aryl}), 129.6 (*C*H_{aryl}), 128.9 (*C*H_{aryl}), 121.7 (*C*_{C=C}), 120.6 (*C*_{C=C}), 119.8 (q, *C*F₃, *J* = 318 Hz), 119.7 (q, *C*F₃, *J* = 319 Hz), 61.7 (N*C*Me₃), 61.3 (*C*Me₃), 32.8 (C*Me₃),* 30.5 (*CMe₃),* 30.1 (C*Me₃),* 21.1 (*C*H₃), 18.4 (*C*H₃). Anal. ber. für C₂₆H₃₉F₆MoN₃O₆S₂: C, 40.84; H, 5.27; N, 5.50. Gefunden: C, 40.88; H, 5.20; N, 5.56.
Beispiel 3 (Herstellung von N-2,6-Me₂-C₆H₃)(IMesH₂)(CHCMe₃)(OTf)(OEt)) **(3)** Natriumethoxid (0.0120 g, 0.1842 mmol) wurde in 5 mL Diethylether:THF, 1:1, gelöst. Dann wurde Mo(N-2,6-Me₂-C₆H₃)(IMesH₂)(CH-*t*Bu)(OTf)₂ (0,080 g, 0,090 mmol) zugegeben. Nach zweistündigem Rühren wurde das Lösungsmittel entfernt, der Rückstand in 5 mL Dichlormethan gelöst und durch Celit filtriert. Umkristallisation aus Dichlormethan ergab gelbes kristallines Material in 40% Ausbeute. ¹H NMR (CD₂Cl₂): *δ* 12,30 (s, 1H, CHCMe₃), 6,94-6,65 (7H, ArH), 4,15 (4H, CH₂NC), 3,69 (2H, OCH*₂*CH₃), 2,53-2,24 (24H, Me), 1,82 (3H, OCH₂CH₃), 1,14 (s, 9H, CH₂CMe₃); ¹⁹F NMR (CD₂Cl₂): *δ*-79,05 (CF₃SO₃).
Beispiel 4 (Herstellung von Mo(N-2,6-Cl₂-C₆H₃)(CHCMe₃)(OTf)₂(IMes)) **(4):** In der Glove Box wurde Mo(N-2,6-Cl-C₆H₃)(CHCMe₃)(OTf)₂(DME) (0,432 g, 0,605 mmol) in einem 25 mL Schlenk-Kolben vorgelegt. Der Komplex wurde in 15 mL Toluol gelöst und 30 min bei -40°C gekühlt. 1,3-Dimesitylimidazol-2-yliden (0,184 g, 0,605 mmol, 1 Äquiv.) wurde in 3 mL Toluol gelöst und ebenfalls gekühlt. Unter Rühren wurde die kalte NHC-Lösung tropfenweise zum Metallkomplex gegeben. Die Farbe änderte sich langsam zu Dunkelorange. Die Reaktionsmischung wurde 2 h bei Raumtemperatur gerührt. Nach einigen Minuten setzte eine Trübung ein und es bildete sich ein Niederschlag. Anschließend wurde das Lösemittel auf ca. 1/3 eingeengt und die Suspension für 30 min kaltgestellt. Der ausgefallene Feststoff wurde abfiltriert und mit etwas kaltem Toluol gewaschen. Das Rohprodukt wird als gelber Feststoff erhalten und kann aus Dichlormethan umkristallisiert werden (0,450 g, 80%). ¹H NMR (400 MHz, CD₂Cl₂): δ = 1,12 (s, 9H, *t*Bu), 2,10 (s, 6H, o-Mes-Me), 2,11 (s, 6H, o-Mes-Me), 2,24 (s, 6H, *p*-Mes-Me), 6,68 (s, br, 2H, Mes-Ar), 6,98 (s, br, 2H, Mes-Ar), 7,14 (m, 3H, Ar), 7,22 (s, 2H, N-CH-CH-N), 12,94 (s, 1H, Mo=CH); ¹³C NMR (100 MHz, CD₂Cl₂): δ = 18,9 (o-Mes-Me), 19,0 (o-Mes-Me), 21,3 (*p*-Mes-Me), 31,4 (CMe₃), 50,6 (CMe₃), 124,4, 126,4, 128,3, 129,5, 130,3, 130,9, 134,7, 135,9, 136,3, 136,5, 141,0 (*ipso*-Mes), 149,9 (*ipso*-Imido), 185,2 (N-C-N), 327,4 (Mo=CH, *J*_{C-H} = 119,5 Hz); ¹⁹F NMR (375 MHz, CD₂Cl₂): δ = -75,07, -76,56.
Beispiel 5 (Herstellung von Mo(N-2,6-Cl₂-C₆H₃)(CHCMe₃)(OTf)₂(IMesH₂)) **(5).** In der Glove Box wurde Mo(N-2,6-Cl-C₆H₃)(CHCMe₃)(OTf)₂(DME) (0,198 g, 0,277 mmol) in einem 25 mL Schlenk-Kolben vorgelegt. Der Komplex wurde in 15 mL Toluol gelöst und 30 min bei -40°C gekühlt. 1,3-Dimesitylimidazolin-2-yliden (0,085 g, 0,277 mmol, 1 Äquiv.) wurde in 3 mL Toluol gelöst und ebenfalls gekühlt. Unter Rühren wurde die kalte NHC-Lösung tropfenweise zum Metallkomplex gegeben. Die Farbe änderte sich langsam zu dunkelorange. Die Reaktionsmischung wurde 2 h bei Raumtemperatur gerührt. Nach einigen Minuten setzte eine Trübung ein und es bildete sich ein Niederschlag. Anschließend wurde das Lösemittel auf ca. 1/3 eingeengt und die Suspension für 30 min kaltgestellt. Der ausgefallene Feststoff wurde abfiltriert und mit etwas kaltem Toluol gewaschen. Das Rohprodukt wird als gelber Feststoff erhalten und kann aus Dichlormethan umkristallisiert werden (0,185 g, 72 %).
Beispiel 6 (Herstellung von Mo(N-2,6-Me₂-C₆H₃)(IMesH₂)(CHCMe₂Ph)(OTf)₂) **(6):** Mo(N-2,6-Me₂-C₆H₃)(CHCMe₂Ph)(OTf)₂(DME) (0,20 g, 0,2720 mmol) wurde in 8 mL Benzol vorgelegt: 1,3-Bis(2,4,6-trimethylphenyl)-2-imidazolidin-2-yliden (0,0830 g, 0,2720 mmol) wurde in 1 mL Benzol gelöst und zugetropft. Hierbei konnte ein schneller Farbumschlag von gelb nach dunkelrot bei gleichzeitiger Niederschlagsbildung beobachtet werden. Nach drei Stunden Rühren wurde das Benzol anschließend abdekantiert, der Rückstand mit Benzol gewaschen und im Vakuum getrocknet. Das Produkt konnte als gelber Feststoff isoliert werden (0,15 g, 81 %). Alternativ kann der gelbe Feststoff in einer minimalen Menge an Dichlormethan gelöst und bei -30 °C für 24 h kristallisiert werden, wobei ein kristallines gelbes Material mit 69 % Ausbeute erhalten wird. ¹H NMR (CD₂Cl₂): *δ*= 13,11 (s, 1H, CHCMe₂Ph, *J*_{CH} = 114 Hz), 7,19-6,95 (m, 9H, ArH), 6,51 (s, 2H, ArH), 3,97 (s, 4H, C*H*NC), 2,69-1,71 (s, 27H, Me), 1,25 (s, 3*H*, CHC*Me₂*Ph) ppm; ¹⁹F NMR (CD₂Cl₂): *δ* = -74,59 (s, CF₃SO₃, *trans* zum NHC-Ligand), -76,53 (s, CF₃SO₃); ¹³C NMR (CD₂Cl₂): *δ* = 317,4 (CHCMe₃), 208,7 (CN_{carben}), 154,6 (Cᵢₚₛₒ), 149,0, 140,4 (Cₒᵣₜₕₒ), 137,0 (C_{aryl}), 136,4 (C_{aryl}), 135,6 (C_{aryl}), 130,9 (C_{aryl}), 130,5 (C_{aryl}), 130,2 (C_{aryl}), 128,4 (C_{aryl}), 128,2 (C_{aryl}), 126,9 (C_{aryl}), 125,9 (C_{aryl}), 121,6 (q, CF3, J = 319 Hz), 118,5 (q, CF3, J = 320 Hz), 56,8 (CMe₂Ph), 53,1 (CH_{2-Imidazolyliden}), 32,9 (CMe₂Ph), 29,6 (CMe₂Ph), 21,3 (CH₃), 19,0 (CH₃), 18,9 (CH₃); Elementaranalyse: C₄₁H₄₇F₆,MoN₃O₆S₂; berechnet: C 51,68, H 5,02, N 4,41; gefunden: C 51,81, H 4,88, N 4,35.
Beispiel 7 (Herstellung von Mo(N-2,6-Me₂-C₆H₃)(IMes)(CHCMe₂Ph)(OTf)₂) **(7):** Mo(N-2,6-Me₂-C₆H₃)(CHCMe₂Ph)(OTf)₂(DME) (0,1500 g, 0,204 mmol) wurde in 6 mL Benzol gelöst und zu der vorgelegten Lösung von 1,3-Bis(2,4,6-trimethylphenyl)imidazol-2-yliden (0,0620 g, 0,2040 mmol) in einem 1 mL Benzol zugegeben. Es erfolgte ein sofortiger Farbumschlag von gelb nach dunkelrot bei gleichzeitiger Niederschlagsbildung. Die Reaktionsmischung wurde für drei Stunden gerührt und anschließend das Lösungsmittel abdekantiert. Der Rückstand wurde mit Benzol gewaschen und im Vakuum getrocknet. Es wurde ein gelber Feststoff erhalten (0,13 g, 85%). Das gelbe Produkt kann aus einer minimalen Menge an Dichlormethan bei -30 °C umkristallisiert werden (65 %). ¹H NMR (CD₂Cl₂): *δ* = 13,18 (s, 1H, CHCMe₂Ph, *J*_{CH} = 118 Hz), 7,21-6,95 (m, 9H, ArH), 6,56 (s, 2H,), 4,29 (s, 2H, C*H*NC), 2,60-1,97 (s, 27H, Me), 1,29 (s, 3H, CHC*Me*₂Ph) ppm; ¹⁹F NMR (CD₂Cl₂): *δ* = -74,92 (s, CF₃SO₃, *trans* zum NHC-Ligand), -76,53 (s, CF₃SO₃); ¹³C NMR (CD₂Cl₂): *δ* = 317,0 (*C*HCMe₃), 184,3 (CN_{Carben}), 154,8 (Cᵢₚₛₒ), 149,0, 141,3 (Cₒᵣₜₕₒ), 136,4 (C_{aryl}), 135,9 (C_{aryl}), 135,5 (C_{aryl}), 130,6 (C_{aryl}), 130,1 (C_{aryl}), 130,0 (C_{aryl}), 128,6 (C_{aryl}), 128,2 (C_{aryl}), 126,9 (C_{aryl}), 126,4 (C_{aryl}), 125,9 (C_{aryl}), 121,6 (C_{C=C}), 121,4 (C_{C=C}), 118,4 (q, CF3, J = 318 Hz), 118,3 (q, CF3, J = 319 Hz), 56,8 (CMe₂Ph), 33,2 (CMe₂Ph), 29,8 (CMe₂Ph), 21,4 (CH₃), 20,6 (CH₃), 18,7 (CH₃); Elementaranalyse: C₄₁H₄₅F₆MoN₃O₆S₂; berechnet: C 51,79, H 4,88, N 4,42; gefunden: C 51,73, H 4,80, N 4,39.
Der Katalysator Mo(N-2,6-Me₂-C₆H₃)(IMes)(CH-tBu)(OTf)₂) **(8)** wurde analog Beispiel 2 hergestellt, wobei anstelle von 1,3-Di-t-butylimidazol-2-yliden eine entsprechende Menge 1,3-Bis(2,4,6-trimethylphenyl)imidazol-2-yliden verwendet wurde.
Beispiel 8 (Herstellung von Mo(N-2,6-Me₂-C₆H₃)(IMesH₂)(CHCMe₂Ph)(OTf)(OCH (CF₃)₂)) **(9):**
Mo(N-2,6-Me₂-C₆H₃)(CHCMe₂Ph)(OTf)₂(DME) (0,0400 g, 0,0420 mmol) wurde in einer minimalen Menge (∼2 mL) C₂H₄Cl₂ gelöst, die Lösung auf -30°C abgekühlt und anschließend LiOCH(CF₃)₂ (0,0050 g, 0,0420 mmol) zugegeben. Die Reaktionsmischung wurde zwei Stunden bei Raumtemperatur gerührt und nachfolgend über Celit abfiltriert. Nach Entfernung des Lösungsmittels im Vakuum wurde ein gelber Feststoff erhalten. Das Rückstand wurde in einer minimalen Menge Dichlormethan gelöst und für mehrere Tage bei -30°C kristallisiert, um gelbe Kristalle (63 %) zu erhalten. ¹H NMR (CD₂Cl₂): *δ* = 13,49 (s, 1H, C*H*CMe₂Ph, *J*_{CH} = 114 Hz), 7,28-6,41 (m, 14H, Ar*H*), 3,97-3,82 (m, 4H, C*H*₂NC), 2,32-1,79 (s, 30H, Me); ¹³C NMR (CD₂Cl₂): *δ* 323,8 (*C*HCMe₃), 210,0 (CN_{Carben}), 155,2 (Cᵢₚₛₒ), 150,7, 139,5 (Cₒᵣₜₕₒ), 136,9 (C_{aryl}), 135,8 (C_{aryl}), 135,2 (C_{aryl}), 130,1 (C_{aryl}), 129,9 (C_{aryl}), 129,1 (C_{aryl}), 128,6 (C_{aryl}), 127,8 (C_{aryl}), 126,6 (C_{aryl}), 125,8 (C_{aryl}), 121,6 (CF₃), 118,4 (q, CF₃), 76,07-75,11 (q, O*C*H(CF₃)₂), 56,1 (CMe₂Ph), 51,9 (CH_{2Imidazolyden}), 37,2 (CMe₂Ph), 29,3 (CMe₂Ph), 21,5 (CH₃), 21,3 (CH₃), 19,0 (CH₃), 18,9 (CH₃); ¹⁹F NMR (CD₂Cl₂): *δ* = -73,07-73,14 (q, CF₃), -77,33-73,40 (q, CF₃), -78,07 (s, CF₃SO₃, *trans* zum NHC-Ligand). Elementaranalyse: C₄₄H₅₀Cl₂F₉MoN₃O₄S; berechnet: C 50,05, H 4,87, N 3,98; gefunden: C 50,51, H 4,85, N 4,07.
Beispiel 9 (Herstellung von [Mo(N-2,6-Me₂-C₆H₃)(CHCMe₂Ph)(OTf)(IMesH₂)⁺ B(3,5-(CF₃)₂-C₆H₃)₄⁻]) **(10):** [Ag⁺B(3,5-(CF₃)₂-C₆H₃)₄⁻] (0,0874 g, 0,0879 mmol) wurde in 1 mL C₂H₄Cl₂ gelöst und bei -30 °C zu einer Lösung von Mo(N-2,6-Me₂-C₆H₃)(CHCMe₂Ph)(OTf)₂(DME) (0,08370 g, 0,0879 mmol) in 2 mL C₂H₄Cl₂ zugegeben. Die Reaktionsmischung wurde über Nacht gerührt, anschließend über Celit gefiltert und das Lösungsmittel im Vakuum entfernt. Der gelbe Rückstand wurde in minimaler Menge Dichlormethan aufgenommen und bei -30 °C für mehrere Tage gelagert um das Produkt in Form gelber Kristalle in ∼60 % Ausbeute zu isolieren. ¹H NMR (CD₂Cl₂): *δ* = 12,90 (s, 1H, C*H*CMe₂Ph, *J*_{CH} = 127 Hz), 7,72-6,97 (m, 20H, Ar*H*), 4,06 (s, 4H, C*H*₂NC), 2,37-0,92 (s, 33H, Me); ¹³C NMR (CD₂Cl₂): *δ* = 325,0 (*C*HCMe₃), 206,7 (CN_{carben}), 163,1-162,59 (q, ¹*J*_{B-C} = 50 Hz), 154,0 (Cᵢₚₛₒ), 144,3, 143,2, 142,4, 141,3 (Cₒᵣₜₕₒ), 137,1 (C_{aryl}), 135,4 (C_{aryl}), 132,5 (C_{aryl}), 131,6 (C_{aryl}), 130,6 (C_{aryl}), 130,0 (C_{aryl}), 129,6 (C_{aryl}), 129,2 (C_{aryl}), 129,0 (C_{aryl}) 128,8 (C_{aryl}), 128,6 (C_{aryl}), 127,8 (C_{aryl}), 126,7 (C_{aryl}), 126,3 (C_{aryl}), 123,8, 121,1, 118,1, 57,5 (CMe₂Ph), 52,8 (CH_{2Imidazolyden}), 28,8 (CMe₂Ph), 21,4 (CH₃), 21,3 (CH₃), 20,7 (CH₃), 19,8 (CH₃), 18,7 (CH₃), 18,0 (CH₃). ¹⁹F NMR (CD₂Cl₂): *δ* = -62,89 (s, 3F), - 75,66 (s, CF₃SO₃, *trans* zum Imidoligand).
Beispiel 10 (Herstellung von Mo(N-2,6-Me₂-C₆H₃)(3-mesityl-1-(1-phenylethyl)-imidazolin-2-ylidene)(CHCMe₂Ph)(OTf)₂) **(11):** 3-Mesityl-1-(1-phenylethyl)-4,5-dihydro-1H-imidazol-3-iumtetrafluoroborat (0,0820 g, 0,2160 mmol) wurde in 2 mL Benzol suspendiert. Zu der Suspension wurde unter Rühren KHMDS (0,0430 g, 0,2160 mmol) zugegeben. Nach einer Stunde Reaktionszeit wurde die klare Benzollösung über Celit gefiltert. Mo(N-2,6-Me₂-C₆H₃)(CHCMe₂Ph)(OTf)₂·(DME) (0,1580 g, 0,2160 mmol; J. Organomet. Chem. 1993, 459, 185) wurde in 8 mL Benzol gelöst und die Lösung für 15 Minuten gerührt. Zu dieser wurde anschließend die zuvor gefilterte Benzollösung des freien NHCs zugegeben, wobei ein sofortiger Farbumschlag von gelb nach dunkelrot zu beobachten war. Nach drei Stunden Rühren wurde das Benzol entfernt, der gelbe Rückstand mit *n*-Pentan gewaschen und im Vakuum getrocknet (0,0110 g, 85 %). Der Rückstand wurde in minimaler Menge Dichlormethan gelöst und für mehrere Tage bei -30 °C gelagert, um gelbe Kristalle (60 %) zu erhalten. ¹H NMR (CD₂Cl₂): *δ* = 14,73 (s, 1H, C*H*CMe₂Ph), 7,36-6,99 (m, 14H, Ar*H*), 6,29 (s, 1H, ArMes), 4,08-3,80 (m, 4H, C*H*₂NC), 2,46-1,39 (s, 24H, Me); ¹⁹F NMR (CD₂Cl₂): *δ* = - 77,06 (s, CF₃SO₃), -77,77 (s, CF₃SO₃, *trans* zum NHC-Ligand); Elementaranalyse berechnet für C₄₁H₄₇Cl₂F₆MoN₃O₆S₂; C 48,14, H 4,73, N 4,10; gefunden: C 48,17, H 4,68, N 4,06.
Beispiel 11 (Herstellung von Mo(N-2,6-Me₂-C₆H₃)(IMesH₂)(CHCMe₃)(OTf)(OCH (CH₃)₂)) **(13):**
Mo(N-2,6-Me₂-C₆H₃)(IMesH₂)(CHCMe₃)(OTf)₂ (0,080 g, 0,0900 mmol wurde in einer minimalen Menge (∼2 mL) C₂H₄Cl₂ gelöst, die Lösung auf -30°C abgekühlt und anschließend LiOCH(CH₃)₂ (0,0050 g, 0,0900 mmol) zugegeben. Die Reaktionsmischung wurde zwei Stunden bei Raumtemperatur gerührt, anschließend über Celit gefiltert und das Lösungsmittel im Vakuum entfernt. Der gelbe Rückstand wurde in minimaler Menge Dichlormethan aufgenommen und bei -30°C für mehrere Tage gelagert um das Produkt in Form gelber Kristalle in ∼63 % Ausbeute zu isolieren.
Der Katalysator Mo(N-2,6-Me₂-C₆H₃)(IMesH₂)(CHCMe₃)(OTf)(OOCCF₃) **(14)** wurde analog Beispiel 11 hergestellt, wobei anstelle von LiOCH(CH₃)₂ eine entsprechende Menge Lithiumtrifluoracetat verwendet wurde.
Beispiel 12 (Herstellung von Mo(N-2,6-Me₂-C₆H₃)(IMesH₂)(CHCMe₃)(OTf)(OC₆F₅)) **(15):** Mo(N-2,6-Me₂-C₆H₃)(IMesH₂)(CHCMe₃)(OTf)₂ (0,0300 g, 0,0315 mmol) wurde in einer minimalen Menge (∼2 mL) C₂H₄Cl₂ gelöst, die Lösung auf -30°C abgekühlt und anschließend LiOC₆F₅ (0,0050 g, 0,0315 mmol) zugegeben. Die Reaktionsmischung wurde zwei Stunden bei Raumtemperatur gerührt und nachfolgend über Celit abfiltriert. Nach Entfernung des Lösungsmittels im Vakuum wurde ein gelber Feststoff erhalten. Der Rückstand wurde in minimaler Menge Dichlormethan gelöst und für mehrere Tage bei -30°C gelagert, um gelbe Kristalle zu erhalten.
Beispiel 13 (Herstellung von Mo(N-2-*^{t}*Bu-C₆H₄)(IMesH₂)(CHCMe₂Ph)(OTf)₂ **(12):** Mo(N-2-*^{t}*Bu-C₆H₄)(CHCMe₂Ph)(OTf)₂(DME)) (0,0320 g, 0,0430 mmol) wurde zunächst in 2 mL Toluol gelöst. 1,3-Bis(2,4,6-Trimethylphenyl)-2-imidazolidin-2-yliden (0,0130 g, 0,0430 mmol) wurde in 1 mL Toluol gelöst. Hierbei konnte ein Farbumschlag von gelb nach hellorange beobachtet werden. Nach drei Stunden Rühren wurde das Toluol anschließend entfernt und der Rückstand im Vakuum getrocknet. Das Produkt konnte als gelber Feststoff isoliert werden.
Beispiel 14 (Herstellung von Mo(N*t*Bu)(Cl)₂(1,3-*i*Pr₂-4,5-Cl₂-imidazol-2-yliden)(Pyridin)(CHCMe₃)) **(16):** Mo(N*t*Bu)(Cl)₂(Pyridin)₂(CHCMe₃) (0,036 g, 0,078 mmol) wurde in 5 mL Dichlormethan gelöst. 1,3-Me₂-4,5-Cl₂-imidazol-2-yliden-AgI (0,036 g, 0,36 mmol, 1,0 Äquiv.) wurde als Feststoff zugegeben. Die Suspension wurde für 1 Stunde bei Raumtemperatur gerührt. Anschließend wurde die Suspension über Celit filtriert und das Lösemittel entfernt. Der hellgelbe Feststoff wurde in 4 mL Dichlormethan aufgenommen und ein weiteres Mal filtriert. Das Lösemittel wurde entfernt und der Feststoff mit n-Pentan gewaschen. Das Produkt wurde als hellorangener Feststoff erhalten. Ausbeute: 0,039 g (82%). ¹H NMR (400 MHz, CD₂Cl₂): δ = 1,55 (s, br, 9H, *t*Bu), 1,67 (s, br, 9H, *t*Bu), 1,71 (d, br, 12H, *i*Pr-Me), 4,98 (m, br, 2H, *i*Pr-CH), 7,56 (m, br, 2H, pyr), 8,02 (m, br, 1H, pyr), 9,21 (m, br, 1H, pyr), 9,86 (m, br, 1H, pyr), 14,38 (s, br, 1H, Mo=CH).
Beispiel 15 (Synthese von Kat. **17**): Es wurden 31,1 mg (0,076 mmol) 1-(2,6-Diisopropylphenyl)-3-(2-hydroxyphenyl)-4,5-dihydro-imidazolium tetrafluoroborat und 25,4 mg (0,152 mmol) LiHMDS in Benzol suspendiert. Nachdem 1 h bei Raumtemperatur gerührt wurde, wurde der Feststoff abfiltriert und das Filtrat zu einer Lösung von 60 mg (0,076 mmol) Mo(N-2,6-C₆H₃*ⁱ*Pr₂)(CH₂CMe₂Ph)(OSO₂CF₃)₂-(DME) getropft. Die gelbe Lösung wurde dabei etwas dunkler und trübte sich leicht. Es wurde 3 h bei Raumtemperatur gerührt und anschließend über Celite filtriert. Das Lösemittel wurde unter vermindertem Druck entfernt und der erhaltene gelbe Feststoff in wenig Dichlormethan gelöst. Bei -35 °C wurden gelbe Kristalle erhalten. ¹H-NMR (CD₂Cl₂, 400 MHz) δ = 13,64 (s, 1H, C*H*CMe₂Ph, J_{CH} = 119 Hz); 7,50-7,41 (m, 2H, CH); 7,28-7,18 (m, 5H, CH); 7,17-7,01 (m, 7H, CH); 6,95 (dd, *J* = 7,79, 1,32 Hz; 1H, CH); 4,60-4,47 (m, 1H, CH); 4,38-4,26 (m, 1H, CH); 4,07-3,94 (m, 1H, CH); 3,93-3,80 (m, 1H, CH); 3,72-3,56 (m, 2H, CH); 268 (hept, *J* = 6,88 Hz; 1H, CH); 2,51 (hept, *J* = 6,51 Hz; 1H, CH); 1,14 (d, *J* = 6,81 Hz; 6H, CH₃); 1,03 (s, 3H, CH₃); 0,98 (d, *J* = 6,84 Hz; 3H, CH₃); 0,5 (d, *J* = 6,81 Hz; 3H, CH₃); 0,84 (d, *J* = 6,86 Hz; 6H, CH₃); 0,3 (d, *J* = 6,74 Hz; 3H, CH₃); 0,6 (d, *J* = 6,80 Hz; 3H, CH₃); ¹⁹F-NMR (CD₂Cl₂) δ = -77,94 (SO₃CF₃); ¹³C-NMR (CD₂Cl₂, 100 MHz) δ = 316,9 (*C*H-Me₂Ph), 205,9 (*C*N_{Carben}), 152,2 (C_{ar.}), 151,9 (C_{ar.}), 149,3 (C_{ar.}), 147,4 (C_{ar.}), 146,5 (C_{ar.}), 145,7 (C_{ar.}), 137,2 (C_{ar.}), 130,3 (C_{ar.}), 129,7 (C_{ar.}), 128,5 (C_{ar.}), 128,4 (C_{ar.}), 126,9 (C_{ar.}), 126,7 (C_{ar.}), 126,6 (C_{ar.}), 126,4 (C_{ar.}), 125,3 (C_{ar.}), 123,2 (C_{ar.}), 120,9 (C_{ar.}), 120,6 (C_{ar.}), 119,9 (q, CF₃, *J* = 319 Hz), 117,5 (C_{ar.}), 55,6 (CH_{2-imidazolyliden.}), 54,9 (CH_{2-imidazolyliden.}), 49,4 (*C*Me₂Ph), 34,8, 29,7, 28,7, 28,5, 26,6, 26,1, 25,9, 24,3, 23,4, 22,7, 21,5.
Beispiel 16 (Herstellung von Kat **18**): Es wurden 30,03 mg (0,086 mmol) 1-(Mesityl)-3-(2-hydroxyphenyl)-4,5-dihydroimidazolium tetrafluoroborat und 27,30 mg (0,163 mmol) LiHMDS in Benzol suspendiert und 2 h bei Raumtemperatur gerührt. Das entstandene LiBF₄ wurde abfiltriert und das Filtrat langsam zu einer Lösung von 60 mg (0,086 mmol) Mo(N-2,6-C₆H₃Me₂)(CH₂CMe₂Ph)(OSO₂CF₃)₂-(DME) in Benzol getropft. Die Reaktionsmischung wurde 3 h bei Raumtemperatur gerührt und anschließend über Celite filtriert. Das Lösemittel wurde unter vermindertem Druck entfernt und der Rückstand in wenig Dichlormethan gelöst. Es wurden ein paar Tropfen *n*-Pentan zugegeben und das Produkt wurde bei -35 °C als dunkelgelbe Kristalle erhalten. ¹H-NMR (CD₂Cl₂, 400 MHz) δ = (*anti*/*syn* 2:3) 14,46; 12,81 (s, 1H, C*H*CMe₂Ph, *J_{CH}* = 147 Hz (*anti*)*,* 116 Hz (*syn*)); 7,31-7,13 (m, 7H, CH); 7,11-7,02 (m, 2H, CH); 7,01-6,76 (m, 3H, CH); 6,70; 6,63 (s, br, 1H, CH); 6,14; 6,02 (s, br, 1H, CH); 4,44-4,15 (m, 2H, CH₂); 2,30 (s, CH₃); 2,22 (s, 3H, CH₃); 2,07 (s, CH₃); 2,05 (s, 3H, CH₃); 1,98 (s, CH₃); 1,84 (s, CH₃); 1,70 (s, CH₃); 1,69 (s, CH₃); 1,57 (s, CH₃); 1,44 (s, CH₃); 1,38 (s, CH₃); 1,30 (s, CH₃); ¹⁹F-NMR (CD₂Cl₂) δ = -78,13 (SO₃CF₃); -78,21 (SO₃CF₃); ¹³C-NMR (CD₂Cl₂, 100 MHz) δ = 330,2 (*C*H-Me₂Ph), 309,3 (*C*H-Me₂Ph), 210,4 (*C*N_{Carben}), 208,1 (*C*N_{Carben}), 154,8 (C_{ar.}), 154,6 (C_{ar.}), 153,6 (C_{ar.}), 151,8 (C_{ar.}), 147,8 (C_{ar.}), 147,7 (C_{ar.}), 140,3 (C_{ar.}), 138,9 (C_{ar.}), 136,4 (C_{ar.}), 136,1 (C_{ar.}), 136,0 (C_{ar.}), 135,6 (C_{ar.}), 135,5 (C_{ar.}), 135,3 (C_{ar.}), 134,6 (C_{ar.}), 130,1 (C_{ar.}), 130,1 (C_{ar.}), 130,0 (C_{a.}), 129,7 (C_{ar.}), 129,4 (C_{ar.}), 129,3 (C_{ar.}), 128,2 (C_{ar.}), 127,8 (C_{ar.}), 127,7 (C_{ar.}), 127,3 (C_{ar.}), 126,7 (C_{ar.}), 126,7 (C_{ar.}), 126,6 (C_{ar.}), 126,5 (C_{ar.}), 126,1 (C_{ar.}), 126,0 (C_{ar.}), 121,0 (C_{ar.}), 120,7 (C_{ar.}), 120,4 (C_{ar.}), 120,1 (C_{ar.}), 120,0 (q, CF3, *J* = 319 Hz), 120,0 (q, CF₃, J = 320 Hz), 117,8 (C_{ar.}), 117,3 (C_{ar.}), 54,7 (CH_{2-imidazolyliden.}), 54,2 (CH_{2-imidazolyliden.}), 51,7 (CH_{2-imidazolyliden.}), 51,3 (CH_{2-imidazolyliden.}), 49,7 (*C*Me₂Ph), 49,5 (*C*Me₂Ph), 32,4 (CH₃), 29,2 (CH₃), 29,2 (CH₃), 27,3 (CH₃), 21,1 (CH₃), 21,0 (CH₃), 20,5 (CH₃), 19,0 (CH₃), 18,5 (CH₃), 18,0 (CH₃), 17,8 (CH₃), 17,3 (CH₃).
Beispiel 17 (ROMP von 5,6-Bis((pentyloxy)methyl)bicyclo[2.2.1]hept-2-en): Zu einer vorgelegten Lösung des Monomers (0,05 g, 0,167 mmol) in 2 mL Dichlormethan wurde bei Raumtemperatur eine Katalysatorlösung von Mo(N-2,6-Me₂-C₆H₃)(IMesH₂)(CHCMe₂Ph)(OTf)₂ (**6**) (0,0032 g, 0,0033 mmol) in 0,5 mL Dichlormethan auf einmal zugegeben. Die Mischung wurde vier Stunden gerührt und das Polymer anschließend in *n*-Pentan ausgefällt. Die Waschphase wurde eingeengt und erneut ausgefällt. Das farblose Polymer wurde mit *n*-Pentan gewaschen und getrocknet(0,045 g, 90 %)). ¹H NMR (400 MHz, CDCl₃): *δ* = 5,27-5,15 (m, 2H), 3,34 (brs, 10H), 2,70 (brs, 1H), 2,31 (brs, 1H), 1,93 (brs, 2H), 1,54 (brs, 4H), 1,32 (brs, 8H), 0,89 (brs, 6H). ¹³C NMR (101 MHz, CDCl₃): *δ* = 134, 133,7, 71,25-70,25 (m), 50,9-39,91 (m), 29,7, 29,6, 28,7, 22,7, 14,2; FT-IR (ATR, cm⁻¹): 2928 (s), 2854 (s), 1460 (m), 1369 (m), 1104 (s), 967 (w), 734(w); *M*ₙ= 4000 g/mol, PDI = 1,03, *σₜᵣₐₙₛ* = 88 %.
Mit Mo(N-2,6-Me₂-C₆H₃)(IMes)(CHCMe₂Ph)(OTf)₂ **(7)** (0,0032 g, 0,0033 mmol) in Dichlormethan (0,5 mL) und dem Monomer (0,05 g, 0,167 mmol) in Dichlormethan (2 mL) wurde das Polymer mit einer Ausbeute von 84 % isoliert (0,042 g). ¹H NMR (400 MHz, CDCl₃): *δ* = 5,27-5,17 (m, 2H), 3,34 (brs, 10H), 2,70 (brs, 1H), 2,31 (brs, 1 H), 1,93 (brs, 2H), 1,54 (brs, 4H), 1,32 (brs, 8H), 0,89 (brs, 6H). ¹³C NMR (101 MHz, CDCl₃): *δ* = 134, 133,7, 71,12-70,63 (m), 47,60-39,92 (m), 29,7, 28,7, 22,7, 14,2; FT-IR (ATR, cm⁻¹): 2928 (s), 2854 (s), 1460 (m), 1369 (m), 1104 (s), 967 (w), 733 (w); *M*ₙ= 8200 g/mol, PDI = 1,06, *σₜᵣₐₙₛ* = 93 %.
Mit Mo(N-2,6-Me₂-C₆H₃)(IMesH₂)(CHCMe₂Ph)(OTf)(OCH(CF₃)₂) (**9**) (0,0026 g, 0,00271 mmol) in Dichlormethan (0,5 mL) und dem Monomer (0,04 g, 0,1358 mmol) in Dichlormethan (2 mL) wurde das Polymer mit einer Ausbeute von 28 % (0,012 g) hergestellt. ¹H NMR (400 MHz, CDCl₃): *δ* = 5,27-5,17 (m, 2H), 3,34 (brs, 10H), 2,70 (brs, 1H), 2,31 (brs, 1H), 1,93 (brs, 2H), 1,54 (brs, 4H), 1,32 (brs, 8H), 0,89 (brs, 6H). ¹³C NMR (101 MHz, CDCl₃): *δ* = 134, 133,6, 71,11-70,11 (m), 47,60-39,78 (m), 29,6, 29,5, 28,5, 22,6, 22,5, 14,2; FT-IR (ATR, cm⁻¹): 2928 (s), 2854 (s), 1460 (m), 1369 (m), 1104 (s), 966 (w), 737 (w); *M*ₙ= 11400 g/mol, PDI = 1,22, *σₜᵣₐₙₛ* = 64 %.
Beispiel 18
Die Polymerisation des gleichen Monomers mit Mo(N-2,6-Me₂-C₆H₃)(I-*t*Bu)(CH-*t*Bu)(OTf)₂ (**2**) (0,050 g Monomer, 2,6 mg Katalysator) liefert das Polymer in 60% isolierter Ausbeute (*Mₙ* = 8500 g/mol), PDI = 1,1, *σₜᵣₐₙₛ* = 50%).
Beispiel 19 (ROMP von 7-Oxabicyclo[2.2.1]hept-5-en-2,3-diylbis(methylen) diacetat):
Eine gekühlte Lösung (-35°C) von Mo(N-2,6-Me₂-C₆H₃)(I-*t*Bu)(CH-*t*Bu)(OTf)₂ (**2**) (0,0045 g, 0,0050 mmol) in CH₂Cl₂ (0,5 mL) wurde zu einer Lösung des Monomers (0,0600 g, 0,2520 mmol) in CH₂Cl₂ (2 mL) bei -30°C zugefügt. Nach 24 Stunden wurde das Polymer durch Zugabe von Pentan gefällt, mit Pentan gewaschen und getrocknet. Ausbeute: 0,058 g (97%). FT-IR (ATR, cm⁻¹): 2902 (m), 1732 (s), 1431 (w), 1366 (s), 1220 (s), 1104 (w), 1029 (s), 968 (s), 728 (m). ¹H NMR (400 MHz, CDCl₃): *δ* 5,72-5,58 (m, 2 H), 4,49 (brs, 1 H), 4,18 (m, 5 H), 2,40 (brs, 2 H), 2,03 (brs, 6 H); ¹³C NMR (101 MHz, CDCl₃): *δ* 170,8, 133,1, 81,4, 61,9, 45,8, 20,9. *Mₙ* = 13000 g/mol, PDI = 1,7, *σₜᵣₐₙₛ* = 85%.
Beispiel 20
Die Polymerisation von 0,050 g des gleichen Monomers mit 0,0032 g Mo(N-2,6-Me₂-C₆H₃)(I-*t*Bu)(CH-*t*Bu)(OTf)₂ (**2**) liefert das Polymer in 35% Ausbeute (*Mₙ* = 1800 g/mol), PDI = 1,2, *σₜᵣₐₙₛ* = 33%).
Beispiel 21 (ROMP von 2-(N-Cyclohexylmethyl)norborn-5-en):
Zu einer gekühlten Lösung (-30°C) von 2-(N-Cyclohexylmethyl)norborn-5-en (57,7 mg) in CH₂Cl₂ (2 mL) wurde eine Lösung von Mo(N-2,6-Me₂-C₆H₃)(IMesH₂)(CH-tBu)(OTf)₂ (**1**) (40 mg) in CH₂Cl₂ (0,5 ml) gegeben. Die Reaktionsmischung wurde 24 h bei 80°C gerührt; anschließend das Polymer durch Zugabe von Pentan gefällt, abfiltriert und getrocknet (44,5 mg, 90%). FT-IR (ATR, cm⁻¹): 3270 (m), 2935 (s), 2860 (m), 2450 (m), 2075 (s), 1681 (m), 1454 (s), 1225 (m), 1159 (s), 1030 (s), 807 (s), 636 (s); ¹H NMR (400 MHz, D₂O, Hydrochlorid Salz): *δ* = 6,40-5,86 (m), NH₂⁺ Teil des D₂O Signals bei 4,7, 3,17 (b), 3,03-2,96 (m), 2,60-2,0 (b, m), 1,93 (b), 1,74 (b), 1,40 (b), 1,28 (b); *Mₙ*= 13100 g/mol, PDI = 1,10.
Mit Mo(N-2,6-Me₂-C₆H₃)(IMesH₂)(CHCMe₂Ph)(OTf)₂ (**6**) (0,0074 g, 0,0078 mmol) und Monomer (0,0400 g, 0,1951 mmol) in CH₂Cl₂ (3 mL) wurde das Polymer in 70% Ausbeute erhalten (0,028 g). FT-IR (ATR, cm⁻¹): 3421 (m), 2935 (s), 2858 (m), 2424 (m), 1630 (m), 1454 (s), 1222 (s), 1155 (s), 1030 (s), 724 (s), 637 (s); ¹H NMR (400 MHz, CDCl₃): δ = 8,69, 8,02, 7,04, 6,92, 6,19, 5,79, 5,77, 5,36, 5,28, 4,52, 3,83, 3,10, 2,96, 2,80, 2,36, 2,14, 2,00, 1,25, 0,86; ¹³C NMR (101 MHz, CDCl₃): δ = 141,2, 138,8, 134,9, 132,0, 130,3, 129,3, 58,1, 49,8, 48,6, 44,5, 42,3, 35,7, 34,3, 24,8, 22,2, 17,8, 14,12.
Mit Mo(N-2,6-Me₂-C₆H₃)(IMes)(CHCMe₂Ph)(OTf)₂ (**7**) (0,0075 g, 0,0078 mmol) in Dichlormethan (0,5 mL) und dem Monomer (0,04 g, 0,1951 mmol) in Chloroform (2 mL) wurde das Polymer mit ∼65 % Ausbeute erhalten (0,026 g). FT-IR (ATR, cm⁻¹): 3425 (m), 2920 (s), 2858 (m), 2424 (m), 1630 (m), 1454 (s), 1222 (s), 1155 (s), 1030 (s), 724 (s), 637 (s); ¹H NMR (400 MHz, CDCl₃): δ = 8,64, 7,06, 6,22, 6,21, 5,69, 5,34, 3,09, 2,85, 2,34, 2,17, 1,84, 1,82, 1,24, 0,88; ¹³C NMR (101 MHz, CDCl₃): δ = 142,0, 138,8, 134,0, 131,7, 129,8, 50,10, 42,68, 34,4, 24,1, 22,8, 14.
Mit Mo(N-2,6-Me₂-C₆H₃)(IMesH₂)(CHCMe₂Ph)(OTf)(OCH(CF₃)₂) (**9**) (0,0082 g, 0,0078 mmol) in Dichlormethan (0,5 mL) und dem Monomer (0,04 g, 0,1951 mmol) in Dichlormethan (2 mL) wurde das Polymer mit einer Ausbeute von 54 % isoliert (0,022 g).¹H NMR (400 MHz, CDCl₃): δ = 6,97, 6,17, 6,07, 5,79, 5,77, 5,35, 5,33, 4,54, 3,45, 3,18, 2,96, 2,82, 2,64, 2,36, 2,31, 1,81, 1,60, 1,24; ¹³C NMR (101 MHz, CDCl₃): δ = 140,7, 138,5, 137,1, 136,6, 135,2, 132,3, 130,3, 58,2, 48,7, 45,0, 42,8, 42,6, 36,2, 31,5, 29,0, 24,8, 21,0, 17,5, 14,4; FT-IR (ATR, cm⁻¹): 3421 (m), 2935 (s), 2858 (m), 2424 (m), 1630 (m), 1454 (s), 1222 (s), 1155 (s), 1030 (s), 724 (s), 637 (s).
Beispiel 22 (ROMP von 2-(N,N-Dimethylaminomethyl)norborn-5-en)):
Zu einer gekühlten Lösung (-30°C) von 2-(N,N-Dimethylaminomethyl)norborn-5-en) (79,7 mg) in CH₂Cl₂ (2 mL) wurde eine Lösung von Mo(N-2,6-Me₂-C₆H₃)(IMesH₂)(CH-tBu)(OTf)₂ (**1**) (10,5 mg) in CH₂Cl₂ (0,5 ml) gegeben. Nach 24-stündigem Rühren wurde das Polymer durch Zusatz von Pentan gefällt und abfiltriert und getrocknet. Ausbeute: 27 mg, (34%). FT-IR (ATR, cm⁻¹): 2955 (s), 1629 (s), 1464 (s), 1259 (s), 1151 (s), 1029 (s), 636 (s); ¹H NMR (400 MHz, DMSO-d₆): *δ* = 6,98, 5,42, 3,14, 2,66, 2,32, 2,20, 2,17, 2,05, 0,85; *Mₙ*= 10500 g/mol, PDI = 1,21.
Beispiel 23 (ROMP von Norborn-5-en-2,3-dimethanol): Eine Lösung von Mo(N-2,6-Me₂-C₆H₃)(IMesH₂)(CH-tBu)(OTf)₂ (**1**) (0,0173 g, 0,0194 mmol) in CHCl₃ (1,5 mL) wurde zu einer Lösung des Monomers (0,0300 g, 0,1940 mmol) in CHCl₃ (2 mL) bei Raumtemperatur zugefügt. Das Reaktionsgemisch wurde dann 5 Stunden bei 55°C gerührt. Anschließend wird das Polymer aus Pentan gefällt, mit Pentan gewaschen und getrocknet. Ausbeute 80% (0,024 g). FT-IR (ATR, cm⁻¹): 3373 (s), 2930 (s), 2884 (s), 1477 (m), 1261 (s), 1109 (s), 1023 (s), 921 (w), 632 (s); ¹H NMR (400 MHz, DMSO-d₆): *δ* 5,48-5,40 (m, 2H), 3,86 (brs, 2H), 3,44 (brs, 4H), 2,45 (brs, 1H), 2,10 (brs, 1H), 1,83 (brs, 1H), 1,57 (brs, 1H), 1,33 (brs, 1H). ¹³C NMR (101 MHz, CDCl₃): *δ* = 134,8, 129,5, 69,4, 59,3, 47,8, 47,0, 43,8, 43,4, 37,5 (b), 32,3; *Mₙ*= 2800 g/mol, PDI = 1,12.
Beispiel 24 (ROMP von Bicyclo[2.2.1]hept-5-en-2-carbaldehyd):
Eine Lösung von Mo(N-2,6-Me₂-C₆H₃)(IMesH₂)(CH-tBu)(OTf)₂ (**1**) (0,006 g, 0,0068 mmol) in CH₂Cl₂ (1,0 mL) wurde zu einer Lösung des Monomers (0,0400 g, 0,3438 mmol) in CH₂Cl₂ (1,0 mL) bei Raumtemperatur zugefügt. Das Reaktionsgemisch wurde dann 20 Stunden bei Raumtemperatur gerührt. Anschließend wurde die Reaktion mit MeOH:HCl (90:10 Vol./Vol.) gequencht. Das so gefällte Polymer wurde mit Pentan gewaschen und getrocknet. Ausbeute: 55% (0,022 g). FT-IR (ATR, cm⁻¹): 2942 (s), 2830 (m), 1720 (s), 1630 (s), 1470 (s), 1255 (s), 1158 (s), 1026 (s), 719 (s), 636 (s); ¹H NMR (400 MHz, THF-d8): *δ* = 9,53 (C*H*O), 6,13 (b), 5,95 (b), 4,62 (b), 2,75 (b); ¹³C NMR (101 MHz, THF-d₈): *δ* = 204,6 (*C*HO), 141,2, 136,9, 131,9, 130,7, 128,7, 43,2, 30,4, 21,2, 19,0, 17,9; *Mₙ*= 5000 g/mol, PDI = 2,1 (*M_{n, theor.}* = 6100 g/mol).
Beispiel 25 (Allgemeine Vorschrift für die Cyclopolymerisation von Diinen):
Der Katalysator wurde im angegebenen Lösemittel gelöst und diese Lösung rasch zu einer des Monomers im selben Lösemittel zugegeben. Nach 2 Stunden wurden die Polyreaktionen durch Zugabe von nassem Methanol terminiert. Nach weiteren 10 min wurde das Polymer durch Zugabe von Methanol oder Pentan gefällt und getrocknet.
Beispiel 26 (Herstellung von Poly-(4,4,5,5-tetrakis(ethoxycarbonyl)-1,7-octadiin)):
Das Polymer wurde nach Beispiel 25 unter Verwendung von Mo(N-2,6-Me₂-C₆H₃)(IMesH₂)(CH-*t*Bu)(OTf)₂ (**1**) (3,6 mg, 0,004 mmol) und dem Monomer (80 mg, 0,203 mmol) in 81% isolierter Ausbeute erhalten (64 mg). IR (cm⁻¹): 2901 (m), 1730 (s), 1461 (m), 1444 (m), 1387 (m), 1363 (m), 1265 (s), 1198 (m), 1122 (w), 1095 (m), 1052 (m), 1027 (s), 941 (m), 856 (m), 781 (w), 703 (w); ¹H-NMR (400 MHz, CDCl₃): δ = 6,71 (s, 2H, C*H*), 4,41-4,25 (bs, 8H, C*H*₂), 3,25-3,18 (bs, 4H, C*H*₂), 1,38-1,23 (bs, 12H, C*H*₃); ¹³C-NMR (101 MHz, CDCl₃): δ = 169,7, 130,8, 124,7, 61,7, 56,9, 32,5, 13,7; UV/Vis (CHCl₃): *λₘₐₓ* = 484 nm. *Mₙ* = 13200 g/mol, PDI = 1,9 (*Mₙ, _{theor.}* = 19700 g/mol).
Das Polymer konnte durch die Verwendung von Mo(N-2,6-Me₂-C₆H₃)(IMesH₂)(CHCMe₂Ph)(OTf)₂ (**6**) (0,0036 g, 0,004 mmol) und dem Monomer (0,0800 g, 0,203 mmol) mit 81 % Ausbeute isoliert werden (64 mg). Die Polymerisation wurde bei -30 °C gestartet und für eine Stunde bei 80 °C weitergerührt. ¹H-NMR (CDCl₃): δ = 7,01 (br, m, 2H), 4,21 (br, m, 8H), 3,18 (br, m, 4H), 1,28 (br, m, 12H); ¹³C-NMR (CDCl₃): δ = 169,9, 131,0, 125,0, 61,9, 57,1, 32,7, 14,0; FT-IR (ATR, cm⁻¹): 2981 (m), 1729 (s), 1444 (w), 1368 (s), 1262 (s), 1199 (w), 1092 (s), 1027 (w), 945 (s), 862 (w), 700 (w), 636 (w), 579(w). UV/Vis (CHCl₃): *λ*ₘₐₓ = 483 nm, *M*ₙ = 15000 g/mol, PDI = 2,2, *α*-Insertion: ≥ 96%.
Mit dem Monomer (0,0400 g, 0,1014 mmol) und Mo(N-2,6-Me₂-C₆H₃)(IMes)(CHCMe₂Ph)(OTf)₂ (7) (0,0019 g, 0,0020 mmol) wurde das Polymer mit einer Ausbeute von 75 % hergestellt (0,03 g). ¹H-NMR (CDCl₃): δ = 7,02 (br, m, 2H), 4,21 (br, m, 8H), 3,18 (br, m, 4H), 1,27 (br, m, 12H); ¹³C-NMR (CDCl₃): δ = 169,9, 131,0, 125,0, 61,9, 57,1, 32,7, 14,0; FT-IR (ATR, cm⁻¹): 2981 (m), 1729 (s), 1444 (w), 1368 (s), 1262 (s), 1199 (w), 1092 (s), 1027 (w), 945 (s), 862(w), 700 (w), 636 (w), 579(w). UV/Vis (CHCl₃): *λ*ₘₐₓ = 481 nm, *M*ₙ = 14000 g/mol, PDI = 1,8, *α*-Insertion: ≥ 96%.
Mit Mo(N-2,6-Me₂-C₆H₃)(IMesH₂)(CHCMe₂Ph)(OTf)(OCH(CF₃)₂) (**9**) (0,0022 g, 0,0020 mmol) und dem Monomer (0,0400 g, 0,1014 mmol) wurde das Polymer ebenfalls mit einer Ausbeute von 75 % isoliert (0,03 g). ¹H-NMR (CDCl₃): δ = 7,02 (br, m, 2H), 4,22 (br, m, 8H), 3,19 (br, m, 4H), 1,27 (br, m, 12H); ¹³C-NMR (CDCl₃): δ = 169,9, 131,0, 125,0, 61,9, 57,1, 32,7, 14,0; FT-IR (ATR, cm⁻¹): 2981 (m), 1729 (s), 1444 (w), 1368 (s), 1262 (s), 1199 (w), 1092 (s), 1027 (w), 945 (s), 862(w), 700 (w), 636 (w), 579(w). UV/Vis (CHCl₃): *λ*ₘₐₓ = 482 nm, *M*ₙ = 22000 g/mol, PDI = 2,1, *α*-Insertion: ≥ 96%.
Beispiel 27 (Herstellung von Poly-(2-(prop-2-yn-1-yl)pent-4-insäure)):
Das Polymer wurde aus Mo(N-2,6-Me₂-C₆H₃)(IMesH₂)(CHCMe₂Ph)(OTf)₂ (**6**) (0,0055 g, 0,0059 mmol) und dem Monomer (0,004 g, 0,294 mmol)mit einer Ausbeute von 65 % (0,0260 g) hergestellt. Die Polymerisation wurde bei -30 °C gestartet und für eine Stunde bei 80 °C weiter gerührt ¹H-NMR (400 MHz, *d*₆-DMSO): δ = 12,25, 7,07-6,84, 3,23, 2,34; ¹³C-NMR (400 MHz, *d*₆-DMSO): δ · = 177,0, 135,4, 129,5, 71,1, 58,1; FT-IR (ATR, cm⁻¹): 2981 (m), 1729 (s), 1444 (w), 1368 (s), 1262 (s), 1199 (w), 1092 (s), 1027 (w), 945 (s), 862(w), 700 (w), 636 (w), 579(w). UV/Vis (CHCl₃): *λ*ₘₐₓ = 587, 547 nm.
Mit Mo(N-2,6-Me₂-C₆H₃)(IMes)(CHCMe₂Ph)(OTf)₂ (**7**) (0,0055 g, 0,0059 mmol) und dem Monomer (0,0400 g, 0,294 mmol) wurde das Polymer mit 55 % Ausbeute isoliert (0,022 g). ¹H-NMR (400 MHz, *d*₆-DMSO): δ = 12,27, 6,68-6,76, 3,23, 2,34; ¹³C-NMR (400 MHz, *d*₆-DMSO): δ = 177,0, 135,4, 129,5, 71,1, 58,1; FT-IR (ATR, cm⁻¹): 2981 (m), 1729 (s), 1444 (w), 1368 (s), 1262 (s), 1199 (w), 1092 (s), 1027 (w), 945 (s), 862 (w), 700 (w), 636 (w), 579 (w). UV/Vis (CHCl₃): *λ*ₘₐₓ = 587, 547 nm.
Mit dem Monomer (0.0400 g, 0.294 mmol) und Mo(N-2,6-Me₂-C₆H₃)(IMesH₂)(CHCMe₂Ph)(OTf)(OCH(CF₃)₂) (**9**) (0,0062 g, 0,0059 mmol) konnte das Polymer ebenfalls mit einer Ausbeute von 55 % gewonnen werden (0,022 g). ¹H-NMR (400 MHz, *d*₆-DMSO): δ = 12,27, 6,68-6,76, 3,23, 2,34; ¹³C-NMR (400 MHz, *d*₆-DMSO): δ = 177,0, 135,4, 129,5, 71,1, 58,1; FT-IR (ATR, cm⁻¹): 2981 (m), 1729 (s), 1444 (w), 1368 (s), 1262 (s), 1199 (w), 1092 (s), 1027 (w), 945 (s), 862(w), 700 (w), 636 (w), 579 (w). UV/Vis (CHCl₃): *λ*ₘₐₓ = 587, 547 nm.
Beispiel 28 (Herstellung von Poly-(2,2-di(prop-2-yn-1-yl)propane-1,3-diol)): Das Polymer wurde unter Verwendung von Mo(N-2,6-Me₂-C₆H₃)(IMesH₂)(CH-*t*Bu)(OTf)₂ (**1**) in 80% isolierter Ausbeute erhalten (0,030 g). ¹H NMR (400 MHz, d₆-DMSO): *δ* = 7,09-6,66 (m, 2H), 4,60 (brs, 2H), 3,17 (s, 2H), 2.08 (s, 2H). UV-Vis: *λₘₐₓ* = 593, 554 nm (DMSO).
Mit Mo(N-2,6-Me₂-C₆H₃)(IMesH₂)(CHCMe₂Ph)(OTf)₂ (**6**) (0,010 g, 0,0105 mmol) und dem Monomer (0,04 g, 0,2628 mmol) wurde das Polymer mit einer Ausbeute von 70 % (0,027 g) hergestellt. Die Polymerisation wurde bei -30 °C gestartet und für eine Stunde bei Raumtemperatur weitergerührt. ¹H NMR (400 MHz, *d*₆-DMSO): *δ* = 7,27-6,66, 4,42, 2,34, 2,29, 1,9; ¹³C NMR (101 MHz, *d*₆-DMSO): *δ* = 139,7, 135,4, 130,9, 129,4, 50,9, 20,5, 17,6, 17,2; IR (ATR-mode, cm⁻¹): 3400 (w), 2977 (w), 1444 (w), 1367 (w), 1247 (m), 1159 (m), 1065 (m), 946 (w), 856 (w), 629 (w). UV-Vis: *λₘₐₓ* = 593, 554 nm (DMSO); *M*ₙ = 5000 g/mol, PDI = 2,1.
Bei der Verwendung von Mo(N-2,6-Me₂-C₆H₃)(IMes)(CHCMe₂Ph)(OTf)₂ (7) (0,010 g, 0,0105 mmol) und dem Monomer (0,0400 g, 0,2628 mmol) konnte das Polymer mit einer Ausbeute von 65 % hergestellt werden (0,026 g). ¹H NMR (400 MHz, *d*₆-DMSO): *δ* = 7,19-6,72, 2,86, 2,34, 2,26, 2,1, 1,82; ¹³C NMR (101 MHz, d₆-DMSO): *δ* = 139,7, 135,4, 130,9, 129,4, 50,9, 20,5, 17,6, 17,2; IR (ATR-Modus, cm⁻¹): 3420 (w), 2963 (w), 1465 (w), 1353 (w), 1233 (m), 1122 (m), 1072 (m), 920 (w), 863 (w), 640 (w). UV-Vis: *λ*ₘₐₓ = 595, 554 nm (DMSO); *Mₙ* = 3900 g/mol, PDI = 1,8.
Bei der Verwendung von Mo(N-2,6-Me₂-C₆H₃)(IMesH₂)(CHCMe₂Ph)(OTf)(OCH (CF₃)₂) (**9**) (0,0011 g, 0,0052 mmol) und dem Monomer (0,0400 g, 0,212 mmol) wurde das Polymer mit 54 % Ausbeute isoliert (0,022 g). IR (ATR-Modus, cm⁻¹): 3400 (w), 2977 (w), 1444 (w), 1367 (w), 1247 (m), 1159 (m), 1065 (m), 946 (w), 856 (w), 629 (w). UV-Vis: *λ*ₘₐₓ= 593, 554 nm (DMSO); *Mₙ* = 3000 g/mol, PDI = 1,3.
Beispiel 29 (Poly(dipropargylmalodinitrile)):
Eine Lösung von Mo(N-2,6-Me₂-C₆H₃)(IMesH₂)(CH-tBu)(OTf)₂ (**1**) (0,016 g, 0,0211 mmol) in CH₂Cl₂ (0,5 mL) wurde zu einer Lösung des Monomers (0,0300 g, 0,211 mmol) in CH₂Cl₂ (2 mL) bei -30°C zugefügt. Die Mischung wurde 90 min lang bei Raumtemperatur gerührt dann mit MeOH-HCI (90:10, Vol./Vol.) gequencht. Das gefällte Polymer wurde mit Pentan gewaschen und getrocknet. Ausbeute: 60% (0,018 g). IR (cm⁻¹): 2960 (m), 2252 (w), 1588 (w), 1484 (m), 1267 (s), 1232 (s), 1027 (s), 810 (w), 636 (s); ¹H-NMR (DMSO-d₆): *δ* = 7,5-6,5 (b), 53,8 (b); ¹³C-NMR (DMSO-d₆): *δ* = 160,2, 139,6, 135,4, 130,8, 129,4, 50,9, 30,2, 20,6, 17,6; UV/Vis (DMSO): *λₘₐₓ* = 530 nm. *Mₙ* = 1100 g/mol, PDI = 1,15 (*Mₙ, theor.* = 1420 g/mol).
Beispiel 30 (Poly-(1,7-octadiin-4,5-dicarbonsäure):
Eine Lösung von Mo(N-2,6-Me₂-C₆H₃)(IMesH₂)(CH-tBu)(OTf)₂ (**1**) (0,0137 g, 0,0154 mmol) in CH₂Cl₂ (1,0 mL) wurde zu einer Lösung des Monomers (0,0300 g, 0,1956 mmol) in THF (2 mL) bei -30°C zugefügt. Das Reaktionsgemisch wurde 1 Stunde bei Raumtemperatur gerührt, dann wurde das Polymer mit Pentan gefällt, mit Pentan gewaschen und getrocknet. Ausbeute: 90% (0,034 g). IR (cm⁻¹): 3288 (m), 2918 (m), 1702 (s), 1431 (m), 1213 (s), 1168 (s), 1026 (s), 946 (m), 634 (s); ¹³C-NMR (CDCl₃): *δ* = 174,8, 128-140 (b), 40,4, 30,1; ¹³C-NMR (Li-OD/D₂O): *δ* = 184,5, 132-128, 44,4, 30,5; UV/Vis (THF): *λₘₐₓ* = 432 nm. *Mₙ* = 2600 g/mol, PDI = 1,3 (*Mₙ, _{theor.}* = 2500 g/mol).
Beispiel 31 (Poly-(4,4-bis(ethoxycarbonyl)-1,6-heptadiin; Poly(DEDPM)):
Das Polymer wurde unter Verwendung von Mo(N-2,6-Me₂-C₆H₃)(IMesH₂)(CH-tBu)(OTf)₂ (1) (4,5 mg, 0,0051 mmol) und des Monomers (60 mg, 0,2540 mmol) in 89% Ausbeute dargestellt (53 mg). Die Polymerisation wurde bei -30°C gestartet und dann für eine weitere Stunde bei Raumtemperatur geführt. ¹H-NMR (CDCl₃): *δ* = 6,95-6,83 (s, 1H, CH), 6,45 (s, 1H, CH), 4,10-3,37 (bm, 6H, CH₂), 2,82 (s, 1H, CH), 2,05-1,80 (m, 2H, CH₂), 1,17 (s, 3H); ¹³C-NMR (CDCl₃): *δ* = 170,9, 170,8, 169,0, 137,0, 123,2, 61,9, 58,2, 58,0, 57,3, 57,1, 54,3, 54,1, 41,5, 29,7, 14,1; IR (ATR, cm⁻¹): 3367 (m), 2969 (s), 2929 (s), 2864 (s), 1673 (s), 1519 (m), 1453 (m), 1366 (s), 1337 (w), 1258 (w), 1190 (w), 1125 (s), 1077 (s), 947 (m), 770 (s), 690 (w); UV/Vis (CHCl₃): *λₘₐₓ* = 548, 584 nm, α-Insertion: 81%, *kₚ*/*kᵢ* = 7.
Mit Mo(N-2,6-Me₂-C₆H₃)(IMesH₂)(CHCMe₂Ph)(OTf)₂ (**6**) (0,004 g, 0,0042 mmol) und Monomer (0,05 g, 0,213 mmol) wurde das Polymer in 84% Ausbeute erhalten. ¹H-NMR (400 MHz, CDCl₃): δ = 6,68 (br m, 2H), 4,27 (br m, 4H), 3,43 (br m, 4H), 1,30 (br m, 6H) ppm; ¹³C-NMR (101 MHz, CDCl₃): δ = 172,1, 137,1, 128,4, 126,3, 123,3, 62,1, 57,4, 41,6, 14,2 ppm; IR (ATR-Modus, cm⁻¹): 2977 (w), 1720 (s), 1444 (w), 1367 (w), 1247 (m), 1159 (m), 1065 (m), 946 (w), 856 (w), 629 (w). UV/Vis (CHCl₃): *λ*ₘₐₓ = 586, 546 nm. *M*ₙ = 8500 g/mol, PDI = 2,1, *α-*Insertion: ≥ 95%.
Bei der Verwendung von Mo(N-2,6-Me₂-C₆H₃)(IMes)(CHCMe₂Ph)(OTf)₂ (**7**) (0,0040 g, 0,0042 mmol) und dem Monomer (0,0500 g, 0,212 mmol) konnte Poly(DEDPM) mit einer Ausbeute von 86 % (0,043 g) isoliert werden. ¹H-NMR (CDCl₃): δ =6,68 (br m, 2H), 4,27 (br m, 4H), 3,43 (br m, 4H), 1,31 (br m, 6H); ¹³C-NMR (CDCl₃): δ = 172,1, 138,7, 128,0, 125,8, 122,9, 62,1, 57,4, 41,5, 14,2; FT-IR (ATR, cm-1): 2979 (m), 1722 (s), 1446 (w), 1367 (s), 1248 (s), 1158 (w), 1067 (s), 947 (s), 631 (m). UV/Vis (CHCl₃): *λ*ₘₐₓ=587, 546 nm, *M*ₙ = 84000 g/mol, PDI = 2,8, *α*-Insertion: ≥ 99%.
Bei der Verwendung von Mo(N-2,6-Me₂-C₆H₃)(IMesH₂)(CHCMe₂Ph)(OTf) (OCH(CF₃)₂) (**9**) (0,0040 g, 0,0042 mmol) mit dem Monomer (0,0500 g, 0,212 mmol) konnte Poly(DEDPM) mit einer Ausbeute von 54% isoliert werden (0,043 g). ¹H-NMR (CDCl₃): δ = 6,68 (br m, 2H), 4,27 (br m, 4H), 3,43 (br m, 4H), 1,31 (br m, 6H); ¹³C-NMR (CDCl₃): δ = 172, 1, 137,1, 128,2, 126,4, 123,3, 62,1, 57,4, 41,6, 14,2; FT-IR (ATR, cm⁻¹): 2977 (m), 1721 (s), 1444 (w), 1367 (s), 1248 (s), 1158 (w), 1067 (s), 947 (s), 631 (m). UV/Vis (CHCl₃): *λ*ₘₐₓ=581, 546 nm, *M*ₙ=67400 g/mol, PDI = 2,7, *α*-Insertion: ≥ 96%.
Beispiel 32 (Poly-(4,4-bis[(3,5-diethoxybenzoyloxy)methyl]-1,6-heptadiin)):
Das Polymer wurde unter Verwendung von Mo(N-2,6-Me₂-C₆H₃)(IMesH₂)(CH-tBu)(OTf)₂ (**1**) (1,9 mg, 0,0022 mmol) und des Monomers (60 mg, 0,1120 mmol) in 94% Ausbeute dargestellt (57 mg). Die Polymerisation wurde bei -30°C gestartet und dann für eine weitere Stunde bei Raumtemperatur geführt. ¹H-NMR (CDCl₃): *δ* = 7,04-6,92 (m, 4H), 6,71-6,60 (m, 2H), 6,45-6,31 (m, 2H), 4,44-4,31 (m, 4H), 3,90-3,81 (m, 8H), 2,89-2,82 (m, 4H), 1,40-1,25 (m, 12H); ¹³C-NMR (CDCl₃): *δ* = 168,3, 159,3, 138,2, 131,2, 107,7, 107,6, 106,3, 63,6, 40,7, 27,1, 14,7; IR (ATR, cm⁻¹): 3367 (m), 2969 (s), 2929 (s), 2864 (s), 1673 (s), 1519 (m), 1453 (m), 1366 (s), 1337 (w), 1258 (w), 1190 (w), 1125 (s), 1077 (s), 947 (m), 770 (s), 690 (w); UV/Vis (CHCl₃): *λₘₐₓ* = 550, 590 nm, α-Insertion: > 91%, *kₚ*/ *kᵢ* = 33.
Das Polymer konnte durch die Verwendung von Mo(N-2,6-Me₂-C₆H₃)(IMesH₂)(CHCMe₂Ph)(OTf)₂ (**6**) (0,0014 g, 0,0015 mmol) und dem Monomer (0,04 g, 0,0745 mmol) in quantitativer Ausbeute isoliert werden (0,0392 g). Die Polymerisation wurde bei -30 °C gestartet und für eine Stunde bei 80 °C weitergerührt. ¹H-NMR (CDCl₃): δ = 6,90 (br, m, 4H), 6,36 (br, m, 4H), 4,30 (brs, 4H), 3,85 (brs, 8H), 2,81 (brs, 4H), 1,29 (brs, 12H); ¹³C-NMR (CDCl₃): δ = 168,5, 159,9, 138,3, 131,1, 123,4, 107,7, 106,4, 69,6, 63,7, 43,4, 40,8, 14,8; FT-IR (ATR, cm⁻¹): 2978 (w), 1788 (w), 1716 (s), 1592 (s), 1446 (m), 1385 (w), 1296 (m), 1216 (s), 1166 (s), 1101 (m), 1051 (m), 990 (w), 817 (w), 757 (m), 675 (w), 619 (m); UV/Vis (CHCl₃): *λ*ₘₐₓ= 591, 550 nm, *α*-Insertion: ≥ 93%.
Mit dem Monomer (0,0400 g, 0,754 mmol) und Mo(N-2,6-Me₂-C₆H₃)(IMes)(CHCMe₂Ph)(OTf)₂ (**7**) (0,0014 g, 0,0015 mmol) wurde das Polymer mit einer Ausbeute von 60 % hergestellt (0,0255 g). ¹H-NMR (CDCl₃): δ = 6,90 (br, m, 4H), 6,36 (br, m, 4H), 4,30 (br, s, 4H), 3,84 (br, S, 8H), 2,81 (br, s, 4H), 1,28 (br, s, 12H); ¹³C-NMR (CDCl₃): δ = 166,5, 159,9, 138,5, 131,2, 123,4, 107,7, 106,4, 69,6, 63,8, 43,4, 40,8, 14,5; FT-IR (ATR, cm⁻¹): 2978 (w), 1787 (w), 1716 (s), 1591 (s), 1446 (m), 1385 (w), 1297 (m), 1216 (s), 1166 (s), 1101 (m), 1051 (m), 990 (w), 817 (w), 757 (m), 674 (w), 618 (m), UV/Vis (CHCl₃): *λ*ₘₐₓ= 590, 550 nm, *α*-Insertion: ≥ 93%.
Das Polymer wurde mit Mo(N-2,6-Me₂-C₆H₃)(IMesH₂)(CHCMe₂Ph)(OTf)(OCH(CF₃)₂) (**9**) (0,0015 g, 0,0015 mmol) und dem Monomer (0,0400 g, 0,0745 mmol) mit einer Ausbeute von nur 50 % (0,020g) hergestellt. ¹H-NMR (CDCl₃): δ = 6,90 (br, m, 4H), 6,36 (br, m, 4H), 4,30 (br, s, 4H), 3,84 (br, s, 8H), 2,81 (br, s, 4H), 1,28 (br, s, 12H); ¹³C-NMR (CDCl₃): δ = 166,4, 159,9, 138,3, 131,2, 123,4, 107,.6, 106,4, 69,5, 63,6, 43,4, 40,8, 14,8; FT-IR (ATR, cm⁻¹): 2978 (w), 1787 (w), 1716 (s), 1591 (s), 1446 (m), 1385 (w), 1297 (m), 1216 (s), 1166 (s), 1101 (m), 1051 (m), 990 (w), 817 (w), 757 (m), 674 (w), 618 (m), UV/Vis (CHCl₃): *λ*ₘₐₓ= 591, 550 nm, *α*-Insertion: ≥ 95 %.
Beispiel 33 (Allgemeine Vorgehensweise für die Reaktionen mit den Katalysatoren **1 - 18**):
Homometathese und Ringschlussmetathese (RCM): Die Reaktionen werden in 1,2-Dichlorethan (5 mL) und den entsprechenden Substraten (siehe Tabelle 1) durchgeführt. T = 80 °C; Katalysator : Substrat (soweit nicht anders angegeben) = 1:1000. Der Umsatz wurde nach vier Stunden Reaktionszeit mit GC-MS bestimmt. Interner Standard: Dodekan. Die Ergebnisse dieser Untersuchungen sind in der folgenden Tabelle 1 wiedergegeben.
Ring öffnenden Metathese Polymerisation (ROMP): Alle Reaktionen wurden bei 80 °C in 1,2-Dichlorethan über einen Zeitraum von 4 Stunden durchgeführt. Monomer/Katalysator = 50 : 1) Die Ergebnisse dieser Untersuchungen sind in der folgenden Tabelle 2 wiedergegeben.
Cyclopolymerisation von α,ω-Diinen: Alle Reaktionen wurden bei -30 °C bis Raumtemperatur in Dichlormethan bei einem Monomer/Katalysator-Verhältnis von 50:1 und, soweit nicht anders angegeben, über einen Zeitraum von 1 Stunde durchgeführt. Die Ergebnisse dieser Untersuchungen sind in den folgenden Tabelle 3 bis 7 wiedergegeben. Die im Rahmen der ROMP und Cyclopolymerisationen eingesetzten Monomere sind im Folgenden dargestellt:

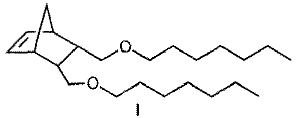

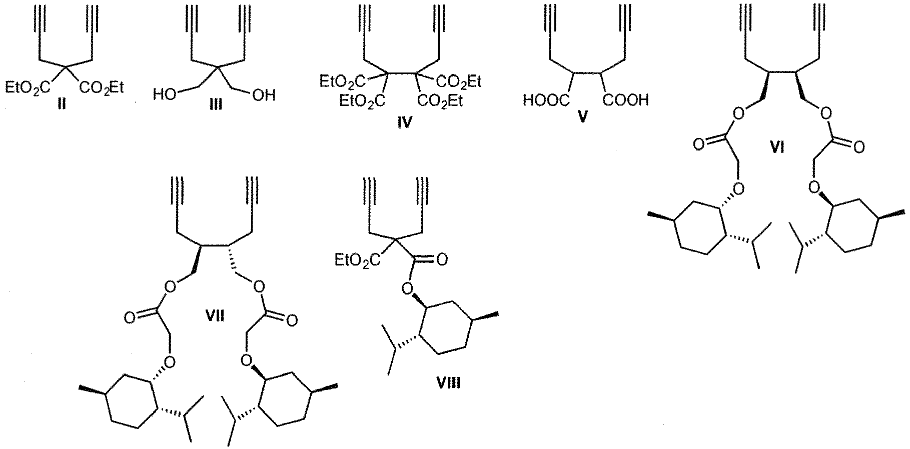

**Tabelle 1. Wechselzahlen der Katalysatoren 9 - 14 in verschiedenen Olefinmetathesereaktionen.**

| | **9** | **10** | **11** | **12** | **13** | **14** |
|---|---|---|---|---|---|---|
| **Substrat** | | | | | | |
| *Homometathese (HM), (Werte in Klammern geben die E-Fraktion in % an)* | | | | | | |
| Allyltrimethylsilan | 520 (60) | 435 (55) | - | - | 460 (60) | 350 (60) |
| 1-Hexen | 340 (100) | 490 (100) | 790 (100) | 85000^{[c]} (100) | 660 (100) | 540 (100) |
| | | | | 140000^{[d]} (100) | | |
| Styrol | 60 (100) | 80 (100) | 200 (100) | 45000^{[d]} (100) | 30 | - |
| 1-Okten | 680 (85) | 560 (85) | | 210000^{[d]} | 400(100) | 480 (100) |
| | | | | 150000 (86) | | |

| *Ringschlussmetathese (RCM)* | | | | | | |
|---|---|---|---|---|---|---|
| Diethyldiallyl malonat | 175 | 90 | 3200^{[b]} | | 150 | 350 |
| Diallyldiphenylsilan | 620 | 490 | 390 | | 660 | 520 |
| 1,7-Oktadien | 140 | 920 | 4100^{[b]} | 80000^{[c]} | 830 | 650 |
| | | | | 100000^{[d]} | | |
| N, N-Diallyl-*t-*butylcarbamid | 390 | 50 | | | 270 | 0 |
| N,N-Diallyl-*p*-tosylamid | 180 | 160 | 420 | | 250 | 350 |
| N,N-Diallytrifluoracetamid | 62 | - | - | | 15 | 0 |
| Diallylmalodinitril | 190 | 70 | 360 | | 100 | 150 |
| Diallylether | 220 | 245 | 690 | | 0 | 0 |

| | | | | | | |
|---|---|---|---|---|---|---|
| ^{[a]} ClCH₂CH₂Cl, 80 °C, 4 h, Kat : Substrat = 1:1000; ^{[b]} ClCH₂CH₂Cl, 80 °C, 4 h, Kat : Substrat = 1:5000;^{[c]} ClCH₂CH₂Cl, RT, über Nacht, Kat : Substrat = 1:100000; ^{[d]} ClCH₂CH₂Cl, RT, 1 h, Kat : Substrat = 1:500000. | | | | | | |

**Tabelle 2. Zusammenfassung der Polymerisationsergebnisse mit den Katalysatoren 9 - 11. Monomer : Katalysator = 50 : 1. Alle Reaktionen wurden in CH₂Cl₂ bei Raumtemperatur durchgeführt.**

| **Monomer** | **Katalysator** | **Ausbeute (%)** | **Selektivität (*cis*/*trans*)** | ***M*ₙ (g/mol)** | **PDI** |
|---|---|---|---|---|---|
| **I** | **9** | 84 | ≥ 95% | 4000 | 1.03 |
| **I** | **10** | 86 | ≥ 99% | 8200 | 1.06 |
| **I** | **11** | 28 | ≥ 64% | 11400 | 1.22 |

**Tabelle 3. Reaktivität der Katalysatoren 8, 9 und 11 in der Cyclopolymerisation von α,ω-Diinen. Monomer : Katalysator = 50 : 1.**

| **Monomer** | **Kat.** | **LM/T (°C)/t** | **Ausbeute (%)** | ***α-*Selektivität** | ***M*ₙ (g/mol)** | **PDI** |
|---|---|---|---|---|---|---|
| **II** | **9** | CH₂Cl₂, - 30 °C - RT, 1 h | 84 | ≥ 95% | 8500 | 2.1 |
| **II** | **10** | CH₂Cl₂, - 30 °C - RT, 1 h | 86 | ≥ 99% | 84000 | 2.3 |
| **II** | **11** | CH₂Cl₂, - 30 °C - RT, 1 h | 54 | ≥ 96% | 67000 | 2.7 |
| **III** | **9** | CH₂Cl₂, - 30 °C - RT, 1 h | 70 | - | 5000 | 2.1 |
| **III** | **10** | CH₂Cl₂, - 30 °C - RT, 1 h | 56 | - | 3900 | 1.8 |
| **III** | **11** | CH₂Cl₂, - 30 °C - RT, 1 h | 54 | - | 3000 | 1.3 |
| **IV** | **9** | CHCl₃,-30 °C - 80°C 1 h | 81 | 96 | 15000 | 2.2 |
| **IV** | **10** | CHCl₃,-30 °C - 80°C 1 h | 75 | 96 | 14000 | 1.8 |
| **IV** | **11** | CHCl₃,-30 °C - 80°C 1 h | 75 | 96 | 2200 | 2.1 |
| **V** | **9** | CHCl₃,-30 °C - 80°C 1 h | 65 | - | 3300 | 1.9 |
| **V** | **10** | CHCl₃,-30 °C - 80 °C 1 h | 55 | - | 2900 | 1.4 |
| **V** | **11** | CHCl₃,-30 °C - 80 °C 1 h | 55 | - | 6000 | 1.5 |

**Tabelle 4: Cyclopolymerisation von VI mit Initiator 6.**

| **Initiator (I)** | **Verhältnis M : I** | ***M_{n,exp}*^{*a*)} [g/mol]** | **Ausbeute [%]^{b)}** | **PDI** | **α-Selektivität [%]** | ***trans* [%]** | **st [%]** |
|---|---|---|---|---|---|---|---|
| **6** | 50 : 1 | 19.800 | 47 | 1.3 | 96 | 100 | 72 |

CH₂Cl₂, -30°C bis 20°C, 3 h. λₘₐₓ = 469 nm, **poly-VI:** *M_{n,theo}* = 27,900 g/mol. a) GPC in CHCl₃, UV-Vis Detektor, Kalibrierung gegen Poly(styrol)-Standards; b) isolierte, gravimetrisch bestimmte Ausbeuten. st = syndiotaktisch.

**Tabelle 5: Cyclopolymerisation von VII mit Initiator 6.**

| **Initiator (I)** | **Verhältnis M : I** | ***M_{n,exp}*^{a)} [g/mol]** | **Ausbeute [%]^{b)}** | **PDI** | **α-Selektivität [%]** | ***trans* [%]** | **it [%]** |
|---|---|---|---|---|---|---|---|
| **6** | 50 : 1 | 24.800 | 24 | 1.5 | 95 | - | 34 |

CH₂Cl₂, -30°C bis 20°C, 2 h. λₘₐₓ = 463 nm, **poly-VII:** *M_{n,theo}* = 27,900 g/mol. a) GPC in CHCl₃, UV-Vis Detektor, Kalibrierung gegen Poly(styrol)-Standards; b) isolierte, gravimetrisch bestimmte Ausbeuten. it = isotaktisch.

**Tabelle 6: Cyclopolymerisation von Monomer III mit Initiator 6.**

| **Initiator (I)** | **Verhältnis M : I** | ***M_{n,exp}*^{a)} [g/mol]** | **Ausbeute [%]^{b)}** | **PDI** |
|---|---|---|---|---|
| **6** | 50 : 1 | 9.000 | 90 | 1.1 |

CH₂Cl₂, -30°C bis 20°C, 2 h. **Poly-III:** *M_{n,theo}* = 8,300 g/mol. a) GPC in DMSO, UV-Vis Detektor, Kalibrierung gegen Poly(styrol)-Standards; b) isolierte, gravimetrisch bestimmte Ausbeuten.

**Tabelle 7: Cyclopolymerisation von Monomer VIII mit den Initiatoren 1 und 4.**

| **Initiator (I)** | **Verhältnis M : I** | ***M_{n,exp}*^{a)} [g/mol]** | ***λₘₐₓ* [nm]** | **Ausbeute [%]^{b)}** | **PD I** | **α-Selektivität [%]** | ***trans* [%]** | **st [%]** |
|---|---|---|---|---|---|---|---|---|
| **1** | 50 : 1 | 32.300 | 550; 591 | 83 | 2.1 | >90 | 100 | 100 |
| **4** | 50 : 1 | 27.500 | 547; 585 | 77 | 1.8 | >71 | 100 | 74 |

CH₂Cl₂, -30°C bis 20°C, 1 h. **Poly-VIII:** *M_{n,theo}* = 17,300 g/mol. a) GPC in CHCl₃, UV-Vis Detektor, Kalibrierung gegen Poly(styrol)-Standards; b) isolierte, gravimetrisch bestimmte Ausbeuten.
Beispiel 34 (Immobilisierung von 4-(Hydroxymethyl)-1,3-dimesityl-4,5-dihydro-1H-imidazol-3-iumchlorid (**I1**)):
Kieselgel G60 (350 mg) wurde in 10 mL Chloroform suspendiert. Dazu wurden einige Tropfen konzentrierte Schwefelsäure gegeben. 4-(Hydroxymethyl)-1,3-dimesityl-4,5-dihydro-1H-imidazol-3-iumchlorid (500 mg, 1,34 mmol) wurde in 10 mL Chloroform gelöst und zu der Reaktionsmischung zugegeben. Das Reaktionsgemisch wurde über Nacht bei 60 °C gerührt um anschließend auf Raumtemperatur abgekühlt und filtriert zu werden. Der erhaltene Feststoff wurde mehrere Male mit CH₂Cl₂ und demineralisiertem Wasser gewaschen. Um Rückstände an Wasser zu entfernen wurde der Feststoff in trockenem THF suspendiert und eine Stunde gerührt. Der Feststoff wurde abfiltriert und mit Diethylether gewaschen. Alle flüchtigen Bestandteile wurden unter vermindertem Druck entfernt. Der Feststoff wurde in 20 mL CH₂Cl₂ suspendiert, zu dieser Lösung wurde 1 mL Trimethylsilylchlorid (8,14 mmol) zugegeben und über Nacht bei Raumtemperatur gerührt. Alle flüchtigen Bestandteile wurden unter vermindertem Druck entfernt und das Produkt als weißer Feststoff erhalten.
Deprotonierung von **I1** zu **I2: I1** wurde in 20 mL THF suspendiert. Dazu wurde Li-hexamethyldisilazid (LiHMDS, 0,22 g, 1,34 mmol) zugegeben und für zwei Stunden bei Raumtemperatur gerührt. Das Reaktionsgemisch wurde abfiltriert und der erhaltene Feststoff in DMSO suspendiert und für 30 min gerührt. Der Feststoff wurde abfiltriert und mehrmals mit Diethylether gewaschen. Alle flüchtigen Bestandteile wurden unter vermindertem Druck entfernt und das Produkt als leicht gelber Feststoff erhalten. ¹H-MAS-NMR (400,13 MHz): δ = 6,59 (Hₐᵣₒₘ); 3,28 (CH₂, CH); 1,67, 0,87, 0,03 (CH₃).
Beispiel 35 (Immobilisierung von [Mo(N-2,6-Me₂C₆H₃)(CHC(CH₃)2Ph)(OTf)₂(DME)] an **I2 (IMo-1)**):
Mo(N-2,6-Me₂C₆H₃)(CHC(CH₃)₂Ph)(OTf)₂(DME) (100 mg, 0,14 mmol) wurde in 3 mL Benzol gelöst. Zu dieser Lösung wurde I2 zugegeben und für drei Stunden bei Raumtemperatur gerührt. Das Lösungsmittel wurde abdekantiert und der Feststoff mehrmals mit Benzol, Diethylether und CH₂Cl₂ gewaschen, bis die Lösungsmittel keine Färbung mehr aufwiesen. Alle flüchtigen Komponenten wurden unter vermindertem Druck entfernt und das Produkt als orangener Feststoff erhalten. ¹H-MAS-NMR (400,13 MHz): δ = 12,60 (CHCMe₂Ph); 6,88 (Hₐᵣₒₘ); 2,54 (CH₃, CH₂, CH); 0,13 (CH₃).
Beispiel 36 (Immobilisierung von [Mo(N-2,6-Cl₂C₆H₃)(CHC(CH₃)₃)(OTf)₂(DME)] an **I2 (IMo2)**):
Mo(N-2,6-Cl₂C₆H₃)(CHC(CH₃)₃)(OTf)₂(DME) (200 mg, 0,26 mmol) wurde in 3 mL Benzol gelöst. Zu dieser Lösung wurde **I2** zugegeben und für drei Stunden bei Raumtemperatur gerührt. Das Lösungsmittel wurde abdekantiert und der Feststoff mehrmals mit Benzol, Diethylether und CH₂Cl₂ gewaschen, bis die Lösungsmittel keine Färbung mehr aufwiesen. Alle flüchtigen Komponenten wurden unter vermindertem Druck entfernt und das Produkt als orangener Feststoff erhalten. ¹H-MAS-NMR (400,13 MHz): δ = 13,77 (CHCMe₂Ph); 6,96 (Hₐᵣₒₘ); 2,69 (CH₃, CH₂, CH); 0,11 (CH₃).
Beispiel 37 (Allgemeine Arbeitsvorschrift für Metathesereaktionen mit **IMo1** und **IMo2**): Das Metathesesubstrat wurde in über Al₂O₃ filtriertes CH₂Cl₂ (bzw. ClH₂C-CH₂Cl) gelöst und 50 µL Dodekan als interner Standard zur GC-MS Umsatzbestimmung zugegeben. Der immobilisierte Katalysator wurde in über Al₂O₃ filtriertem CH₂Cl₂ (bzw. ClH₂C-CH₂Cl) suspendiert und schnell zu der zuvor hergestellten Lösung zugegeben. Das Reaktionsgemisch wurde für 4 h bei 40 °C (bzw. 80 °C) gerührt. Nach dem Abkühlen auf Raumtemperatur wurde das Reaktionsgemisch über Glasfaserfilterpapier filtriert. Für die GC-MS Messung wurde eine Probe direkt aus dieser Lösung entnommen. Wurde der Umsatz mittels NMR bestimmt, wurde kein interner Standard zugegeben und das Lösungsmittel wurde für die Messung komplett entfernt.

| **Substrat** | **Initiator** | **Verhältnis (Kat:Substrat)** | **Ausbeute [%]** | | **Wechselzahl** |
|---|---|---|---|---|---|
| 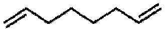 | **IMo1** | 1:563 | 38 | | 210 |
| | **IMo2** | 1:590 | 90 | | 532 |
| 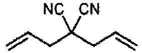 | **IMo1** | 1:1000 | 3 | | 30 |
| | **IMo2** | 1:1000 | 12 | | 120 |
| 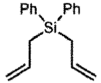 | **IMo1** | 1:440 | 41 | | 184 |
| | **IMo2** | 1:305 | 30 | | 90 |
| 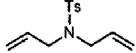 | **IMo1** | 1:1000 | 10 | | 100 |
| | **IMo2** | 1:1000 | 10 | | 100 |
| 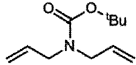 | **IMo1** | 1:448 | 24 | | 106 |
| | **IMo2** | 1:478 | 48 | | 217 |

| **Substrat** | **Initiator** | **Verhältnis (Kat:Substrat)** | **Ausbeute [%]** | **Wechselzahl** | ***trans:cis*** |
|---|---|---|---|---|---|
| 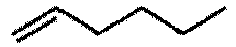 | **IMo1** | 1:481 | 68 | 327 | 1:0 |
| | **IMo2** | 1:836 | 4.3 | 37 | 1:0 |
| 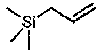 | **IMo1** | 1:571 | 100 | 571 | 1:1.3 |
| | **IMo2** | 1:548 | 100 | 548 | 1:1.1 |
| 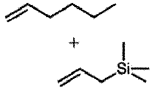 | **IMo1** | 1:123 | 100 | 123 | 1:0.6 |
| | **IMo2** | 1:76 | 100 | 76 | 1:0.7 |
| 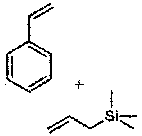 | **IMo1** | 1:251 | 100 | 251 | 1:9.4 |
| | **IMo2** | 1:59 | 100 | 59 | 1:21.8 |

| **Substrat** | **Initiator** | **Verhältnis (Kat:Substrat)** | **Ausbeute [%]** | | **Wechselzahl** |
|---|---|---|---|---|---|
| 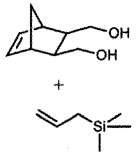 | **IMo1** | 1:59 | 42 | | 25 |

Strukturen hergestellter Wolfram-oxo-alkyliden-NHC-Komplexe. 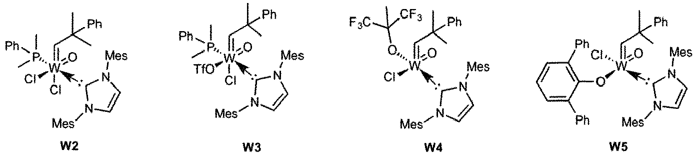 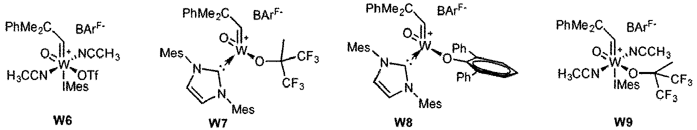 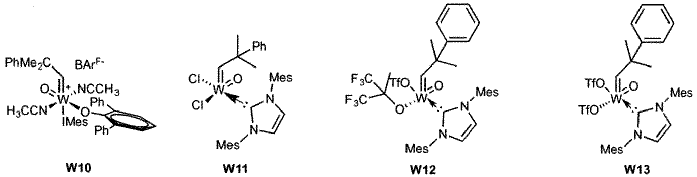 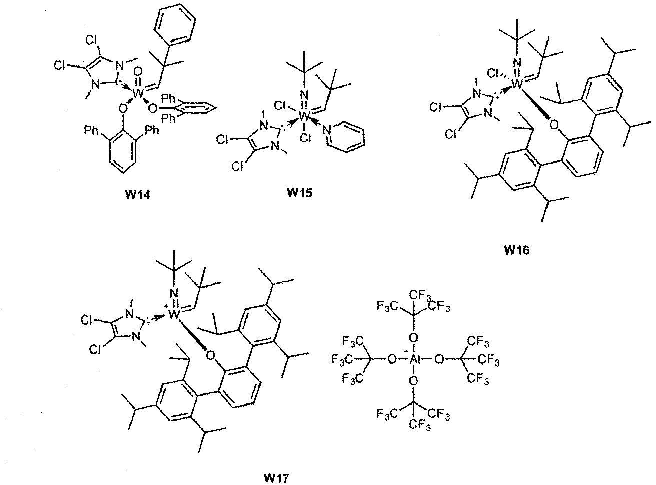 Mes = Mesityl, OTf⁻ = CF₃SO₃⁻, BAr^{F} = Tetrakis(3,5-bis(trifluormethyl)phenyl)borat, Me = Methyl.
Beispiel 38 (Herstellung von W(O)Cl₂(PPhMe₂)(IMes)(CHCMe₂Ph)) **(W2):** W(O)Cl₂(PPhMe₂)₂(CHCMe₂Ph) (2,42 g, 3,56 mmol) wurde in 50 mL Toluol gelöst. Eine Lösung von 1,3-bis(2,4,6-trimethylphenyl)imidazol-2-yliden (1,08 g, 3,56 mmol, 1 Äquiv.) in 10 mL Toluol wurde hergestellt. Beide Lösungen wurden bei-40°C 30 min gekühlt. Die kalte NHC Lösung wurde langsam zur gerührten Lösung von W(O)Cl₂(PPhMe₂)₂(CHCMe₂Ph) gegeben. Die Reaktionsmischung wurde 2 h bei Raumtemperatur gerührt. Die leicht trübe Lösung wurde über Celit filtriert und das Lösemittel wurde bei Unterdruck entfernt. Es wurde ein orangenes Öl erhalten. Das Öl wurde in 50 mL Diethylether aufgenommen und nochmals rasch filtriert. Ein gelber Feststoff beginnt nun auszufallen. Die Lösung wurde über Nacht bei -40°C im Kühlschrank gelagert. Ausbeute: 2,63 g (87%) eines blassgelben Feststoffs. ¹H NMR (400 MHz, C₆D₆): *δ* = 1,28 (d, 3H, PMe₂, *J*_{P-H} = 10,1 Hz), 1,32 (s, 3H, CMe₂Ph), 1,59 (s, 3H, CMe₂Ph), 1,66 (d, 3H, PMe₂, *J*_{P-H} = 10,3 Hz), 2,11 (s, 6H, Mes-Me), 2,24 (s, br, 6H, Mes-Me), 2,38 (s, br, 6H, Mes-Me), 6,16 (s, br, 2H, N-CH=CH-N), 6,80 (s, br, 2H, Mes-Ar), 6,83 (s, br, 2H, Mes-Ar), 6,87 (m, 3H, CMe₂Ph), 6,99-7,09 (m, 5H, Ar), 7,25 (m, 2H, Ar), 7,46 (m, 2H, PMe₂Ph), 11,9 (d, 1H, *J*_{P-H} = 3,6 Hz); ¹³C NMR (100 MHz, C₆D₆): *δ* = 14,0 (d, PMe₂, *J*_{C-P} = 34,8), 15,3 (d, PMe₂, *J*_{C-P} = 31,2), 19,5 (*o*-Mes-Me), 19,7 (*o*-Mes-Me), 21,2 (*p-*Mes-Me), 31,1 (CMe₂Ph), 32,9 (CMe₂Ph), 51,7 (CMe₂Ph), 124,4 (br, N-C=C-N), 126,0 (*p*-CMe₂Ph), 126,8 (*o*-CMe₂Ph), 128,2 (*m*-CMe₂Ph), 128,5 (*p*-PPh), 129,3 (d, *m*-PPh, *J*_{C-P} = 2,0 Hz), 129,5 (d, *o*-PPh, *J*_{C-P} = 2,7 Hz), 131,4 (d, *ipso*-PPh*, J*_{C-P} = 8,6 Hz), 135,9 (br), 137,6 (*m*-Mes), 138,7 (*o*-Mes), 152,3 (*ipso*-CMe₂Ph), 193,1 (d, N-C-N, *J*_{C-P} = 71,1 Hz) 309,5 (W=C, *J*_{C-H} = 125,3 Hz); ³¹P NMR (160 MHz, C₆D₆): *δ* = 8,28 (P-W), -33,2 (PMe₂Ph). CHN Anal. ber. für C₃₉H₄₇Cl₂N₂OPW : C, 55,40; H, 5,60; N, 3,31. Gefunden: C, 55,58; H, 5,74; N, 3,32.
Beispiel 39 (Herstellung von W(O)(OTf)Cl(PPhMe₂)(IMes)(CHCMe₂Ph)) **(W3):**
W(O)Cl₂(PPhMe₂)(IMes)(CHCMe₂Ph) (0,067 g, 0,08 mmol) wurde in 2 mL Dichlormethan gelöst und bei -40 °C gekühlt. Die kalte Lösung wurde zu festem Silbertriflat (0,020 g, 1 Äquiv.) gegeben und stark gerührt. Es bildete sich ein weißer Niederschlag. Die Suspension wurde 30 Minuten unter Ausschluss von Licht gerührt und über Celit abfiltriert. Nachdem das Lösemittel entfernt wurde, wurde das gelbe Öl nochmal in 1 mL Dichlormethan aufgenommen und ein weiteres Mal filtriert. Um Reste von Silberchlorid zu entfernen muss der Schritt einige Male wiederholt worden. Ausbeute: 0,061 g (81%) eines blassgelben Feststoffs. ¹H NMR (400 MHz, CD₂Cl₂): *δ* = 0,97 (s, 3H, CMe₂Ph), 1,15 (d, 3H, PMe₂, *J*_{P-H} = 10,52 Hz), 1,36 (d, 3H, PMe₂, *J*_{P-H} = 10,53 Hz), 1,81 (s, 3H, CMe₂Ph), 1,97 (s, 6H, Mes-Me), 2,16 (s, 6H, Mes-Me), 2,39 (s, 6H, Mes-Me), 6,92 (s, br, 2H, Mes-Ar), 6,93-7,10 (m, 2H, Ar), 7,11 (s, br, 2H, Mes-Ar), 7,11-7,16 (m, 2H, Ar), 7,21-7,38 (m, 6H, Ar), 7,40 (s, 2H, N-CH=CH-N), 7,4-7,5 (m, 1H, Ar), 10,08 (d, 1H, W=CH, *J*_{P-H} = 2,2 Hz); ¹³C NMR (100 MHz, CD₂Cl₂): *δ* = 11,60 (d, PMe₂, *J*_{C-P} = 35,4), 13,9 (d, PMe₂, *J*_{C-P} = 31,2), 18,7 (*p*-Mes-Me), 21,5 (*o*-Mes-Me), 28,7 (CMe₂Ph), 32,7 (CMe₂Ph), 52,0 (CMe₂Ph), 126,1, 126,3 (br, N-C=C-N), 128,0, 128,7, 129,5, 129,5, 129,6, 129,6, 130,5 (d, PPh, *J*_{C-P} = 26,3 Hz), 131,4 (d, PPh, *J*_{C-P} = 9,3 Hz), 131,7 (d, PPh, *J*_{C-P} = 2,8 Hz), 134,5 (*p*-Mes), 135,4 (*m*-Mes), 136,5 (*o*-Mes), 141,4, 147,9 (*ipso*-CMe₂Ph), 191,1 (d, N-C-N, *J*_{C-P} = 55,1 Hz), 302,4 (d, W=C, *J*_{C-H} = 116,9 Hz, *J*_{C-P} = 9,5 Hz); ¹⁹F NMR (375 MHz, CD₂Cl₂): *δ* = -78,82 (OSO₂CF₃); ³¹P NMR (160 MHz, CD₂Cl₂): *δ* = 17,34. CHN Anal. ber. für C₄₀H₄₈ClF₃N₂O₄PSW : C, 50,04; H, 5,04; N, 2,92. Gefunden: C, 49,34; H, 4,80; N, 2,89.
Beispiel 40 (Herstellung von W(O)(OCCH₃(CF₃)₂)Cl(IMes)(CHCMe₂Ph)) **(W4):**
In der Glove Box wurde W(O)Cl₂(PPhMe₂)(IMes)(CHCMe₂Ph) (0,568 g, 0,67 mmol) in einem 25 mL Schlenk-Kolben vorgelegt. Die Verbindung wurde in 10 mL Toluol gelöst und für 30 min bei -40 °C. Anschließend wurde LiOCMe(CF₃)₂ (0,170 g, 0,67 mmol, 1 Äquiv.) als Feststoff zugegeben. Die Suspension färbte sich dunkelorange. Nachdem 3 h bei Raumtemperatur gerührt wurde, wurde die Suspension filtriert und das Lösemittel entfernt. Es wurde ein dunkelorangenes Öl erhalten. Dieses wurde mit 5 mL n-Pentan gewaschen und in einer minimalen Menge Diethylether aufgenommen. Die Lösung wurde über Nacht bei -40 °C gelagert. Dabei fiel ein hellgelber Feststoff aus. Der Feststoff wurde abfiltriert und die Mutterlauge weiter eingeengt um eine zweite Fraktion des Produktes auszufällen. Die vereinten Fraktionen können nochmal aus Diethylether umkristallisiert werden. Das Produkt wird als hellgelber Feststoff oder als gelbe Kristalle erhalten (0,470 g, 82%). ¹H NMR (400 MHz, C₆D₆): *δ* = 1,49 (m, 3H, CMe(CF₃)₂), 1,54 (s, 3H, CMe₂Ph), 1,60 (s, 3H, CMe₂Ph), 1,91 (s, 6H, Mes-Me), 2,05 (s, 6H, Mes-Me), 2,14 (s, 6H, Mes-Me), 5,97 (s, 2H, N-CH=CH-N), 6,39 (s, br, 2H, Mes-Ar), 6,69 (s, br, 2H, Mes-Ar), 7,00 (m, 5H, Ar), 9,76 (s, 1H, W=CH); ¹³C NMR (100 MHz, CD₂Cl₂): *δ* = 17,3 (OCMe(CF₃)₂), 19,1 (*o*-Mes-Me), 19,1 (*o*-Mes-Me), 21,3 (*p*-Mes-Me), 28,8 (CMe₂Ph), 33,4 (CMe₂Ph), 50,3 (CMe₂Ph), 78,4 (m, CMe(CF₃)₂, 3H), 124,7 (N-C=C-N), 126,3 (*p*-CMe₂Ph), 126,6 (*o*-CMe₂Ph), 128,5 (*m*-CMe₂Ph), 129,9, 135,6 (*p*-Mes), 135,9 (*m*-Mes), 137,2 (*o*-Mes), 140,5 (*ipso*-Mes), 151,0 (CMe₂Ph), 192,1 (N-C-N), 282,1 (W=C, *J*_{C-H} = 121,3 Hz); ¹⁹F NMR (375 MHz, C₆D₆): *δ* =-76,70-78,00 (dq). CHN Anal. ber. für C₃₅H₃₉ClF₆N₂O₂W : C, 49,28; H, 4,61; N, 3,28. Gefunden: C, 49,24; H, 4,73; N, 3,28.
Beispiel 41 (Herstellung von W(O)(2,6-diphenylphenolat)Cl(IMes)(CHCMe₂Ph)) **(W5):**
W(O)Cl₂(PPhMe₂)(IMes)(CHCMe₂Ph) (0,850 g, 1 mmol) wurde in 30 mL Toluol gelöst. Li-2,6-diphenylphenolat (0,266 g, 1,06 mmol, 1,05 Äquiv.) wurde als Feststoff bei Raumtemperatur zugegeben. Die Lösung wurde trüb. Das Reaktionsgemisch wurde 12 h bei Raumtemperatur gerührt. Das Toluol wurde zum halben Volumen reduziert und der farblose Niederschlag wurde über Celit abfiltriert. Das Filtrat wurde weiter eingeengt bis sich erneut Niederschlag bildete. Die Lösung wurde bei -40 °C über Nacht im Kühlschrank gelagert. Ein gelborangener Feststoff wurde abfiltriert (0,830 g, 90%). ¹H NMR (400 MHz, CD₂Cl₂): *δ* = 1,33 (s, 3H, CMe₂Ph), 1,40 (s, 6H, Mes-Me), 1,55 (s, 3H, CMe₂Ph), 1,80 (s, 6H, Mes-Me), 2,33 (s, 6H, Mes-Me), 6,66 (m, 2H, Ar), 6,81 (s, 2H, N-CH=CH-N), 6,83 (s, br, 2H, Mes-Ar), 6,86 (m, 1H, Ar), 6,89 (br, 2H, Mes-Ar), 6,97 (m, 4H, Ar), 7,09 (m, 1H, Ar), 7,17 (m, 2H, Ar), 7,22-7,36 (m, 5H, Ar), 7,40 (m, 2H, Ar), 7,81 (m, 2H, Ar), 9,90 (s, 1H, W=CH); ¹³C NMR (100 MHz, CD₂Cl₂): *δ* = 18,6 (*o*-Mes-Me), 19,2 (*o*-Mes-Me), 21,4 (*p*-Mes-Me), 29,6 (CMe₂Ph), 32,3 (CMe₂Ph), 50,3 (CMe₂Ph), 120,5, 125,5, 126,4, 126,5, 127,1, 128,4, 129,2, 129,3, 129,5, 130,5, 130,8, 131, 131,8, 133,2, 134,8, 135,4, 135,4, 136,6, 139,8, 141,1, 142, 150,8 (*ipso-*CMe₂Ph), 159,6 (*ipso*-O-Ar), 191,6 (N-C-N), 288 (W=C, *J*_{C-H} = 123,1 Hz), 298,2 (*J*_{C-H} = 123,3 Hz). CHN Anal. ber. für C₄₉H₄₉ClN₂O₂W : C, 64,16; H, 5,38; N, 3,05. Gefunden: C, 64,16; H, 5,41; N, 3,13.
Beispiel 42 (Herstellung von [W(O)(CHCMe₂Ph)(IMes)(OTf)(MeCN)₂ B(3,5-(CF₃)₂-C₆H₃)₄]) **(W6):**
Die Verbindung wurde *in situ,* unmittelbar vor den durchgeführten Katalysen, hergestellt. W(O)(OTf)Cl(PPhMe₂)(IMes)(CHCMe₂Ph) wurde in 5 mL Dichlormethan gelöst und 30 min bei -40°C gekühlt. Anschließend wurde Ag(MeCN)₂B(Ar^{F})₄ (2,05 Äquiv.) als Feststoff zu gegeben. Es bildete sich sofort ein farbloser Niederschlag. Die Suspension wurde 30 min unter Lichtausschluss bei Raumtemperatur gerührt. Danach wurde der Niederschlag über Celit abfiltriert. Die stark gelbe Lösung wurde als Katalysatorstammlösung genutzt. ¹H NMR (400 MHz, CD₂Cl₂): *δ* = 1,49 (s, 3H, CMe₂Ph), 1,92 (s, 3H, CMe₂Ph), 2,03 (s, 6H, MeCN), 2,12 (s, 6H, Mes-Me), 2,18 (s, 6H, Mes-Me), 2,37 (s, 6H, Mes-Me), 6,96 (s, br, 2H, Mes-Ar), 7,10 (s, br, 2H, Mes-Ar), 7,20-7,38 (m, 5H, Ar), 7,42 (s, 2H, N-CH=CH-N), 7,63 (s, br, 4H, BAr^{F}), 7,80 (s, br, 8H, BAr^{F}), 11,47 (s, 1H, W=CH); ¹³C NMR (100 MHz, CD₂Cl₂): *δ* = 2,9 (MeCN), 18,5 (*o*-Mes-Me), 19,0 (o-Mes-Me), 21,4 (*p*-Mes-Me), 29,2 (CMe₂Ph), 31,0 (CMe₂Ph), 53,5 (CMe₂Ph), 118,2 (sept, *J*_{C-F} = 3,8 Hz, *p*-CH (BAr^{F})), 125,3 (q, *J*_{C-F} = 272,4 Hz, 4x2CF₃ (BAr^{F})), 126,9, 127,2, 127,4, 129,2, 129,7 (qq, *J*_{C-F} = 31,6 Hz, *J*_{C-B} = 2,7 Hz, 4xC-CF3 (BAr^{F})), 129,8, 130,1, 130,9, 132,4, 134,6, 135,5 (s, br, 4x2C, *o*-CH (BAr^{F})), 136,9, 141,7 (*ipso*-Mes), 148,8 (*ipso*-CMe₂Ph), 162,4 (q, *J*_{C-B} = 49,8 Hz, 4xBC(BAr^{F})), 187,0 (N-C-N), 324,3 (W=C, *J*_{C-H} = 125,3 Hz); ¹⁹F NMR (375 MHz, CD₂Cl₂): δ = -62,77 (BAr^{F}), -77,82 (O-SO₂CF₃).
Beispiel 43 (Herstellung von [W(O)(CHCMe₂Ph)(IMes)(OCCH₃(CF₃)₂) B(3,5-(CF₃)₂-C₆H₃)₄]) **(W7):**
W(O)(OCCH₃(CF₃)₂)Cl(IMes)(CHCMe₂Ph) (0,032 g, 0,0375 mmol) wurde in 5 mL Dichlormethan gelöst und bei -40°C für 30 min gekühlt. Die Lösung wurde zu festem NaB(Ar^{F})₄ (0,0333 g, 1 Äquiv.) gegeben. Die Suspension wurde 30 min bei Raumtemperatur gerührt. Ein farbloser Niederschlag bildete sich. Die Lösung wurde 30 min bei -40°C gelagert und kalt durch einen Glasfaserfilter filtriert. Das Filtrat wurde im Vakuum zu einem Drittel des Volumens eingeengt und nochmals filtriert. Nachdem das Lösungsmittel entfernt wurde, wurde ein orangenes Öl erhalten. Dieses wurde mit n-Pentan gerührt bis ein orangener Feststoff entstand. Die Pentanphase wurde dekantiert und der Feststoff am Vakuum getrocknet. Ausbeute: 0,055 g (87%). ¹H NMR (400 MHz, CD₂Cl₂): *δ* = 1,29 (s, 3H, CMe₂Ph), 1,32 (sept, 3H, CCH₃(CF₃)₂), 1,64 (s, 3H, CMe₂Ph), 1,94 (s, 6H, Mes-Me), 2,05 (s, 6H, Mes-Me), 2,37 (s, 6H, Mes-Me), 7,02 (s, br, 2H, Mes-Ar), 7,16 (s, br, 2H, Mes-Ar), 7,18-7,31 (m, 5H, Ar), 7,57 (s, br, 4H, BAr^{F}), 7,68 (s, 2H, N-CH=CH-N), 7,74 (s, br, 8H, BAr^{F}), 10,52 (s, 1H, W=CH); ¹³C NMR (100 MHz, CD₂Cl₂): *δ* = 17,8 (*o-*Mes-Me), 17,9 (*o*-Mes-Me), 19,3 (OCMe(CF₃)₂), 21,5 (*p*-Mes-Me), 29,4 (CMe₂Ph), 31,9 (CMe₂Ph), 52,7 (CMe₂Ph), 86,3 (m, OCMe(CF₃)₂), 118,1 (sept, *J*_{C-F} = 3,8 Hz, *p*-CH (BAr^{F})), 123,8 (q, *J*_{C-F} = 273,4 Hz, 4x2CF₃ (BAr^{F})), 126,3 (N-C=C-N), 127,9 (*o*-Ar), 128,6 (*p*-Ar), 129,4 (*m*-Ar), 129,5 (qq, *J*_{C-F} = 31,6 Hz, *J*_{C-B} = 2,7 Hz, 4xC-CF3 (BAr^{F})), 131,1 (*m*-Mes), 131,2 (*m*- Mes), 133,0 (*o-* Mes), 134,3 (*o*-Mes), 135,3 (*p*-Mes), 135,4 (s, br, 4x2C, *o*-CH (BAr^{F})), 143,6 (*ipso*-Mes), 147,6 (*ipso-*CMe₂Ph), 162,4 (q, *J*_{C-B} = 49,8 Hz, 4xBC(BAr^{F})), 181,8 (N-C-N), 297,3 (W=C, *J*_{C-H} = 123,3 Hz); ¹⁹F NMR (375 MHz, CD₂Cl₂): *δ* = -62,86 (BAr^{F}), -78,61 (dq). CHN Anal. ber. für C₆₇H₅₁BF₃₀N₂O₂W : C, 47,88; H, 3,06; N, 1,67. Gefunden: C, 47,96; H, 3,279; N, 1,84.
Beispiel 44 (Herstellung von [W(O)(CHCMe₂Ph)(IMes)(2,6-diphenylphenolat) B(3,5-(CF₃)₂-C₆H₃)₄]) **(W8):**
W(O)(2,6-diphenylphenolat)Cl(IMes)(CHCMe₂Ph) (0,0171 g, 0,0186 mmol) wurde in 5 mL Dichlormethan gelöst und bei -40°C für 30 min gekühlt. Die Lösung wurde zu festem NaB(Ar^{F})₄ (0,0165 g, 1 Äquiv.) gegeben. Die Suspension wurde 30 min gerührt. Ein farbloser Niederschlag bildete sich. Die Reaktionsmischung wurde 30 min bei -40°C gekühlt und filtriert. Das Filtrat wurde zu einem Drittel eingeengt und nochmal filtriert. Nachdem das Lösemittel entfernt wurde, wurde ein gelber Schaum erhalten. Dieser wurde mit n-Pentan gerührt bis sich ein gelber Niederschlag bildete. Die Pentanphase wurde dekantiert und der Feststoff am Vakuum getrocknet. Ausbeute 0,029 g (89%). ¹H NMR (400 MHz, CD₂Cl₂): *δ* = 0,72 (s, 3H, CMe₂Ph), 1,58 (s, 3H, CMe₂Ph), 1,67 (s, 6H, Mes-Me), 1,71 (s, 6H, Mes-Me), 2,34 (s, 6H, Mes-Me), 6,83 (s, br, 2H, Mes-Ar), 6,99 (s, br, 2H, Mes-Ar), 7,01-7,09 (m, 4H), 7,17-7,27 (m, 12H), 7,30-7,40 (m, 4H), 7,45-7,52 (m, 1H), 7,56 (s, br, *p*-CH (BAr^{F})), 7,73 (s, 8H, *o*-CH (BAr^{F})), 11,82 (s, 1H, W=CH); ¹³C NMR (100 MHz, CD₂Cl₂, MeCN adduct): *δ* = 2.5 (MeCN), 18,6 (*o*-Mes-Me), 18,9 (*o-*Mes-Me), 21,4 (*p*-Mes-Me), 30,1 (CMe₂Ph), 30,5 (CMe₂Ph), 52,1 (CMe₂Ph), 118,1 (sept, *J*_{C-F} = 3,8 Hz, *p*-CH (BAr^{F})), 123,5 (MeCN), 123,8 (q, *J*_{C-F} = 273,4 Hz, 4x2CF₃ (BAr^{F})), 126,7 (br, N-C=C-N), 126,8, 127,2, 128,8, 129,5 (qq, *J*_{C-F} = 31,6 Hz, *J*_{C-B} = 2,7 Hz, 4xC-CF3 (BAr^{F})), 129,9, 130,0, 135,4, 135,4 (s, br, 4x2C, *o*-CH (BAr^{F})), 135,5, 135,6, 141,4 (*ipso*-Mes), 147,3 (*ipso*-CMe₂Ph), 157,5 (*ipso*-O-Ar), 162,4 (q, *J*_{C-B} = 49,8 Hz, 4xBC(BAr^{F})), 188,6 (N-C-N), 309,9 (W=C, *J*_{C-H} = 121,2 Hz); ¹⁹F NMR (375 MHz, CD₂Cl₂): *δ* = -62,87 (BAr^{F}). CHN Anal. ber. für C₈₁H₆₁BF₂₄N₂O₂W : C, 55,75; H, 3,52; N, 1,61. Gefunden: C, 55,69; H, 3,913; N, 1,72.
Beispiel 45 (Herstellung von [W(O)(CHCMe₂Ph)(IMes)(OCCH₃(CF₃)₂)(MeCN)₂ B(3,5-(CF₃)₂-C₆H₃)₄]) **(W9):**
Die Verbindung wurde *in situ,* unmittelbar vor den durchgeführten Katalysen, hergestellt. W(O)(OCCH₃(CF₃)₂)Cl(IMes)(CHCMe₂Ph) wurde in 5 mL Dichlormethan gelöst und 30 min bei -40°C gekühlt. Anschließend wurde Ag(MeCN)₂B(Ar^{F})₄ (1,0 Äquiv.) als Feststoff zu gegeben. Es bildete sich sofort ein farbloser Niederschlag. Die Suspension wurde 30 min unter Lichtausschluss bei Raumtemperatur gerührt. Danach wurde der Niederschlag über Celit abfiltriert. Die stark gelbe Lösung wurde als Katalysatorstammlösung genutzt.
Beispiel 46 (Herstellung von [W(O)(CHCMe₂Ph)(IMes)(2,6-diphenylphenolat)(MeCN)₂ B(3,5-(CF₃)₂-C₆H₃)₄]) **(W10):**
Die Verbindung wurde *in situ,* unmittelbar vor den durchgeführten Katalysen, hergestellt. W(O)(2,6-diphenylphenolat)Cl(IMes)(CHCMe₂Ph) wurde in 5 mL Dichlormethan gelöst und 30 min bei -40°C gekühlt. Anschließend wurde Ag(MeCN)₂B(Ar^{F})₄ (1,0 Äquiv.) als Feststoff zu gegeben. Es bildete sich sofort ein farbloser Niederschlag. Die Suspension wurde 30 min unter Lichtausschluss bei Raumtemperatur gerührt. Danach wurde der Niederschlag über Celit abfiltriert. Die stark gelbe Lösung wurde als Katalysatorstammlösung genutzt.
Beispiel 47 (Herstellung von W(O)Cl₂(IMes)(CHCMe₂Ph)) **(W11):**
W(O)(2,6-diphenylphenolat)Cl(IMes)(CHCMe₂Ph) (0,023 g, 0,249 mmol) wurde in 2 mL Acetonitril gelöst und bei -40°C für 30 min gekühlt. Anschließend wurde eine kalte Lösung von AlCl₃ (0,0033 g, 0,249 mmol, 1 Äquiv.) in 1 mL Acetonitril zugegeben. Die Lösung färbte sich stark gelb und wurde 3 h bei Raumtemperatur gerührt. Danach wurde das Lösemittel entfernt und der ölige Rest in 1 mL Dichlormethan aufgenommen. Die Lösung wurde filtriert und auf 0,3 mL eingeengt. Nach einigen Tagen bildeten sich gelbe Kristalle des Produktes. Ausbeute: 0,013 g (74%).
Beispiel 48 (Herstellung von W(O)(OTf)(OCCH₃(CF₃)₂)(IMes)(CHCMe₂Ph)) **(W12):** W(O)(OCCH₃(CF₃)₂)Cl(IMes)(CHCMe₂Ph) (0,0495 g, 0,058 mmol) wurde in 2 mL Dichlormethan gelöst und bei -40°C 30 min gekühlt. Zur kalten Lösung wurde Silbertriflat (0,015 g, 0,058 mmol, 1 Äquiv.) gegeben. Sofort war ein farbloser Niederschlag zu beobachten. Die Suspension wurde 1 h unter Lichtausschluss gerührt und über Celit filtriert. Das Lösemittel wurde entfernt. Es blieb ein gelbes Öl welches in 1 mL Dichlormethan aufgenommen wurde und nochmals filtriert wurde. Dieser Schritt wurde einige Male wiederholt um Reste von Silberchlorid zu entfernen. Das Produkt wird als gelber Feststoff erhalten. Ausbeute: 0,041 g (74%). ¹H NMR (400 MHz, CD₂Cl₂): *δ* = 0,74 (s, 3H, CMe₂Ph), 0,81 (s, 3H, CMe(CF₃)₂), 1,45 (s, 3H, CMe₂Ph), 2,13 (s, 6H, Mes-Me), 2,18 (s, 6H, Mes-Me), 2,31 (s, 6H, Mes-Me), 6,96 (s, br, 2H, Mes-Ar), 7,03 (s, br, 2H, Mes-Ar), 7,05-7,11 (m, 1H, Ar), 7,14-7,25 (m, 4H, Ar), 7,26 (s, 2H, N-CH=CH-N), 10,69 (s, 1H, W=CH); ¹³C NMR (100 MHz, CD₂Cl₂): *δ* = 17,6 (OCMe(CF₃)₂), 18,4 (*o*-Mes-Me), 18,5 (*o*-Mes-Me), 21,3 (*p*-Mes-Me), 29,0 (CMe₂Ph), 29,9 (CMe₂Ph), 50,8 (CMe₂Ph), 82,3 (m, CMe(CF₃)₂), 125,8, 126,1, 126,5, 128,6, 130,4, 130,4, 136,4 (*p*-Mes), 137,1 (*m*-Mes), 141,7 (*ipso*-Mes), 150,9 (CMe₂Ph), 186,0 (N-C-N), 278,2 (W=C, *J*_{C-H} = 127,1 Hz); ¹⁹F NMR (375 MHz, C₆D₆): *δ* = -77,71 (s, br, OSO₂CF₃),-77,76 (m, br, OCCH₃(CF₃)₂).
Beispiel 49 (Herstellung von W(O)(OTf)₂(IMes)(CHCMe₂Ph)) **(W13):**
W(O)Cl₂(PPhMe₂)(IMes)(CHCMe₂Ph) (0,26 g, 0,83 mmol) wurde in 8 mL Dichlormethan gelöst. Die Lösung wurde 30 min bei -40°C gekühlt. Unter Rühren wurde Silbertriflat (0,200 g, 0,766 mmol, 2,01 Äquiv.) als Feststoff zugegeben. Es fiel sofort ein farbloser Niederschlag aus. Die Suspension wurde unter Lichtausschluss 1 h bei Raumtemperatur gerührt. Die Farbe änderte sich dabei zu gelb. Die Lösung wurde über Celit filtriert. Das Lösemittel wurde entfernt. Es wurde ein hellgelber Schaum erhalten. Dieser wurde in einer kleinen Menge Dichlormethan gelöst und nochmal filtriert. Dieser Schritt wurde einige Male wiederholt um Silberchloridreste zu entfernen. Das Rohprodukt kann aus Dichlormethan/Diethylether umkristallisiert werden. Das Produkt wird als gelber kristalliner Feststoff erhalten. Ausbeute: 0,303 g (85%).
Beispiel 50 (Herstellung von W(O)(2,6-Diphenylphenolat)₂(1,3-Me₂-4,5-Cl₂-imidazol-2-yliden)(CHCMe₂Ph)) **(W14):**
W(O)(2,6-Diphenylphenolat)₂(PMePh₂)(CHCMe₂Ph) (0,12 g, 0,117 mmol) wurde in 8 mL Toluol gelöst. 1,3-Me₂-4,5-Cl₂-imidazol-2-yliden-AgI (0,048 g, 0,12 mmol, 1,01 Äquiv.) wurde als Feststoff zugegeben. Die Suspension wurde für 1 Stunde bei 70°C im Ultraschallbad gehalten. Anschließend wurde die Suspension über Celit filtriert und das Lösemittel entfernt. Der hellgelbe Feststoff wurde in 4 mL Dichlormethan aufgenommen und ein weiteres Mal filtriert. Das Lösemittel wurde entfernt und der ölige Feststoff mit n-Pentan gewaschen. Das Produkt wurde als hellorangener Feststoff erhalten. Ausbeute: 0,1 g (86%). ¹H NMR (400 MHz, C₆D₆): δ = 1,08 (s, 3H, CMe₂Ph), 1,44 (s, 3H, CMe₂Ph), 6,73-6,82 (m, 6H, Ar), 6,96-7,03 (m, 8H, Ar), 7,05-7,12 (m, 6H, Ar), 7,17-7,28 (m, 12H, Ar), 7,47 (d, 2H, *p*-Ar, *J* = 7,56 Hz), 7,47 (m, 4H, *p*-Ar), 10,25 (s, 1H, W=CH); ¹³C NMR (100 MHz, C₆D₆): δ = 29,4 (CMe₂Ph), 31,5 (CMe₂Ph), 36,3 (Me-NHC), 48,5 (CMe₂Ph), 117,1 (NC=CN), 125,6, 125,8, 126,4, 126,8, 128,9, 129,8, 130,4, 131,1, 132,1, 133,2, 133,4, 133,9, 140,6 (*ipso*-Ar), 142,0 (*ipso*-Ar), 151,5 (*ipso*-CMe₂Ph), 157,8 (*ipso*-O-Ar), 163,4 (*ipso*-O-Ar), 191,3 (N-C-N), 279,5 (W=C, *J*_{C-H} = 124,0 Hz).
Beispiel 51 (Herstellung von W(N*t*Bu)(Cl)₂(1,3-Me₂-4,5-Cl₂-imidazol-2-yliden) (Pyridin)(CHCMe₃)) **(W15):**
W(N*t*Bu)(Cl)₂(Pyridin)₂(CHCMe₃) (0,2 g, 0,36 mmol) wurde in 8 mL Dichlorethan gelöst. 1,3-Me₂-4,5-Cl₂-imidazol-2-yliden-AgI (0,145 g, 0,36 mmol, 1,0 Äquiv.) wurde als Feststoff zugegeben. Die Suspension wurde für 1 Stunde bei 70°C im Ultraschallbad gehalten. Anschließend wurde die Suspension über Celit filtriert und das Lösemittel entfernt. Der hellgelbe Feststoff wurde in 4 mL Dichlormethan aufgenommen und ein weiteres Mal filtriert. Das Lösemittel wurde entfernt und der Feststoff mit n-Pentan gewaschen. Das Produkt wurde als hellorangener Feststoff erhalten. Es bilden sich zwei Isomere mit gleichen Anteilen. Ausbeute: 0,19 g (82%). ¹H NMR (400 MHz, CD₂Cl₂): δ = 1,21 (s, 9H, *t*Bu), 1,28 (s, 9H, *t*Bu), 1,38 (s, 9H, *t*Bu), 1,41 (s, 9H, *t*Bu), 3,76 (s, 6H, Me₂-NHC), 4,21 (s, 6H, Me₂-NHC), 7,28 (m, 2H, pyr), 7,41 (m, 2H, pyr), 7,68 (m, 1H, pyr), 7,86 (m, 1H, pyr), 8,60 (m, br, 2H, pyr), 9,40 (m, 2H, pyr), 10,58 (s, 1H, W=CH), 12,0 (s, 1H, W=CH); ¹³C NMR (100 MHz, CD₂Cl₂): δ = 15,7, 31,0, 31,4, 31,4, 32,6, 34,3, 34,8, 39,2, 40,4, 44,8, 45,4, 66,2 (CMe₃), 69,6 (CMe₃), 118,9, 119,0, 124,2, 124,8, 124,9, 136,3, 139,1, 139,4, 150,5, 156,8, 157,1, 191,0 (N-C-N), 191,2 (N-C-N), 279,9 (W=CH), 301,0 (W=CH).
Beispiel 52 (Herstellung von W(N*t*Bu)(Cl)(1,3-Me₂-4,5-Cl₂-imidazol-2-yliden)(OHIPT)(CHCMe₃)) **(W16):** W(N*t*Bu)(Cl)₂(1,3-Me₂-4,5-Cl₂-imidazol-2-yliden)(Pyridin)(CHCMe₃) (0.15 g, 0.234 mmol) wurde in 8 mL Benzol gelöst. Lithium 2,6-Di-(2,4,6-triisopropylphenyl)phenolat (0.118 g, 0.234 mmol, 1,0 Äquiv.) wurde als Feststoff zugegeben. Die Lösung wurde über Nacht bei Raumtemperatur gerührt. Es entstand ein farbloser Niederschlag. Anschließend wurde die Suspension über Celit filtriert und das Lösemittel entfernt. Der dunkelorangene Schaum wurde in 4 mL Toluol aufgenommen und ein weiteres Mal filtriert. Das Lösemittel wurde entfernt und der Feststoff aus n-Pentan umkristallisiert. Das Produkt wurde als orangener Feststoff erhalten. Ausbeute: 0.13 g (87%). ¹H NMR (400 MHz, C₆D₆): δ = 0.72 (d, 3H, iPr), 1.00 (m, 6H, iPr), 1.14 (d, 3H, iPr), 1.20 (s, 9H, *t*Bu), 1.24 (s, 9H, *t*Bu), 1.26 (m, 6H, iPr), 1.30 (m, 9H, iPr), 1.36 (m, 6H, iPr), 1.66 (m, 6H, iPr), 2.70-3.30 (m, 10H, Me₂-NHC, CH-iPr), 3.93 (m, 2H, CH-iPr), 6.9 (m, 1H, Ar), 7.02 (m, 1H, Ar), 7.13 (m, 1H, Ar), 7.16 (m, 1H, Ar), 7.26 (m, 2H, Ar), 7.40 (m, 1H, Ar), 10.37 (s, 1H, W=CH); ¹³C NMR (100 MHz, C₆D₆): δ = 14.3, 22.3, 22.7, 22.9, 24.2, 24.5, 24.7, 24.8, 25.2, 25.5, 27.1, 27.6, 30.4, 31.0, 31.5, 34.2, 34.5, 34.6, 34.8, 43.8, 68.1 (CMe₃), 118.2, 119.7, 120.1, 121.9, 122.7, 131.5, 132.0, 132.4, 138.1, 138.4, 147.2, 147.3, 147.4, 148.1, 149.5, 149.9, 162.0, 192.0 (N-C-N), 281.9 (W=CH).
Beispiel 53 (Herstellung von [W(N*t*Bu)(1,3-Me₂-4,5-Cl₂-imidazol-2-yliden)(OHIPT)(CHCMe₃) Alpf*t*Bu]) **(W17):** W(N*t*Bu)(Cl)(1,3-Me₂-4,5-Cl₂-imidazol-2-yliden)(OHIPT)(CHCMe₃) (0.0331 g, 0.0323 mmol) wurde in 3 mL Dichlormethan gelöst. Lithium Tetrakis-(nonafluoro-t-butoxy)aluminat (Li-Alpf*t*Bu, 0.0315 g, 0.0323 mmol, 1,0 Äquiv.) wurde als Feststoff zugegeben. Die Lösung wurde 1h bei Raumtemperatur gerührt. Es entstand ein farbloser Niederschlag. Die Lösung färbte sich dabei stark gelb. Anschließend wurde die Suspension über Celit filtriert und das Lösemittel entfernt. Der gelbe Schaum wurde in 4 mL Toluol aufgenommen und ein weiteres Mal filtriert. Das Lösemittel wurde entfernt und das gelbe Öl mit n-Pentan gerührt. Es entstand ein gelber Feststoff. Das Produkt wurde abfiltriert und am Vakuum getrocknet. Ausbeute: 0.055 g (87%). ¹H NMR (400 MHz, CD₂Cl₂): δ = 0.81 (d, 6H, iPr), 0.95 (d, 6H, iPr), 0.99 (d, 6H, iPr), 1.01 (s, 9H, *t*Bu), 1.02 (d, 6H, iPr), 1.09 (s, 9H, *t*Bu), 1.23 (d, 12H, iPr), 2.49 (sept, 2H, CH-iPr), 2.58 (sept, 2H, CH-iPr), 2.89 (sept, 2H, CH-iPr), 3.29 (s, 6H, Me₂-NHC), 7.0 (m, 2H, Ar), 7.03 (m, 1H, Ar), 7.05 (s, 1H, Ar), 7.08 (m, 2H, Ar), 7.15 (m, 1H, Ar), 10.74 (s, 1H, W=CH); ¹³C NMR (100 MHz, CD₂Cl₂): δ = 24.2, 24.4, 24.5, 24.6, 24.8, 31.6, 32.7, 33.6, 34.7, 40.2, 47.2, 74.4, 120.4, 122.0, 122.3, 122.8, 123.3, 124.9, 131.8, 132.6, 133.2, 147.5, 147.8, 149.9, 158.6, 178.0, 289.0 (W=CH); ¹⁹F NMR (375 MHz, CD₂Cl₂): *δ* = -75.72 (s, CF₃).
Beispiel 54 (Allgemeine Vorschrift für *in situ* Katalysator Synthesen): Die Wolfram-oxo-Vorstufen **W3-W5** (ca. 0,05 mmol) wurden in 2 mL 1,2-Dichloroethan gelöst. Eine äquimolare Menge Ag(MeCN)₂B(Ar^{F})₄ (**W4, W5**) oder 2 äquiv. (**W3**) bzw. Überschuss AlCl₃ wurden zugegeben. Die Lösung wurde 30 min gerührt und filtriert. Das Filtrat wurde als Katalysatorstammlösung verwendet.
Beispiel 55 (Allgemeine Vorschrift für Ringschluss-, Homo- und Selbstmetathesen): Ungefähr 20 mg des Substrats wurden in ein 10 mL Schraubdeckelglas eingewogen. Die entsprechende Menge Lösemittel wurde zugegeben (0,1 M Lösung). Danach wurden 0,5 Äquiv. Dodekan (interner Standard) zugegeben. Ein Aliquot mit 1 mg Substrat wurde für die t₀ Probe genommen. Eine 0,0005 M Katalysatorstammlösung wurde hergestellt. Die entsprechende Menge Stammlösung wurde zur Substratlösung gegeben. Die Lösung wurde für den angegeben Zeitraum bei angegebener Temperatur gerührt. Die Reaktionen wurden durch Luftatmosphäre gestoppt und es wurde eine Probe für die GC-MS Analyse entnommen. Die genauen Monomer/Katalysator-Zusammensetzungen und die für diese bestimmten Wechselzahlen (TON = turn over number) sind Tabelle 8 zu entnehmen.
Beispiel 56 (Allgemeine Vorschrift für Kreuzmetathesen (CM) mit Allyltrimethylsilan): Es wurde dieselbe allgemeine Vorschrift befolgt wie für die Ringschlussmetathesen (Beispiel 53). Zum Substrat wurden lediglich zusätzlich 10 Äquivalente Allyltrimethylsilan zugegeben. Die genauen Monomer/KatalysatorZusammensetzungen und die für diese bestimmten Wechselzahlen (TON = turn over number) sind ebenfalls in Tabelle 8 wiedergegeben.
Beispiel 57 (Z-selektive Metathese): In einer Schutzgas Box wird 1-Octen (ca. 22 mg) in einem 4 mL Schraubdeckelglas eingewogen und in 1 mL Benzol gelöst. Unter Rühren wird eine Lösung von **W17** (3.6 mg) in Benzol (0.2 mL) zugegeben. Das Gemisch wird 1 Stunde bei Raumtemperatur gerührt. Zur Überprüfung des Reaktionsverlaufs und der Selektivität wird ein Aliquot entnommen und mit nicht getrocknetem CDCl₃ verdünnt um die Reaktion zu stoppen. Das Reaktionsgemisch wurde per ¹H-NMR analysiert (100% Umsatz >99.9% Z-konfiguriertes Produkt).

**Tabelle 8. TONs mit AlCl₃-aktiviertem 3 - 5 und mit kationischen Komplexen W6 - W8. Reaktionsbedingungen, wenn nicht anders angegeben: T = 25°C in 1,2-Dichloroethan für 4 h, Substrat : Katalysator 1 : 2000.**

| **Substrat** | **W6** | **W7** | **W8** | **W3^{[a]}** | **W4^{[a]}** | **W5^{[a]}** |
|---|---|---|---|---|---|---|
| *Ringschlussmetathese (RCM)* | | | | | | |
| Diallyldiphenylsilan | 4800^{[c]} | 3400^{[e]} | 7600^{[e]} | 0^{[e]} | 0^{[c]} | 0^{[c]} |
| N,N-Diallyl-*p-*toluolsulfonamid | 1700^{[c]} | 1350 | 1700^{[b]} | 0^{[c]} | 0^{[c]} | 0^{[c]} |
| Octa-1,7-dien | 970^{[c]} | 710 | 1500^{[b]} | 980^{[c]} | 4300^{[c]} | 4700^{[c]} |
| Diallylmalodinitril | 130^{[c]} | 660 | 1400^{[e]} | 0^{[c]} | 0^{[c]} | 0^{[c]} |
| Diallylether | 0 | 0 | 5700^{[e]} | 0^{[c]} | 0^{[c]} | 0^{[c]} |
| Diallylthioether | 4800^{[c]} | 0 | 4900^{[e]} | 4800^{[c]} | 1200 | 1400 |
| 4,4-Dicyano-octa-1,7-dien | 470^{[c]} | 430 | 2600^{[e]} | 0^{[c]} | 0^{[c]} | 0^{[c]} |
| Diethyldiallylmalonat | 1600^{[c]} | 660 | 3200^{[e]} | 0^{[c]} | 0^{[c]} | 0^{[c]} |

| *Homometathesis (HM), in Klammern* = *E-Anteil (%)* | | | | | | |
|---|---|---|---|---|---|---|
| Allylbenzol | 200 (55)^{[c]} | 480 (60) | 635 (85)^{[e]} | 640 (55) | 430 (55) | 410 (80) |
| 1-Hexen | 2000 (60)^{[c]} | 1640 (65) | 5400 (85)^{[e]} | 4900 (60)^{[c]} | 5000 (65) ^{[c]} | 9800 (80)^{[e]} |
| 1-Octen | 3300 (60)^{[c]} | 1320 (65) | 6100 (85)^{[e]} | 4830 (55) ^{[c]} | 5000 (60) ^{[c]} | 2000 (80) ^{[c]} |
| Allylphenylsulfid | 0 | 0 | 300 (>95)^{[d]} | 0 | 0 | 280 (>95)^{[d]} |
| Trimethylallylsilan | 4100 (55)^{[c]} | 1710 (55) | 1500 (60) | 0 | 0 | 0 |

| *Kreuzmetathese (CM)*^{[d]} *mit Allyltrimethylsilan, in Klammern* = *E-Anteil (%)* | | | | | | |
|---|---|---|---|---|---|---|
| Hex-5-en-1-ylacetat | 480 | 450 | 500 | 0 | 0 | 0 |
| | (55) | (60) | (80) | | | |
| 4-Octen | 500 | 490 | 500 | 0 | 0 | 0 |
| | (50) | (65) | (80) | | | |
| N-Phenyl-(1-phenyl-but-3-ene-1-yl)amin | 200 | 0 | 0 | 0 | 0 | 0 |
| | (60) | | | | | |

| *Selbstmetathese (SM), In Klammern* = *E-Anteil (%)* | | | | | | |
|---|---|---|---|---|---|---|
| Methyloleat | 1500 (60) | 0^{[c]} | 10000 (70)^{[f]} | 0^{[c]} | 0^{[c]} | 0^{[c]} |

| | | | | | | |
|---|---|---|---|---|---|---|
| [a] aktiviert mit Überschuss AlCl₃, CH₂Cl₂, Raumtemperatur, Katalysator : Substrat = 1 : 5000. [b] Katalysator : Substrat = 1 : 2000, 70°C. [c] Katalysator : Substrat = 1 : 5000, 70 °C. [d] Katalysator : Substrat = 1 : 500, 25°C. [e] Katalysator : Substrat = 1 : 10000, 25°C. [f] Katalysator : Substrat = 1 : 20000, 70°C. | | | | | | |

## Patentansprüche

1. N-heterozyklischer Carbenkomplex nach einer der allgemeinen Formeln I - IV 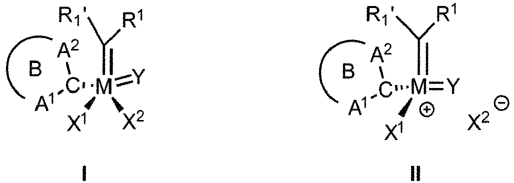 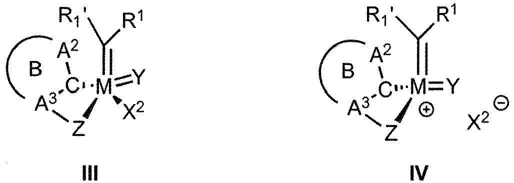 **dadurch gekennzeichnet, dass**
A¹ für NR² oder PR², A² für CR²R^{2'}, NR², PR², O oder S steht, A³ für N oder P steht, C für ein Carben-Kohlenstoffatom steht,
der Ring B ein unsubstituierter oder ein ein- oder mehrfach substituierter 5-bis 7-gliedriger Ring ist, der neben A¹, A² bzw. A³ weitere Heteroatome in Form von Stickstoff, Phosphor, Sauerstoff oder Schwefel enthalten kann und dessen Substituenten die für R² beschriebene Bedeutung haben können,
die Substituenten R² und R^{2'} unabhängig voneinander für H, einen linearen, teilcyclischen oder verzweigten C₁-C₁₈-Alkyl-, insbesondere einen C₁-C₇-Alkyl-, einen linearen, teilcyclischen oder verzweigten C₂-C₁₈-Alkenyl-, insbesondere einen C₂-C₇-Alkenyl-, einen C₃-C₁₂-Cycloalkyl-, insbesondere einen C₃-C₆-Cycloalkyl-, einen linearen, teilcyclischen oder verzweigten C₆-C₁₀₀-Polyoxaalkyl-, insbesondere C₆-C₃₀-Polyoxaalkyl-, einen C₅-C₁₄-Aryl- oder-Heteroaryl-Rest, einen C₅-C₁₄-Aryloxy-, einen linearen, teilcyclischen oder verzweigten C₁-C₁₈-Perfluoralkyl-, insbesondere C₁-C₇-Perfluoralkyl-, einen linearen, teilcyclischen oder verzweigten C₁-C₁₈-Perchloralkyl-, insbesondere einen C₁-C₇-Perchloralkyl-, einen linearen, teilcyclischen oder verzweigten teilfluorierten C₁-C₁₈-Alkyl-, insbesondere einen teilfluorierten C₁-C₇-Alkyl-, einen linearen, teilcyclischen oder verzweigten teilchlorierten C₁-C₁₈-Alkyl-, insbesondere einen teilchlorierten C₁-C₇-Alkyl-, einen per- oder teilfluorierten C₆-C₁₄-Aryl-, einen per- oder teilchlorierten C₅-C₁₄-Aryl-Rest steht, und, wenn A¹ und A² jeweils für NR² oder PR² stehen, R² gleich oder verschieden sein kann, oder
R² und R^{2'} zusammen einen linearen oder verzweigten C₁-C₁₈-Alkylen-, insbesondere einen C₁-C₇-Alkylenrest bilden,
M in den Formeln I, II, III oder IV für Cr, Mo oder W steht,
X¹ bzw. X² in Formeln I bis IV gleich oder verschieden sind und aus der Gruppe umfassend C₁-C₁₈ Carboxylate, C₁-C₁₈-Alkoxide, fluorierte C₁-C₁₈ Alkoxide, C₁-C₁₈ mono- oder polyhalogenierte Carboxylate, un-, ein- oder mehrfach substituierte C₆-C₁₈-Mono-, Bi- oder Terphenolate, Trifluormethansulfonat, nicht koordinierende Anionen, inbesonders Tetrakis(3,5-bis(trifluormethyl)phenyl)borat, Tetrakis(pentafluorophenyl)borat, Tetrakis(nonafluoro-t-butoxy)aluminat, Tetrafluoroborat, Hexafluorophosphat und Hexafluoroantimonat ausgewählt sind, wobei die Substituenten an den Mono-, Bi- oder Terphenolaten neben Halogen die gleiche Bedeutung haben können wie R²,
Y Sauerstoff, Schwefel, ein N-Adamantyl-, ein N-*tert*-Butyl, ein C₆-C₁₄-N-Aryl-Rest, insbesondere ein C₆-C₁₀-N-Aryl-Rest ist, wobei der Aryl-Rest ein- oder mehrfach mit Halogen, einem linearen oder verzweigten C₁-C₁₈ Alkyl-, einem linearen oder verzweigten C₁-C₁₈ Alkyloxy- oder einem unsubstituierten oder substituierten Phenyl-Rest substituiert sein kann, dessen Substituenten die gleiche Bedeutung haben wie R²,
Z eine lineare, teilcyclische oder verzweigte C₁-C₁₀-Alkylenoxy-, insbesondere eine C₁-C₅-Alkylenoxy-, eine lineare, teilcyclische oder verzweigte C₁-C₁₀-Alkylenthio-, insbesondere eine C₁-C₅-Alkylenthio-, eine lineare, teilcyclische oder verzweigte C₁-C₁₀-Alkylen-NR²-, insbesondere eine C₁-C₅-Alkylen-NR²-, eine C₆-C₁₀-Arylenoxy-, eine per- oder teilfluorierte C₆-C₁₄-Arylenoxy-, eine per- oder teilchlorierte C₆-C₁₄-Arylenoxy-, eine per- oder teilbromierte C₆-C₁₄-Arylenoxy-, eine C₆-C₁₄-Arylenthio-, eine per- oder teilfluorierte C₆-C₁₄-Arylenthio-, eine per- oder teilchlorierte C₆-C₁₄-Arylenthio-, eine per- oder teilbromierte C₆-C₁₄-Arylenthio- oder eine C₆-C₁₄-Arylen-NR²-, eine per- oder teilfluorierte C₆-C₁₄-Arylen-NR²-, eine per- oder teilchlorierte C₆-C₁₄-Arylen-NR²-, eine per- oder teilbromierte C₆-C₁₄-Arylen-NR²-, , eine C₆-C₁₄-Arylen-PR²-, eine per- oder teilfluorierte C₆-C₁₄-Arylen-PR²-, eine per- oder teilchlorierte C₆-C₁₄-Arylen-PR²-, eine per- oder teilbromierte C₆-C₁₄-Arylen-PR²-, eine Carboxyl, eine Thiocarboxyl oder eine Dithiocarboxyl-Gruppe und
R¹ und R^{1'} in den Formeln I bis IV unabhängig voneinander H oder ein aliphatischer oder aromatischer Rest sind, insbesondere eine lineare oder verzweigte C₁-C₁₈ Alkyl-Gruppe, bevorzugt in Form einer *tert*-Butyl- oder CMe₂Ph-Gruppe, oder eine unsubstituierte oder ein- oder mehrfach substituierte C₆-C₁₄-Aryl-Gruppe, wobei die Substituenten die für R² genannten Bedeutungen haben, bevorzugt in Form von 2-(2-Propoxy)phen-1-yl, 2-Methoxyphen-1-yl, 2,4,5-Trimethoxyphenyl oder Ferrocenyl.

2. Verbindungen gemäß Anspruch 1, **dadurch gekennzeichnet, dass** der Ring B ein aus der Gruppe umfassend 1,3-disubstituierte Imidazol-2-ylidene, 1,3-disubstituierte Imidazolin-2-ylidene, 1,3-disubstituierte Tetrahydropyrimidin-2-ylidene, 1,3-disubstituierte Diazepin-2-ylidene, 1,3-disubstituierte Dihydrodiazepin-2-ylidene, 1,3-disubstituierte Tetrahydrodiazepin-2-ylidene, N-substituierte Thiazol-2-ylidene, N-substituierte Thiazolin-2-ylidene, N-substituierte Triazol-2-ylidene, N-substituierte Dihydrotriazol-2-ylidene, ein- oder mehrfach substituierte Triazolin-2-ylidene, N-substituierte Thiadiazol-2-ylidene, ein- oder mehrfach substituierte Thiadiazolin-2-ylidene und ein- oder mehrfach substituierte Tetrahydrotriazol-2-ylidene ausgewählter Heterozyklus ist.

3. Verbindungen gemäß Anspruch 1 oder 2, **dadurch gekennzeichnet, dass** das der Ring B kovalent über eine Abstandsgruppe an einen festen Träger gebunden ist.

4. Verbindungen gemäß Anspruch 3, **dadurch gekennzeichnet, dass** der feste Träger ein polymerer Träger, insbesondere auf Basis von PS-DVB, und die Abstandsgruppe eine C₁-C₂₀-α,ω-Dioxaalkylen- oder eine C₁-C₂₀-Alkylenoxy-Gruppe ist.

5. Verbindungen gemäß Anspruch 3, **dadurch gekennzeichnet, dass** der feste Träger ein anorganischer Träger, insbesondere auf Basis von Siliciumdioxid, und die Abstandsgruppe eine Alkyl-Si(O)₃- oder eine Alkyl-SiR(O)₂-Gruppe ist, bei der R die gleiche Bedeutung hat wie R² in Anspruch 1.

6. Verbindungen gemäß einem der vorstehenden Ansprüche, **dadurch gekennzeichnet, dass** R¹ in Formeln I, II, III oder IV für *t*-Butyl, ein unsubstituiertes oder substituiertes Phenyl oder Ferrocenyl oder CMe₂Ph steht, R¹' neben H alle für R¹ genannten Bedeutungen haben kann und die Substituenten am Phenyl die gleiche Bedeutung haben können wie R².

7. Verwendung der Verbindungen mindestens nach einem der vorstehenden Ansprüche als Katalysator in Olefinmetathesereaktionen.

8. Verwendung nach Anspruch 7, **dadurch gekennzeichnet, dass** es sich bei den Olefinmetathesereaktionen um eine asymmetrische oder desymmetrisierende Ringschlussmetathese, eine Kreuzmetathese, eine Ring öffnende Kreuzmetathese, eine (Kreuz-) En-in Metathese, eine Ringschluss En-in Metathese, eine Kreuz En-diin Metathese, eine tandem-Ringöffnungs-Ringschluss Metathese, eine Ring öffnende Metathesepolymerisation (ROMP), eine 1-Alkinpolymerisation, eine azyklische Metathesepolymerisation (ADMET) oder eine Zyklopolymerisation von α,ω-Diinen handelt.

9. Verwendung nach Anspruch 7, **dadurch gekennzeichnet, dass** es sich bei der Olefinmetathesereaktion um die Olefinolyse von Fettsäureestern, insbesondere von pflanzlichen Ölen und Fette handelt, wobei die Öle bevorzugt ausgewählt sind aus der Gruppe umfassend Rizinusöl, Palmöl und Kokusnussöl und die Olefine bevorzugt Ethylen und/oder Buten umfassen.

10. Verwendung nach einem der Ansprüche 7 bis 9, **dadurch gekennzeichnet, dass** eine Verbindung gemäß Formeln II oder IV in einem organischen Lösemittel (I) oder in einer ionischen Flüssigkeit gelöst wird, dass diese Lösung in Form eines dünnen, insbesondere von 0,1 bis 200 µm-dicken Films auf ein Trägermaterial aufgebracht und mit dem Träger in ein Reaktionsgefäß (2) gebracht wird, und anschließend ein oder mehrere Substrate, die in einem mit dem organischen Lösungsmittel (I) oder der ionischen Flüssigkeit für die Verbindung der Formeln II oder IV nicht mischbaren Lösungsmittel (II) gelöst sind, in das Reaktionsgefäß gegeben werden.

11. Verwendung nach Anspruch 10, **dadurch gekennzeichnet, dass** als ionische Flüssigkeit 1,3-Dimethylimidazoliumsalze, 1,2,3-Trimethylimidazoliumsalze, 1-Butyl-3-methylimidazoliumsalze, 1-Butyl-2,3-dimethylimidazoliumsalze und als mit der ionischen Flüssigkeit nicht mischbares Lösungsmittel Toluol, Pentan, Hexan Heptan und/oder Octan herangezogen werden.

12. Verwendung nach Anspruch 10 oder 11, **dadurch gekennzeichnet, dass** die ein oder mehreren Substrate kontinuierlich in das Reaktionsgefäß geleitet und die daraus resultierenden Reaktionsprodukte kontinuierlich aus diesem abgeleitet werden.

13. Verwendung nach mindestens einem der Ansprüche 10 bis 12, **dadurch gekennzeichnet, dass** die Lösung der Verbindungen der Formeln I bis IV auf ein anorganisches Trägermaterial, insbesondere auf Basis von Siliciumdioxid, oder ein polymerorganisches Trägermaterial, insbesondere ein polymerorganisches monolithisches Trägermaterial, aufgebracht wird.

## Claims

1. An N-heterocyclic carbene complex according to one of the general formulae I - IV 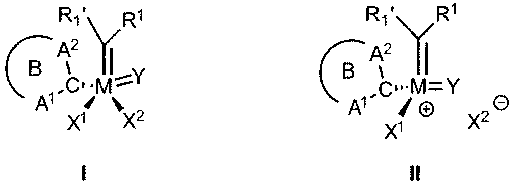 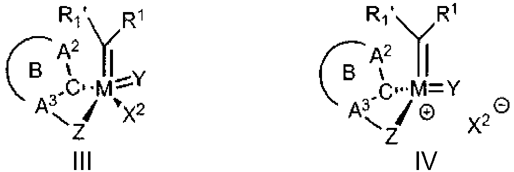 **characterised in that**
A¹ stands for NR² or PR², A² stands for CR²R^{2'}, NR², PR², O or S, A³ stands for N or P, C stands for a carbene carbon atom,
the ring B is an unsubstituted or a monosubstituted or polysubstituted 5-member to 7-member ring which in addition to A¹, A² or A³ may contain further heteroatoms in the form of nitrogen, phosphorus, oxygen or sulphur and the substituents of which may have the meaning described for R²,
the substituents R² and R^{2'} independently of each other stand for H, a linear, partially cyclic or branched C₁-C₁₈ alkyl group, especially a C₁-C₇ alkyl group, a linear, partially cyclic or branched C₂-C₁₈ alkenyl group, especially a C₂-C₇ alkenyl group, a C₃-C₁₂ cycloalkyl group, especially a C₃-C₆ cycloalkyl group, a linear, partially cyclic or branched C₆-C₁₀₀ polyoxaalkyl group, especially C₆-C₃₀ polyoxaalkyl group, a C₅-C₁₄ aryl or heteroaryl group, a C₅-C₁₄ aryloxy group, a linear, partially cyclic or branched C₁-C₁₈ perfluoroalkyl group, especially C₁-C₇ perfluoroalkyl group, a linear, partially cyclic or branched C₁-C₁₈ perchloroalkyl group, especially a C₁-C₇ perchloroalkyl group, a linear, partially cyclic or branched partially fluorinated C₁-C₁₈ alkyl group, especially a partially fluorinated C₁-C₇ alkyl group, a linear, partially cyclic or branched partially chlorinated C₁-C₁₈ alkyl group, especially a partially chlorinated C₁-C₇ alkyl group, a perfluorinated or partially fluorinated C₆-C₁₄ aryl group, a perchlorinated or partially chlorinated C₅-C₁₄ aryl group, and, if A¹ and A² each stand for NR² or PR², R² may be identical or different, or
R² and R^{2'} together form a linear or branched C₁-C₁₈ alkylene group, especially a C₁-C₇ alkylene group,
M in Formulae I, II, III or IV stands for Cr, Mo or W,
X¹ or X² in Formulae I to IV are identical or different and are selected from the group comprising C₁-C₁₈ carboxylates, C₁-C₁₈ alkoxides, fluorinated C₁-C₁₈ alkoxides, C₁-C₁₈ monohalogenated or polyhalogenated carboxylates, unsubstituted, monosubstituted or polysubstituted C₆-C₁₈ mono-, bi- or terphenolates, trifluoromethanesulphonate, non-coordinating anions, especially tetrakis(3,5-bis(trifluoromethyl)phenyl)borate, tetrakis(pentafluorophenyl)borate, tetrakis(nonafluoro-t-butoxy)aluminate, tetrafluoroborate, hexafluorophosphate and hexafluoroantimonate, wherein the substituents on the mono-, bi- or terphenolates in addition to halogen may have the same meaning as R²,
Y is oxygen, sulphur, an N-adamantyl group, an N-*tert* butyl, a C₆-C₁₄ N-aryl group, especially a C₆-C₁₀ N-aryl group, wherein the aryl group may be monosubstituted or polysubstituted with halogen, a linear or branched C₁-C₁₈ alkyl group, a linear or branched C₁-C₁₈ alkyloxy group or an unsubstituted or substituted phenyl group, the substituents of which have the same meaning as R²,
Z is a linear, partially cyclic or branched C₁-C₁₀ alkyleneoxy group, especially a C₁-C₅ alkyleneoxy group, a linear, partially cyclic or branched C₁-C₁₀ alkylenethio group, especially a C₁-C₅ alkylenethio group, a linear, partially cyclic or branched C₁-C₁₀ alkylene-NR² group, especially a C₁-C₅ alkylene-NR² group, a C₆-C₁₀ aryleneoxy group, a perfluorinated or partially fluorinated C₆-C₁₄ aryleneoxy group, a perchlorinated or partially chlorinated C₆-C₁₄ aryleneoxy group, a perbrominated or partially brominated C₆-C₁₄ aryleneoxy group, a C₆-C₁₄ arylenethio group, a perfluorinated or partially fluorinated C₆-C₁₄ arylenethio group, a perchlorinated or partially chlorinated C₆-C₁₄ arylenethio group, a perbrominated or partially brominated C₆-C₁₄ arylenethio group or a C₆-C₁₄ arylene-NR² group, a perfluorinated or partially fluorinated C₆-C₁₄ arylene-NR² group, a perchlorinated or partially chlorinated C₆-C₁₄ arylene-NR² group, a perbrominated or partially brominated C₆-C₁₄ arylene-NR² group, a C₆-C₁₄ arylene-PR² group, a perfluorinated or partially fluorinated C₆-C₁₄ arylene-PR² group, a perchlorinated or partially chlorinated C₆-C₁₄ arylene-PR² group, a perbrominated or partially brominated C₆-C₁₄ arylene-PR² group, a carboxyl, a thiocarboxyl or a dithiocarboxyl group, and
R¹ and R^{1'} in Formulae I to IV independently of each other are H or an aliphatic or aromatic group, especially a linear or branched C₁-C₁₈ alkyl group, preferably in the form of a *tert* butyl or CMe₂Ph group, or an unsubstituted or monosubstituted or polysubstituted C₆-C₁₄ aryl group, wherein the substituents have the meanings given for R², preferably in the form of 2-(2-propoxy)phen-1-yl, 2-methoxyphen-1-yl, 2,4,5-trimethoxyphenyl or ferrocenyl.

2. Compounds according to Claim 1, **characterised in that** the ring B is a heterocycle selected from the group comprising 1,3-disubstituted imidazol-2-ylidenes, 1,3-disubstituted imidazolin-2-ylidenes, 1,3-disubstituted tetrahydropyrimidin-2-ylidenes, 1,3-disubstituted diazepin-2-ylidenes, 1,3-disubstituted dihydrodiazepin-2-ylidenes, 1,3-disubstituted tetrahydrodiazepin-2-ylidenes, N-substituted thiazol-2-ylidenes, N-substituted thiazolin-2-ylidenes, N-substituted triazol-2-ylidenes, N-substituted dihydrotriazol-2-ylidenes, monosubstituted or polysubstituted triazolin-2-ylidenes, N-substituted thiadiazol-2-ylidenes, monosubstituted or polysubstituted thiadiazolin-2-ylidenes and monosubstituted or polysubstituted tetrahydrotriazol-2-ylidenes.

3. Compounds according to Claim 1 or Claim 2, **characterised in that** the ring B is bonded covalently to a solid support via a spacer group.

4. Compounds according to Claim 3, **characterised in that** the solid support is a polymeric support, especially based on PS-DVB, and the spacer group is a C₁-C₂₀ α,ω-dioxaalkylene group or a C₁-C₂₀ alkyleneoxy group.

5. Compounds according to Claim 3, **characterised in that** the solid support is an inorganic support, especially based on silicon dioxide, and the spacer group is an alkyl-Si(O)₃ group or an alkyl-SiR(O)₂ group in which R has the same meaning as R² in Claim 1.

6. Compounds according to one of the preceding claims, **characterised in that** R¹ in Formulae I, II, III or IV stands for *t*-butyl, an unsubstituted or substituted phenyl or ferrocenyl or CMe₂Ph, R^{1'} in addition to H may have all the meanings given for R¹ and the substituents on the phenyl may have the same meaning as R².

7. Use of the compounds at least according to one of the preceding claims as a catalyst in olefin metathesis reactions.

8. Use according to Claim 7, **characterised in that** the olefin metathesis reactions are an asymmetrical or desymmetrising ring-closing metathesis, a cross metathesis, a ring-opening cross metathesis, a (cross) ene-yne metathesis, a ring-closing ene-yne metathesis, a cross ene-diyne metathesis, a tandem ring-opening ring-closing metathesis, a ring-opening metathesis polymerisation (ROMP), a 1-alkyne polymerisation, an acyclic metathesis polymerisation (ADMET) or a cyclopolymerisation of α,ω-diynes.

9. Use according to Claim 7, **characterised in that** the olefin metathesis reaction is the olefinolysis of fatty acid esters, especially of vegetable oils and fats, the oils preferably being selected from the group comprising castor oil, palm oil and coconut oil, and the olefins preferably comprising ethylene and/or butene.

10. Use according to one of Claims 7 to 9, **characterised in that** a compound according to Formulae II or IV is dissolved in an organic solvent (I) or in an ionic liquid, **in that** this solution is applied to a support material in the form of a thin film, especially of a thickness of 0.1 to 200 µm, and with the support is introduced into a reaction vessel (2), and then one or more substrates which are dissolved in a solvent (II) which is non-miscible with the organic solvent (I), or with the ionic liquid for the compound of Formulae II or IV, are charged into the reaction vessel.

11. Use according to Claim 10, **characterised in that** 1,3-dimethylimidazolium salts, 1,2,3-trimethylimidazolium salts, 1-butyl-3-methylimidazolium salts, 1-butyl-2,3-dimethylimidazolium salts are used as the ionic liquid, and toluene, pentane, hexane, heptane and/or octane are used as the solvent which is not miscible with the ionic liquid.

12. Use according to Claim 10 or Claim 11, **characterised in that** the one or more substrate(s) is/are passed continuously into the reaction vessel and the reaction products resulting therefrom are discharged continuously from the latter.

13. Use according to at least one of Claims 10 to 12, **characterised in that** the solution of the compounds of Formulae I to IV is applied to an inorganic support material, especially based on silicon dioxide, or a polymer-organic support material, especially a polymer-organic monolithic support material.

## Revendications

1. Complexe carbène N-hétérocyclique selon l'une des formules générales I à IV 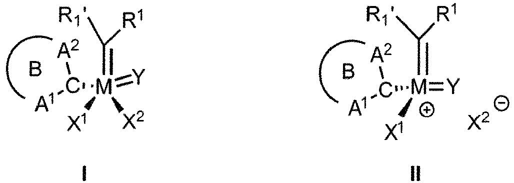 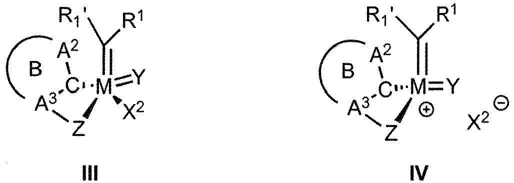 **caractérisé en ce que**
A¹ représente NR² ou PR², A² représente CR²R^{2'}, NR², PR², O ou S, A³ représente N ou P, C représente un atome de carbone-carbène,
le cycle B est un cycle non substitué ou un cycle mono- ou polysubstitué de 5 à 7 chaînons, qui peut comprendre, en plus de A¹, A² et/ou A³, d'autres hétéroatomes sous la forme d'azote, de phosphore, d'oxygène ou de soufre, et dont les substituants peuvent avoir la signification décrite pour R²,
les substituants R² et R^{2'} représentent, indépendamment les uns des autres, H, un radical alkyle en C₁-C₁₈ linéaire, partiellement cyclique ou ramifié, en particulier un radical alkyle en C₁-C₇, un radical alcényle en C₂-C₁₈ linéaire, partiellement cyclique ou ramifié, en particulier un radical alcényle en C₂-C₇, un radical cycloalkyle en C₃-C₁₂, en particulier un radical cycloalkyle en C₃-C₆, un radical polyoxaalkyle en C₆-C₁₀₀ linéaire, partiellement cyclique ou ramifié, en particulier polyoxaalkyle en C₆-C₃₀, un radical aryle ou hétéroaryle en C₅-C₁₄, un radical aryloxy en C₅-C₁₄, un radical perfluoroalkyle en C₁-C₁₈ linéaire, partiellement cyclique ou ramifié, en particulier perfluoroalkyle en C₁-C₇, un radical perchloroalkyle en C₁-C₁₈ linéaire, partiellement cyclique ou ramifié, en particulier un radical perchloroalkyle en C₁-C₇, un radical alkyle en C₁-C₁₈ partiellement fluoré linéaire, partiellement cyclique ou ramifié, en particulier un radical alkyle en C₁-C₇ partiellement fluoré, un radical alkyle en C₁-C₁₈ partiellement chloré linéaire, partiellement cyclique ou ramifié, en particulier un radical alkyle en C₁-C₇ partiellement chloré, un radical aryle en C₆-C₁₄ perfluoré ou partiellement fluoré, un radical aryle en C₅-C₁₄ perchloré ou partiellement chloré, et, si A¹ et A² représentent respectivement NR² ou PR², les R² peuvent être identiques ou différents, ou
R² et R^{2'} forment conjointement un radical alkylène en C₁-C₁₈ linéaire ou ramifié, en particulier un radical alkylène en C₁-C₇,
M dans les formules I, II, III ou IV représente Cr, Mo ou W,
X¹ et/ou X² dans les formules I à IV sont identiques ou différents et sont choisis dans le groupe comprenant les carboxylates en C₁-C₁₈, les alcoxydes en C₁-C₁₈, les alcoxydes en C₁-C₁₈ fluorés, les carboxylates en C₁-C₁₈ mono- ou polyhalogénés, les mono-, bi- ou terphénolates en C₆-C₁₈ non substitués, mono- ou polysubstitués, le trifluoro-méthane-sulfonate, les anions ne formant pas de liaison de coordination, en particulier le tétrakis(3,5-bis(trifluoro-méthyl)phényl)borate, le tétrakis(pentafluorophényl)borate, le tétrakis(nonafluoro-t-butoxy)aluminate, le tétrafluoro-borate, l'hexafluorophosphate et l'hexafluoro-antimoniate, les substituants sur les mono-, bi- ou terphénolates à côté de l'halogène pouvant avoir la même signification que R²,
Y est l'oxygène, le soufre, un radical N-adamantyl, un radical N-*tert*-butyle, un radical N-aryle en C₆-C₁₄, en particulier un radical N-aryle en C₆-C₁₀, le radical aryle pouvant être mono- ou polysubstitué par un halogène, un radical alkyle en C₁-C₁₈ linéaire ou ramifié, un radical alkyloxy en C₁-C₁₈ linéaire ou ramifié ou un radical phényle non substitué ou substitué, dont les substituants ont la même signification que R²,
Z est un groupe alkylèneoxy en C₁-C₁₀ linéaire, partiellement cyclique ou ramifié, en particulier un groupe alkylèneoxy en C₁-C₅, un groupe alkylènethio en C₁-C₁₀ linéaire, partiellement cyclique ou ramifié, en particulier un groupe alkylènethio en C₁-C₅, un groupe alkylène en C₁-C₁₀-NR² linéaire, partiellement cyclique ou ramifié, en particulier un groupe alkylène en C₁-C₅-NR², un groupe arylèneoxy en C₆-C₁₀, un groupe arylèneoxy en C₆-C₁₄ perfluoré ou partiellement fluoré, un groupe arylèneoxy en C₆-C₁₄ perchloré ou partiellement chloré, un groupe arylèneoxy en C₆-C₁₄ perbromé ou partiellement bromé, un groupe arylènethio en C₆-C₁₄, un groupe arylènethio en C₆-C₁₄ perfluoré ou partiellement fluoré, un groupe arylènethio en C₆-C₁₄ perchloré ou partiellement chloré, un groupe arylènethio en C₆-C₁₄ perbromé ou partiellement bromé ou un groupe arylène en C₆-C₁₄-NR², un groupe arylène en C₆-C₁₄-NR² perfluoré ou partiellement fluoré, un groupe arylène en C₆-C₁₄-NR² perchloré ou partiellement chloré, un groupe arylène en C₆-C₁₄-NR² perbromé ou partiellement bromé, un groupe arylène en C₆-C₁₄-PR², un groupe arylène en C₆-C₁₄-PR² perfluoré ou partiellement fluoré, un groupe arylène en C₆-C₁₄-PR² perchloré ou partiellement chloré, un groupe arylène en C₆-C₁₄-PR² perbromé ou partiellement bromé, un groupe carboxyle, un groupe thiocarboxyle ou un groupe dithio-carboxyle et
R¹ et R^{1'} dans les formules I à IV, indépendamment les uns des autres, représentent H ou un radical aliphatique ou aromatique, en particulier un groupe alkyle en C₁-C₁₈ linéaire ou ramifié, de préférence sous la forme d'un groupe *tert*-butyle ou CMe₂Ph, ou un groupe aryle en C₆-C₁₄ non substitué ou mono- ou polysubstitué, les substituants ayant les significations indiquées pour R², de préférence sous la forme du 2-(2-propoxy)phén-1-yle, 2-méthoxyphén-1-yle, 2,4,5-triméthoxyphényle ou ferrocényle.

2. Composés selon la revendication 1, **caractérisés en ce que** le cycle B est un hétérocycle choisi dans le groupe comprenant les imidazole-2-ylidènes 1,3-disubstitués, les imidazoline-2-ylidènes 1,3-disubstitués, les tétrahydropyrimidine-2-ylidènes 1,3-disubstitués, les diazépine-2-ylidènes 1,3-disubstitués, les dihydrodiazépine-2-ylidènes 1,3-disubstitués, les tétrahydrodiazépine-2-ylidènes 1,3-disubstitués, les thiazole-2-ylidènes N-substitués, les thiazoline-2-ylidènes N-substitués, les triazole-2-ylidènes N-substitués, les dihydrotriazole-2-ylidènes N-substitués, les triazoline-2-ylidènes mono- ou polysubstitués, les thiadiazole-2-ylidènes N-substitués, les thiadiazoline-2-ylidènes mono- ou polysubstitués, et les tétrahydrotriazole-2-ylidènes mono- ou polysubstitués.

3. Composés selon la revendication 1 ou 2, **caractérisés en ce que** le cycle B est lié de manière covalente à un support solide par le biais d'un groupe écarteur.

4. Composés selon la revendication 3, **caractérisés en ce que** le support solide est un support polymère, en particulier à base de PS-DVB, et le groupe écarteur est un groupe α,ω-dioxaalkylène en C₁-C₂₀ ou un groupe alkylèneoxy en C₁-C₂₀ .

5. Composés selon la revendication 3, **caractérisés en ce que** le support solide est un support inorganique, en particulier à base de dioxyde de silicium, et le groupe écarteur est un groupe alkyle-Si(O)₃ ou un groupe alkyle-SiR(O)₂ dans lequel R a la même signification que R² dans la revendication 1.

6. Composés selon l'une des revendications précédentes, **caractérisés en ce que** R¹ dans les formules I, II, III ou IV représente t-butyle, un phényle non substitué ou substitué ou ferrocényle ou CMe₂Ph, R^{1'} à côté de H peut avoir toutes les significations indiquées pour R¹ et les substituants sur le phényle peuvent avoir la même signification que R².

7. Utilisation des composés au moins selon l'une des revendications précédentes comme catalyseurs dans les réactions de métathèse des oléfines.

8. Utilisation selon la revendication 7, **caractérisée en ce que** les réactions de métathèse des oléfines sont une métathèse de fermeture de cycle asymétrique ou dissymétrique, une métathèse croisée, une métathèse croisée d'ouverture de cycle, une métathèse ène-ine (croisée), une métathèse ène-ine de fermeture de cycle, une métathèse ènediine croisée, une métathèse d'ouverture de cycle-de fermeture de cycle en tandem, une métathèse de polymérisation par ouverture de cycle (ROMP), une polymérisation en 1-alcyne, une métathèse de polymérisation acyclique (ADMET) ou une cyclo-polymérisation des α,ω-diines.

9. Utilisation selon la revendication 7, **caractérisée en ce que** la réaction de métathèse des oléfines est l'oléfinolyse d'esters d'acides gras, en particulier d'huiles et de graisses végétales, les huiles étant choisies de préférence dans le groupe comprenant l'huile de ricin, l'huile de palme et l'huile de noix de coco et les oléfines comprenant de préférence de l'éthylène et/ou du butène.

10. Utilisation selon l'une des revendications 7 à 9, **caractérisée en ce qu'**un composé selon les formules II ou IV est dissous dans un solvant (I) organique ou un fluide ionique, **en ce que** ladite solution est déposée sous la forme d'une couche mince, notamment d'une épaisseur de 0,1 à 200 µm, sur un matériau support et est amenée avec le support dans une cuve de réaction (2), et puis un ou plusieurs substrats dissous dans un solvant (II) non miscible avec le solvant (I) organique ou le fluide ionique pour le composé des formules II ou IV sont versés dans la cuve de réaction.

11. Utilisation selon la revendication 10, **caractérisée en ce que** l'on utilise comme liquide ionique les sels de 1,3-diméthylimidazolium, les sels de 1,2,3-triméthyl-imidazolium, les sels de 1-butyl-3-méthylimidazolium, les sels de 1-butyl-2,3-diméthylimidazolium et comme solvant non miscible avec le fluide ionique le toluène, le pentane, l'hexane, l'heptane et/ou l'octane.

12. Utilisation selon la revendication 10 ou 11, **caractérisée en ce que** les un ou plusieurs substrats sont conduits en continu dans la cuve de réaction et les produits réactionnels en résultant sont dérivés en continu de celle-ci.

13. Utilisation selon au moins l'une des revendications 10 à 12, **caractérisée en ce que** la solution des composés de formules I à IV est déposée sur un matériau support inorganique, en particulier à base de dioxyde de silicium, ou un matériau support organo-polymère, en particulier un matériau support monolithique organo-polymère.
